# EUROPÄISCHE PATENTANMELDUNG

(11) **EP 3 048 156 A1**
(43) Veröffentlichungstag der Anmeldung: **27.07.2016**
(21) Anmeldenummer: 15168363.8
(22) Anmeldetag: 20.05.2015
(51) Int. Cl.: C09K 11/06, C07D 307/00, H01L 51/00

(54) **ORGANISCHE MOLEKÜLE, INSBESONDERE ZUR VERWENDUNG IN OPTOELEKTRONISCHEN BAUELEMENTEN**

(30) Priorität: 20.01.2015 EP 15151870
(71) Anmelder: cynora GmbH, 76646 Bruchsal (DE)
(72) Erfinder: Ambrosek, David, 10437 Berlin (DE); Danz, Michael, 76344 Eggenstein-Leopoldshafen (DE); Flügge, Harald, 76135 Karlsruhe (DE); Friedrichs, Jana, 76137 Karlsruhe (DE); Grab, Tobias, 76133 Karlsruhe (DE); Jacob, Andreas, 76137 Karlsruhe (DE); Seifermann, Stefan, 77815 Bühl (DE); Volz, Daniel, 76137 Karlsruhe (DE); Liaptsis, Georgios, 68161 Mannheim (DE)
(74) Vertreter: Hoppe, Georg Johannes

(57) **Zusammenfassung**

Die Erfindung betrifft organische Moleküle, insbeonsdere zur Verwendung in optoelektronischen Bauelementen, wie OLED. Erfindungsgemäß weist das organisches Molekül eine Struktur der Formel 1 auf 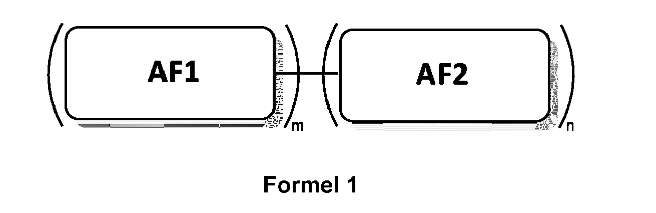 wobei gilt:
n und m sind unabhängig voneinander ausgewählt aus einer ganzzahligen Zahl von 1 bis 3,
AF1 ist eine erste chemische Einheit, aufweisend ein konjugiertes System, insbesondere mindestens sechs in Konjugation stehende π-Elektronen (z. B. in Form mindestens eines aromatischen Systems);
AF2 ist eine zweite chemische Einheit, aufweisend ein konjugiertes System, insbesondere mindestens sechs in Konjugation stehende π-Elektronen (z. B. in Form mindestens eines aromatischen Systems);
AF1 ≠ AF2;
und wobei mindestens eine der beiden chemischen Einheiten AF1 oder AF2 eine Struktur gemäß Formel 1 a aufweist

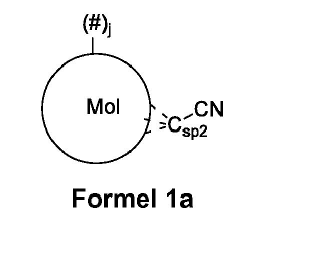

## Beschreibung

Die Erfindung betrifft rein organische Moleküle und deren Verwendung in organischen lichtemittierenden Dioden (OLEDs) und in anderen optoelektronischen Bauelementen.

### Stand der Technik

In den letzten Jahren hat sich die auf OLED (organische lichtemittierende Dioden) basierende Technik im Bereich Bildschirmtechnik etabliert, so dass nun die ersten hierauf aufbauenden kommerziellen Produkte erhältlich werden. Neben der Bildschirmtechnik eignen sich OLEDs auch für die Anwendung in flächiger Beleuchtungstechnik. Aus diesem Grund wird bezüglich der Entwicklung neuer Materialien intensive Forschung betrieben.

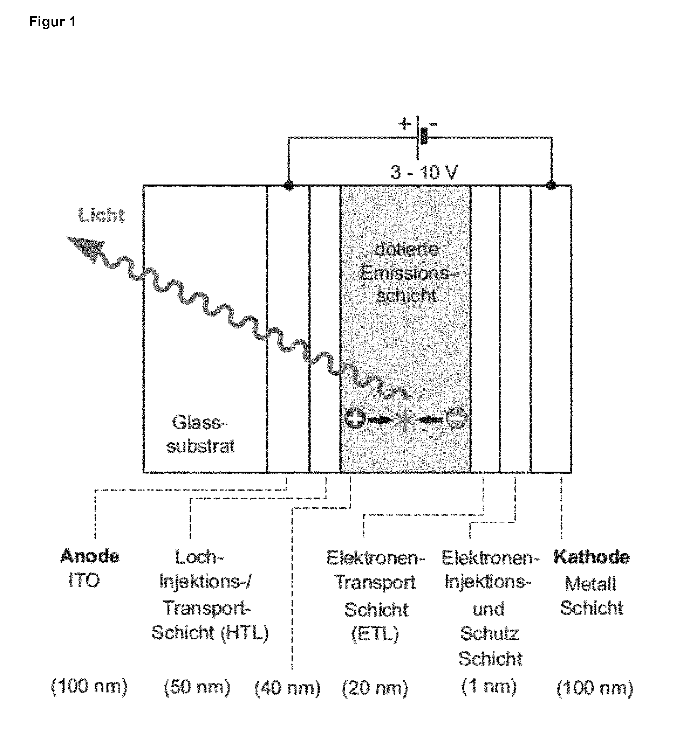

OLEDs sind in der Regel in Schichtenstrukturen realisiert, welche überwiegend aus organischen Materialien bestehen. Zum besseren Verständnis ist in Figur 1 ein vereinfachter Aufbau exemplarisch dargestellt. Herzstück solcher Bauteile ist die Emitterschicht, in der in der Regel emittierende Moleküle in einer Matrix eingebettet sind. In dieser Schicht treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten bezüglich OLEDs (Tang et al. Appl. Phys. Lett. 1987, 51, 913) ist diese Technik besonders auf dem Gebiet der Emittermaterialien immer weiterentwickelt worden. Während die ersten Materialien, die auf rein organischen Molekülen beruhen, aufgrund von Spinstatistik maximal 25 % der Exzitonen in Licht umwandeln konnten, konnte durch die Verwendung von phosphoreszierenden Verbindungen dieses grundsätzliche Problem umgangen werden, so dass zumindest theoretisch alle Exzitonen in Licht umgewandelt werden können. Bei diesen Materialien handelt es sich in der Regel um Übergangsmetall-Komplexverbindungen, in denen das Metall aus der dritten Periode der Übergangsmetalle gewählt wird. Hierbei werden vorwiegend sehr teure Edelmetalle wie Iridium, Platin oder auch Gold eingesetzt. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Neben den Kosten ist auch die Stabilität der Materialien zum Teil nachteilig für die Verwendung.

Eine neue Generation von OLEDs basiert auf der Ausnutzung von verzögerter Fluoreszenz (TADF: thermally activated delayed fluorescence oder auch singlet harvesting). Hierbei können beispielsweise Cu(l)-Komplexe verwendet werden, die aufgrund eines geringen Energieabstandes zwischen dem untersten Triplett-Zustand T₁ und dem darüberliegenden Singulett-Zustand S₁ (ΔE(S₁-T₁) Triplett-Exitonen thermisch in einen Singulett-Zustand rückbesetzen können. Neben der Verwendung von Übergangsmetallkomplexen können auch rein organische Moleküle diesen Effekt ausnutzen.

Einige solcher TADF-Materialien wurden bereits in ersten optoelektronischen Bauelementen eingesetzt. Die bisherigen Lösungen weisen jedoch Nachteile und Probleme auf: Die TADF-Materialien weisen in den optoelektronischen Bauelementen oftmals keine ausreichende Langzeitstabilität, keine ausreichende thermische oder keine ausreichende chemische Stabilität gegenüber Wasser und Sauerstoff auf. Außerdem sind nicht alle wichtigen Emissionsfarben verfügbar. Weiterhin sind einige TADF-Materialien nicht verdampfbar und dadurch für den Einsatz in kommerziellen optoelektronischen Bauteilen nicht geeignet. Auch weisen einige TADF-Materialien keine passende Energielagen zu den weiteren im optoelektronischen Bauteil verwendeten Materialien (z.B. HOMO-Energien von TADF-Emittern von größer gleich -5,9 eV) auf. Nicht mit allen TADF-Materialien lassen sich ausreichend hohe Effizienzen der optoelektronischen Bauelemente bei hohen Stromdichten bzw. hohe Leuchtdichten erreichen. Weiterhin sind die Synthesen einiger TADF-Materialien aufwendig.

Vor diesem Hintergrund lag der vorliegenden Erfindung die Aufgabe zu Grunde, zumindest einige der oben genannten Nachteile zu überwinden.

### Beschreibung

Das der Erfindung zu Grunde liegende Problem wird in einem Aspekt durch die Bereitstellung von organischen Molekülen gelöst, die eine Struktur der Formel 1 aufweisen oder eine Struktur der Formel 1 haben: 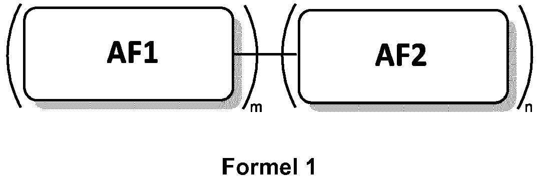 wobei gilt:
n und m sind unabhängig voneinander ausgewählt aus einer ganzzahligen Zahl von 1 bis 3,
AF1 ist eine erste chemische Einheit, aufweisend ein konjugiertes System, insbesondere mindestens sechs in Konjugation stehende π-Elektronen (z. B. in Form mindestens eines aromatischen Systems);
AF2 ist eine zweite chemische Einheit, aufweisend ein konjugiertes System, insbesondere mindestens sechs in Konjugation stehende π-Elektronen (z. B. in Form mindestens eines aromatischen Systems);
AF1 # AF2;
und wobei mindestens eine der beiden chemischen Einheiten AF1 oder AF2 eine Struktur gemäß Formel 1a aufweist 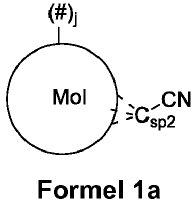
und wobei bevorzugt nur eine der beiden chemischen Einheiten AF1 oder AF2 ausgewählt ist aus einer der durch die Formel 1a beschriebenen Struktur;
und wobei bevorzugt die jeweils andere chemische Einheit AF ausgewählt ist aus einer der in Tabelle 2 aufgeführten Strukturen; und wobei diese andere chemische Einheit AF ein Substituent eines Atoms ist, welches direkt benachbart mindestens ein Atom mit einem Substituenten ungleich H oder D aufweist;
wobei in Formel 1a bedeutet:
   # Anknüpfungspunkte an weitere chemische Einheit AF;
   j ist eine ganze Zahl von 1 bis 5, bevorzugt von 1 bis 3;
   Cₛₚ₂ ist ein aromatisches oder olefinisches Kohlenstoffatom und Bestandteil eines olefinischen, aromatischen oder heteroaromatischen Molekülfragments Mol;
und wobei insbesondere
   - die Differenz der Energie des HOMO der zweiten chemischen Einheit AF2 und der Energie des HOMO der ersten chemischen Einheit AF1 > 0,8 eV ist (Δ HOMO = HOMO(AF2)-HOMO(AF1) > 0,8 eV);
   - die Differenz der Energie des LUMO der zweiten chemischen Einheit AF2 und der Energie des LUMO der ersten chemischen Einheit AF1 > 0,8 eV ist (Δ LUMO = LUMO(AF2)-LUMO(AF1) > 0,8 eV); und/oder
   - die Differenz der Energie des LUMO der ersten chemischen Einheit AF1 und der Energie des HOMO der zweiten chemischen Einheit AF2 > 0,9 eV ist (Δ Gap = LUMO (AF1)-HOMO(AF2)> 0,9 eV).

Dabei werden die Energiewerte HOMO(AF1), HOMO(AF2), LUMO(AF1), LUMO(AF2) mithilfe der Dichtefunktionaltheorie (DFT) berechnet, wobei die Anknüpfungspositionen der ambifunktionalen Einheiten mit einem Wasserstoffatom entsprechend ihrer chemischen Valenzen abgesättigt werden. Die angegeben Grenzen beziehen sich auf Orbitalenergien in eV, die mit dem BP86-Funktional berechnet werden (Becke, A. D. Phys. Rev. A1988, 38, 3098-3100; Perdew, J. P. Phys. Rev. B1986, 33, 8822-8827).

Definitionsgemäß weist AF2 im Vergleich zu AF1 immer einen betragsmäßig niedrigeren HOMO-Zahlenwert auf (und damit auch entsprechend einen betragsmäßig niedrigeren LUMO-Zahlenwert) als AF2 (| E_{HOMO}(AF2) | < |E_{HOMO}(AF1)| und | E_{LUM}o(AF2) | < | E_{LUMO}(AF1) |);

In einer Ausführungsform weist mindestens eine der chemischen Einheiten AF1 und AF2 eine Struktur nach einer der Formeln 2a bis 2c auf 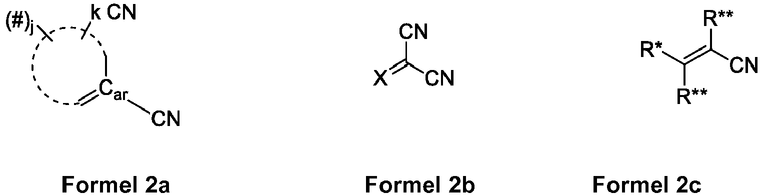 wobei gilt:
# Anknüpfungspunkte an weitere chemische Einheit AF;
k ist eine ganze Zahl von 0 bis 2;
Cₐᵣ ist ein aromatisches Kohlenstoffatom und Bestandteil eines aromatischen oder heteroaromatischen mono-, oder di- oder tri- oder tetrazyklischen annelierten Ringsystems, bevorzugt ausgewählt aus der Gruppe von Phenyl, Naphthyl, Anthracenyl, Pyrrolyl, Thienothienyl, Triphenylenyl und Spirobiphenylenyl;
X ist ausgewählt aus der Gruppe von PPh₃, 4H-Pyran, 9,10-Dihydroacridin, Diphenylmethylen oder 2-Methylen-2,5-dihydrothiazol, wobei weitere chemischen Einheiten AF über diesen Teil des Moleküls angeknüpft sind;
R** ist entweder ein Rest R* oder kennzeichnet die Anknüpfungsposition an eine weitere chemische Einheit AF oder ist eine Nitrilgruppe, wobei ein Rest R** zwingend eine Anknüpfungsposition an eine chemische Einheit AF kennzeichnet; und wobei optional zwei oder mehrere Substituenten R** auch miteinander ein mono- oder polycyclisches, aliphatisches Ringsystem bilden;
R* ist bei jedem Auftreten unabhängig voneinander ausgewählt aus der Gruppe bestehend aus H, Deuterium, Phenyl, Naphthyl, N(R²)₂, -CN, -NC, -SCN, -CF₃, -NO₂, C(=O)OH, C(=O)OR³, C(=O)N(R³)₂, C(=O)SR³, C(=S)SR³, Si(R⁴)₃, B(OR⁵)₂, B(N(R⁶)₂)₂, C(=O)R³, P(=O)(R⁷)₂, As(=O)(R⁷)₂, P(=S)(R⁷)₂, As(=S)(R⁷)₂, S(=O)R³, S=NR³, S(=O)NR³, S(=O)₂NR³, S(=O)₂R³, O-S(=O)₂R³, SF₅, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C=C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, - C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R* auch miteinander ein monozyklisches aliphatisches Ringsystem mit insgesamt fünf oder sechs Ringgliedern bilden;
R² ist bei jedem Auftreten unabhängig voneinander ausgewählt aus der Gruppe bestehend aus H, Deuterium, Phenyl, Naphthyl, CF₃, C(=O)OR³, C(=O)N(R²)₂, Si(R⁴)₃, C(=O)R³, P(=O)(R⁷)₂, As(=O)(R⁷)₂ P(=S)(R⁷)2, As(=S)(R⁷)₂, S(=O)R³, S(=O)₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C=C-, bzw. eine benachbarte CH₂-Gruppe durch -Si(R⁴)₂-,-Ge(R⁴)₂-, -Sn(R⁴)₂, -C(=O)-, -C(=S)-, -C(=Se)-, -C=N-, -C(=O)O-, -C(=O)N(R³)-, -P(=O)(R⁷)-,-As(=O)(R⁷)-, -P(=S)(R⁷)-, -As(=S)(R⁷)-, -S(=O)-,-S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R² auch miteinander ein mono- oder polycyclisches, aliphatisches, aromatisches und/oder benzoannelliertes Ringsystem bilden;
R³ ist bei jedem Auftreten unabhängig voneinander ausgewählt aus der Gruppe bestehend aus H, Deuterium, Phenyl, Naphthyl, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R³ auch miteinander ein mono- oder polycyclisches, aliphatisches Ringsystem bilden;
R⁴ ist bei jedem Auftreten unabhängig voneinander ausgewählt aus der Gruppe bestehend aus H, Deuterium, Phenyl, Naphthyl, N(R²)₂, CN, CF₃, OH, C(=O)OR³, C(=O)N(R³)₂, C(=O)R³, P(=O)(R⁷)₂, As(=O)(R⁷)₂, P(=S)(R⁷)₂, As(=S)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C=C-, bzw. eine benachbarte CH₂-Gruppe durch -Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷), -As(=S)(R⁷)-, -S(=O)-,-S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁴ auch miteinander ein mono- oder polycyclisches, aliphatisches, aromatisches und/oder benzoannelliertes Ringsystem bilden;
R⁵ ist bei jedem Auftreten unabhängig voneinander ausgewählt aus der Gruppe bestehend aus Phenyl, Naphthyl, CF₃, C(=O)R³,P(=O)(R⁷)₂, As(=O)(R⁷)₂, eine lineare Alkyl-, Alkoxy-oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy-oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C=C-, bzw. eine benachbarte CH₂-Gruppe durch -Si(R⁴)₂-, -Ge(R⁴)₂-, -Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-,-P(=S)(R⁷)-, -As(=S)(R⁷)-, -S(=O)-,-S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁵ auch miteinander ein mono- oder polycyclisches, aliphatisches, aromatisches und/oder benzoannelliertes Ringsystem bilden;
R⁶ ist bei jedem Auftreten unabhängig voneinander ausgewählt aus der Gruppe bestehend aus Phenyl, Naphthyl, CF₃, Si(R⁴)₃, C(=O)R³, P(=O)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch-R⁹C=CR⁹-, -C=C-, bzw. eine benachbarte CH₂-Gruppe durch-Si(R⁴)₂-, -Ge(R⁴)₂-, Sn(R⁴)₂,-C(=O)-, -C(=S)-, -C(=Se)-, -C=N-, -C(=O)O-, -C(=O)N(R³)-, -P(=O)(R⁷)-, -As(=O)(R⁷)-,-P(=S)(R⁷), -As(=S)(R⁷)-, -S(=O)-,-S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁶ auch miteinander ein mono- oder polycyclisches, aliphatisches, aromatisches und/oder benzoannelliertes Ringsystem bilden;
R⁷ ist bei jedem Auftreten unabhängig voneinander ausgewählt aus der Gruppe bestehend aus, Phenyl, Naphthyl, N(R²)₂, CN, CF₃, C(=O)OR³, C(=O)N(R³)₂, Si(R⁴)₃, C(=O)R³,eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C=C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, -C=N-, -C(=O)O-, - C(=O)N(R³)-, -P(=O)(R⁷)-, -As(=O)(R⁷)-, -P(=S)(R⁷), -As(=S)(R⁷)-, -S(=O)-,-S(=O)₂-, -NR²-, _ O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination dieser Systeme; dabei können zwei oder mehrere dieser Substituenten R⁷ auch miteinander ein mono- oder polycyclisches, aliphatisches, aromatisches und/oder benzoannelliertes Ringsystem bilden;
R⁸ ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R⁸ auch miteinander ein mono- oder polycyclisches, aliphatisches Ringsystem bilden;
R⁹ ist bei jedem Auftreten unabhängig voneinander ausgewählt aus der Gruppe bestehend aus H, Deuterium, Phenyl, Naphthyl, N(R²)₂, CN, CF₃, NO₂, OH, COOH, C(=O)OR³, C(=O)N(R³)₂, Si(R⁴)₃, B(OR⁵)₂, C(=O)R³, P(=O)(R⁷)₂, P(=S)(R⁷)₂, As(=O)(R⁷)₂, P(=S)(R⁷)₂, S(=O)R³, S(=O)₂R³, OSO₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁸ substituiert sein kann, wobei eine oder mehrere nicht benachbarte CH₂-Gruppen durch -R³C=CR³-, -C=C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, - C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁸ substituiert sein kann, oder eine Kombination dieser Systeme; dabei können zwei oder mehrere dieser Substituenten R⁹ auch miteinander ein mono- oder polycyclisches, aliphatisches, aromatisches und/oder benzoannelliertes Ringsystem bilden.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt N, O, und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Napthalin, Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus, N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10% der verschiedenen Atome), wie z.B. ein sp3-hybridisiertes C-, Si-, oder N-Atom, ein sp2-hybridisiertes C-, N- oder O-Atom oder ein sp-hybridisiertes C-Atom, verbunden sein können. So sollen beispielsweise auch Systeme wie 9,9'-Diarylfluoren, Triarylamin, Diarylether, Stilben etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Napthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyarzinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3 Oxadiazol, 1,2,3-Thiadiazol, 1,2,5-Thiadiazol, 1,3,4-Thiadiazol, 1,3,5-Triazin, 1,2,4-Triazin, 1,2,3-Triazin, Tetrazol, 1,2,3,5-Tetrazin, 1,2,3,4-Tetrazin, Purin, Pteridin, Indolizin und Benzothiadiazol oder Kombinationen dieser Gruppen.

In einer Ausführungsform weist mindestens eine der chemischen Einheiten AF1 und AF2 eine Struktur nach einer der Formeln 3a bis 3e auf 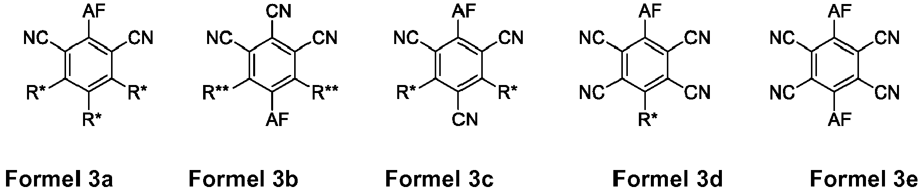 wobei die für die Formeln 1a und 2a-2c angegebenen Definitionen gelten und AF hier die Anknüpfungsposition einer weiteren chemischen Einheit kennzeichnet.

In einer weiteren Ausführungsform hat mindestens eine der chemischen Einheiten AF1 und AF2 eine Struktur ausgewählt aus einer der in Tabelle 1 gezeigten Strukturen;
und wobei die Anknüpfungspositionen für ein weitere chemische Einheiten AF mit Kleinbuchstaben gekennzeichnet sind;
und wobei die Reste AF1 und AF2 bevorzugt über Einfachbindungen direkt miteinander verbunden sind.

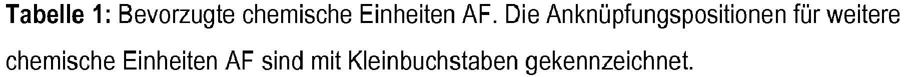

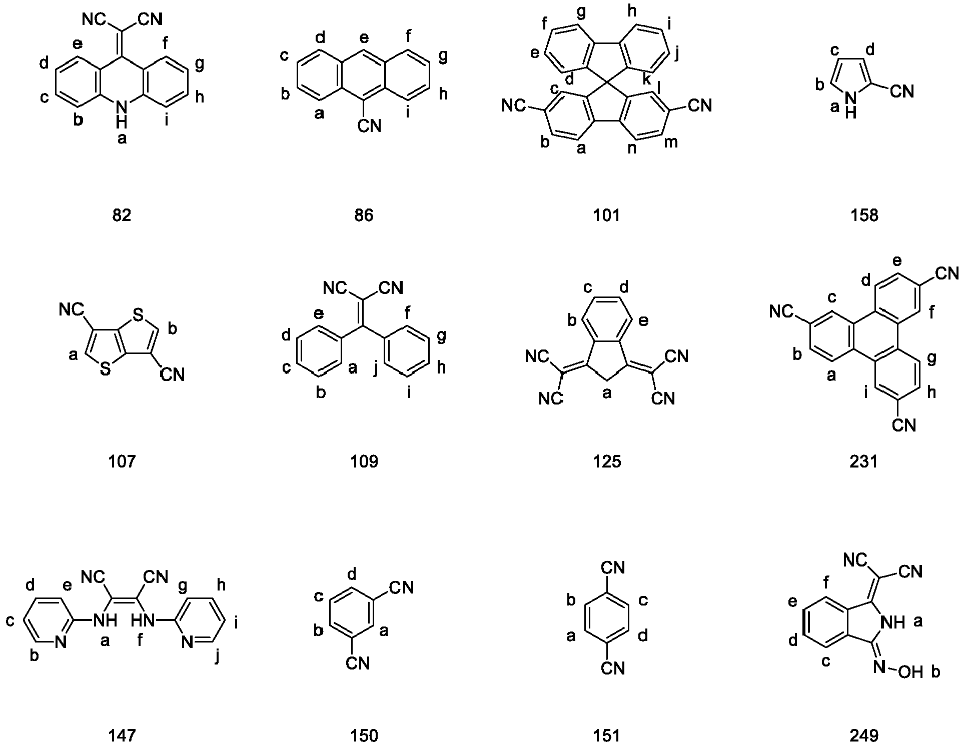

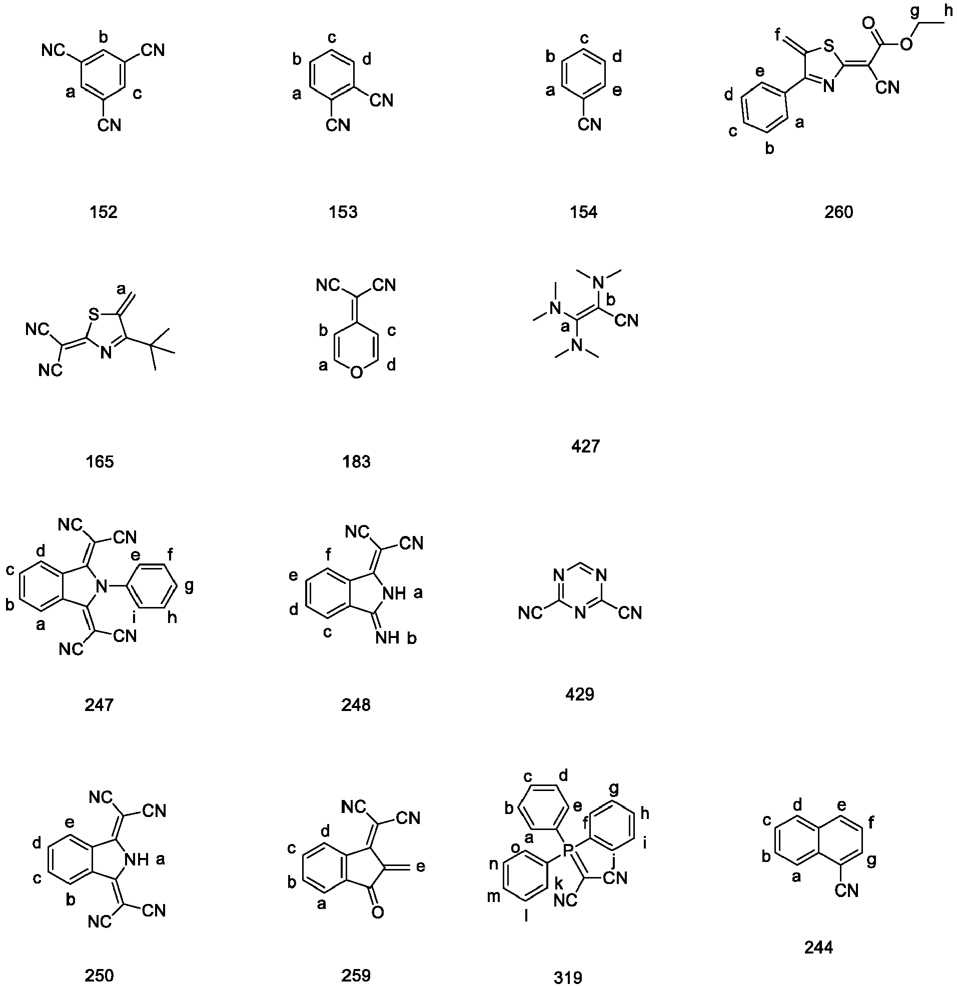

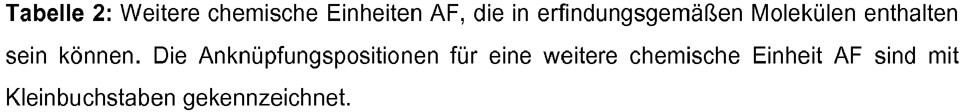

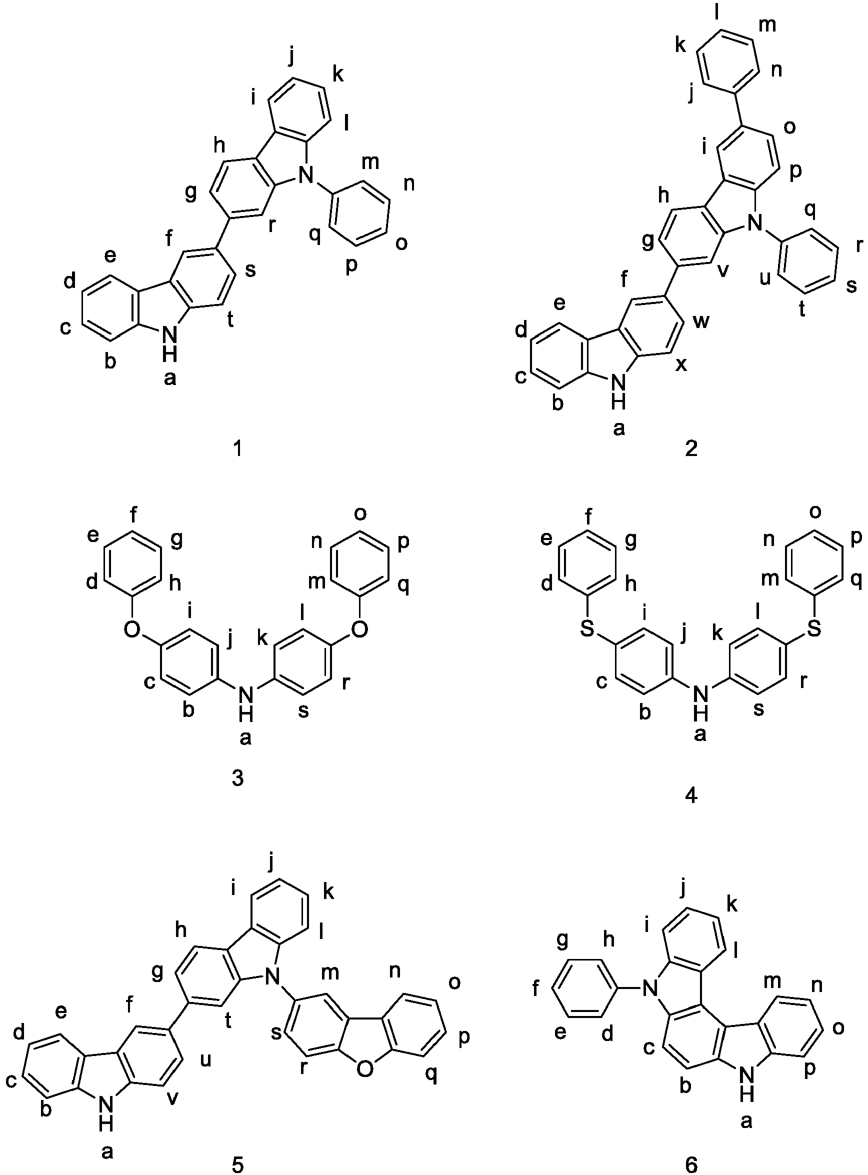

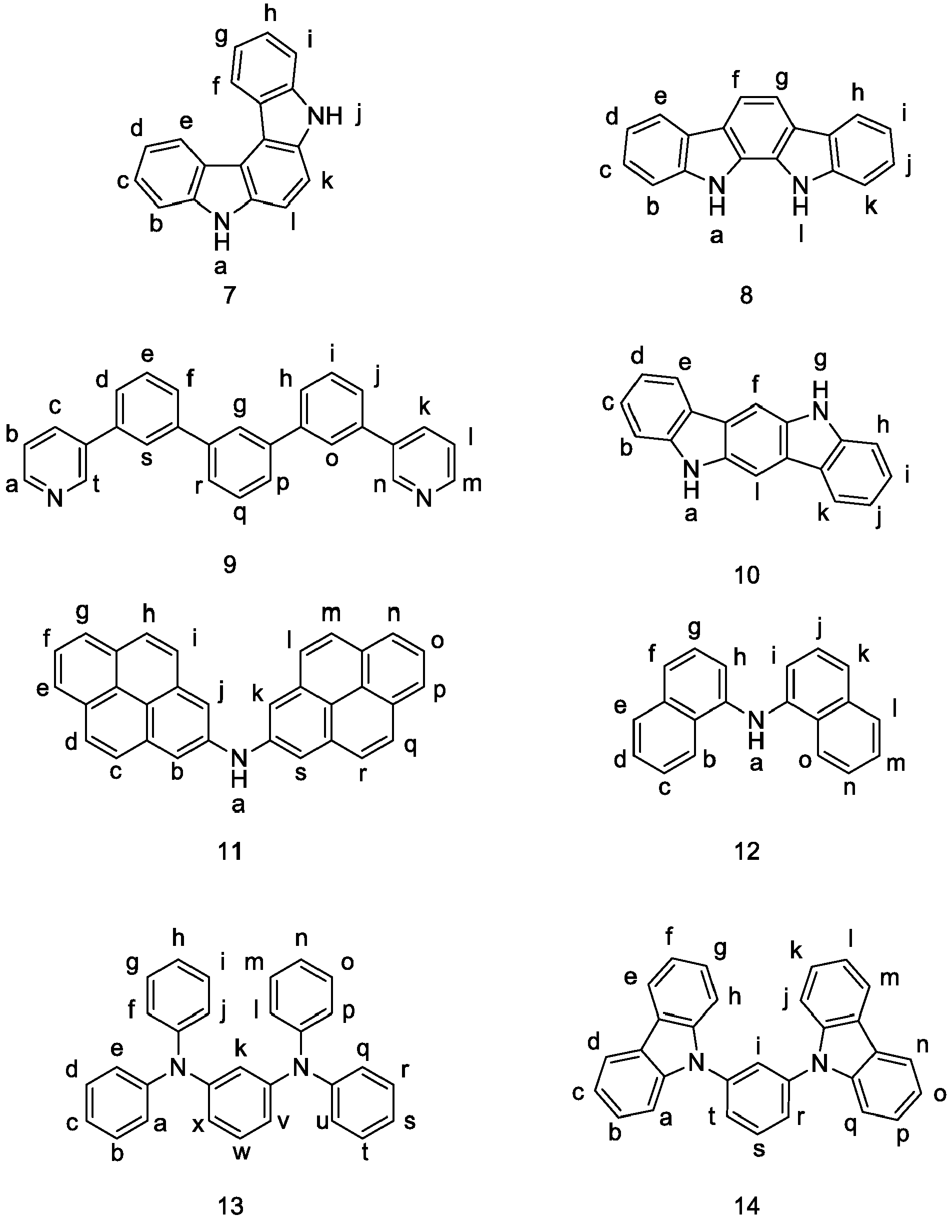

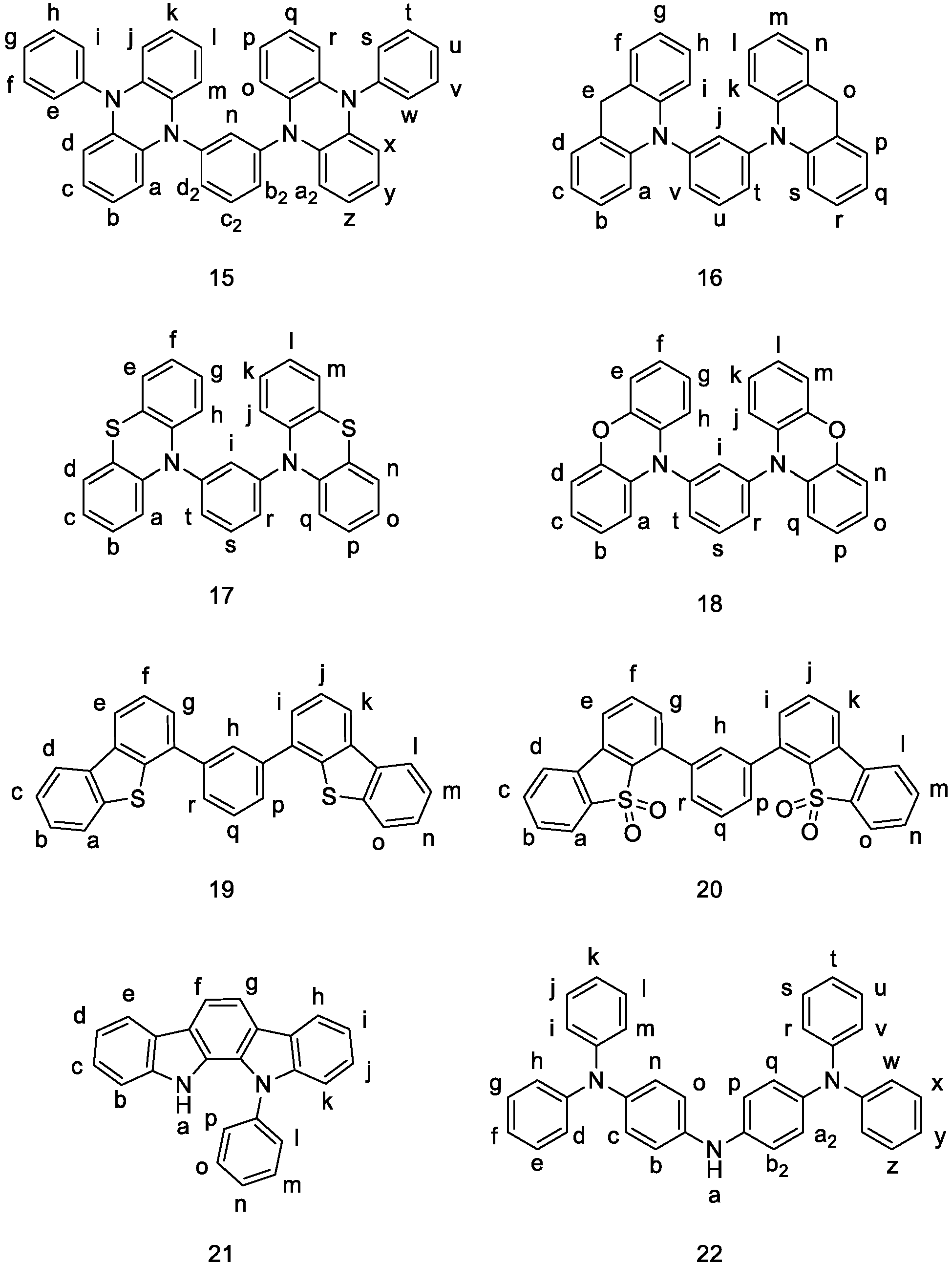

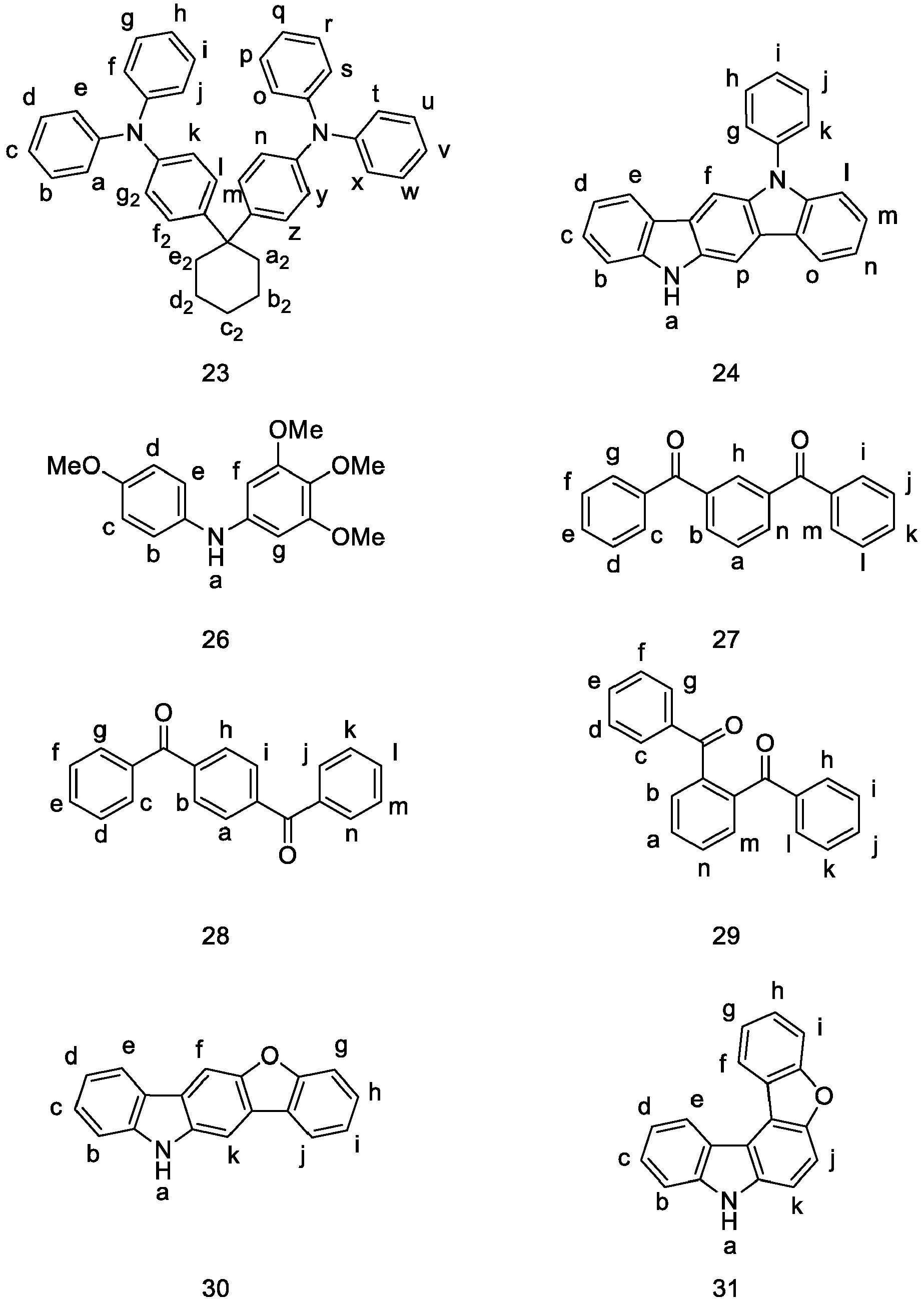

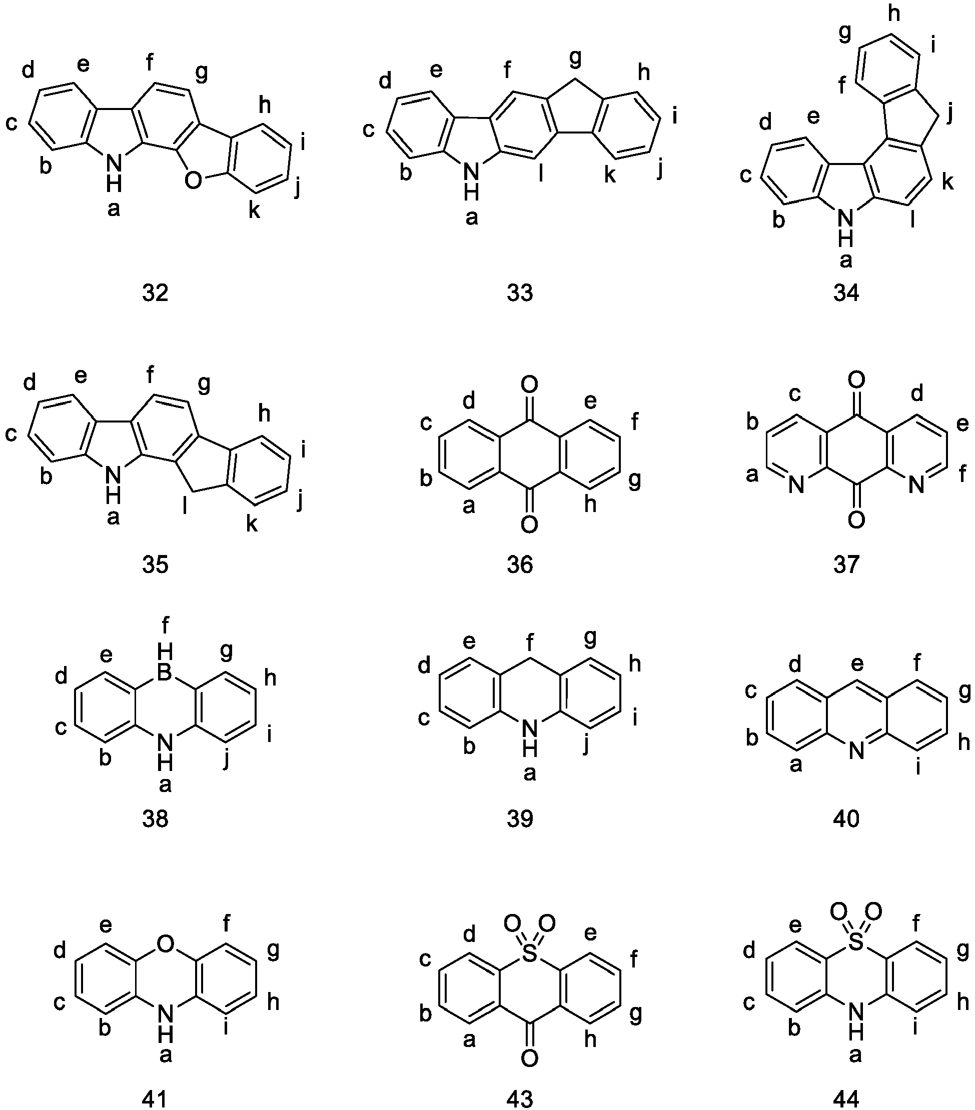

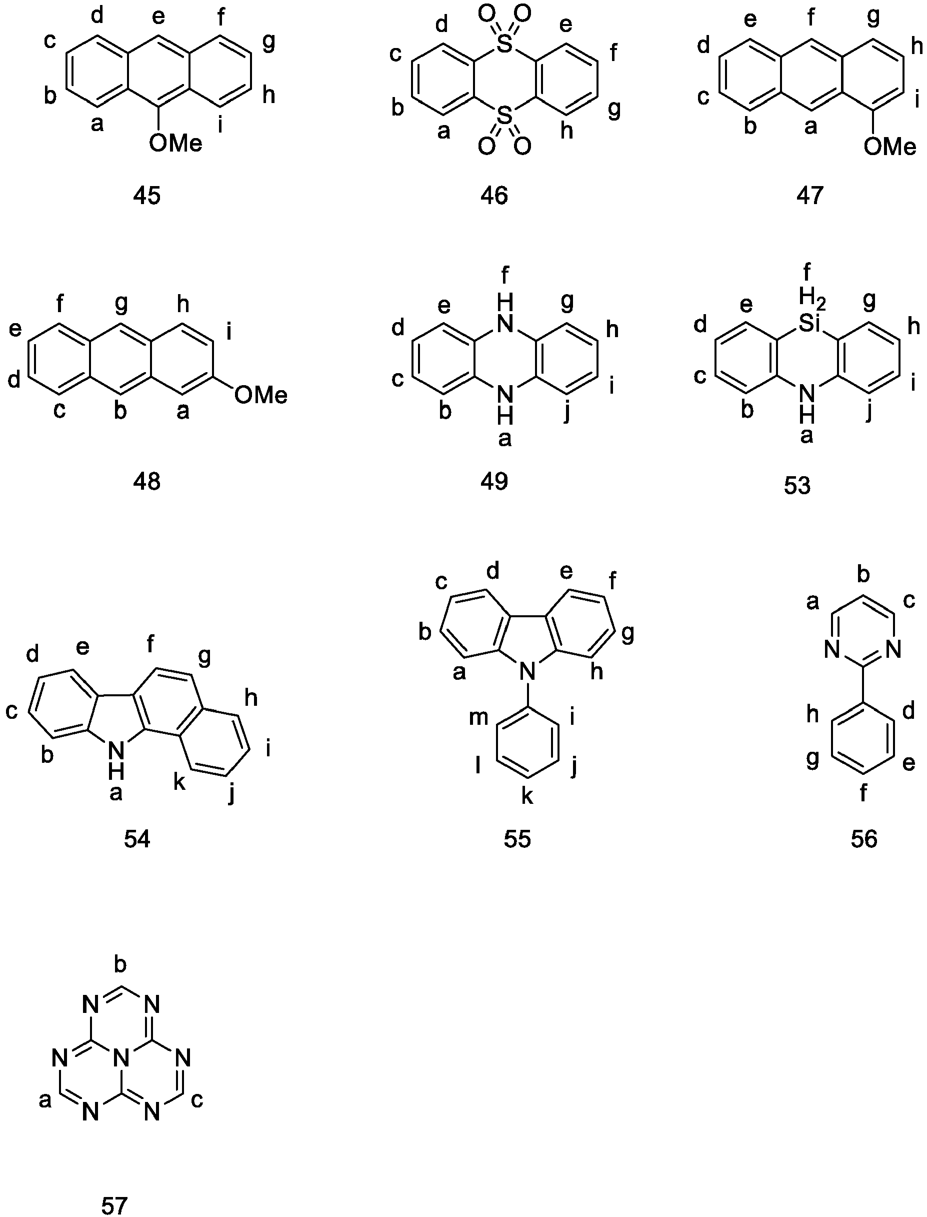

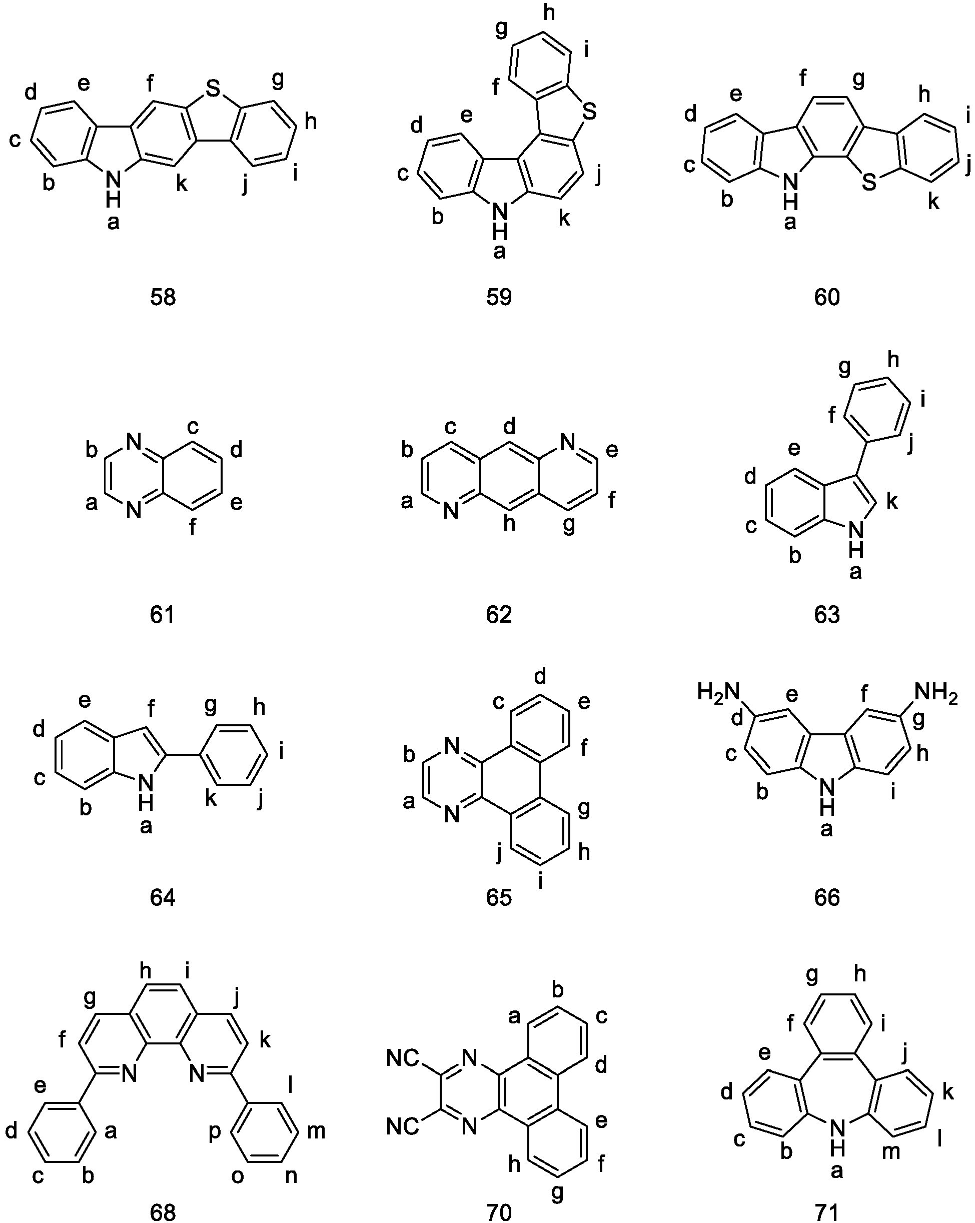

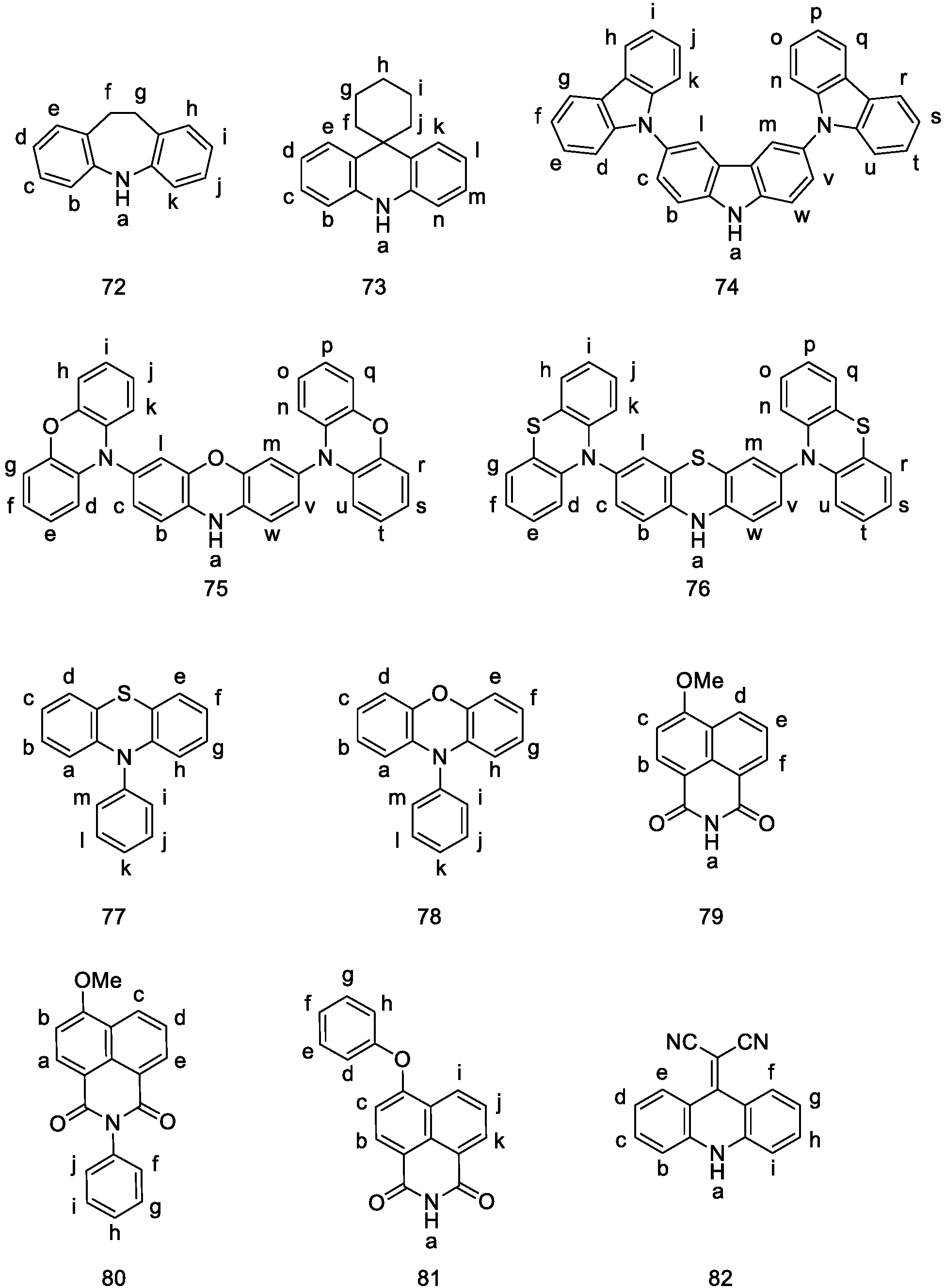

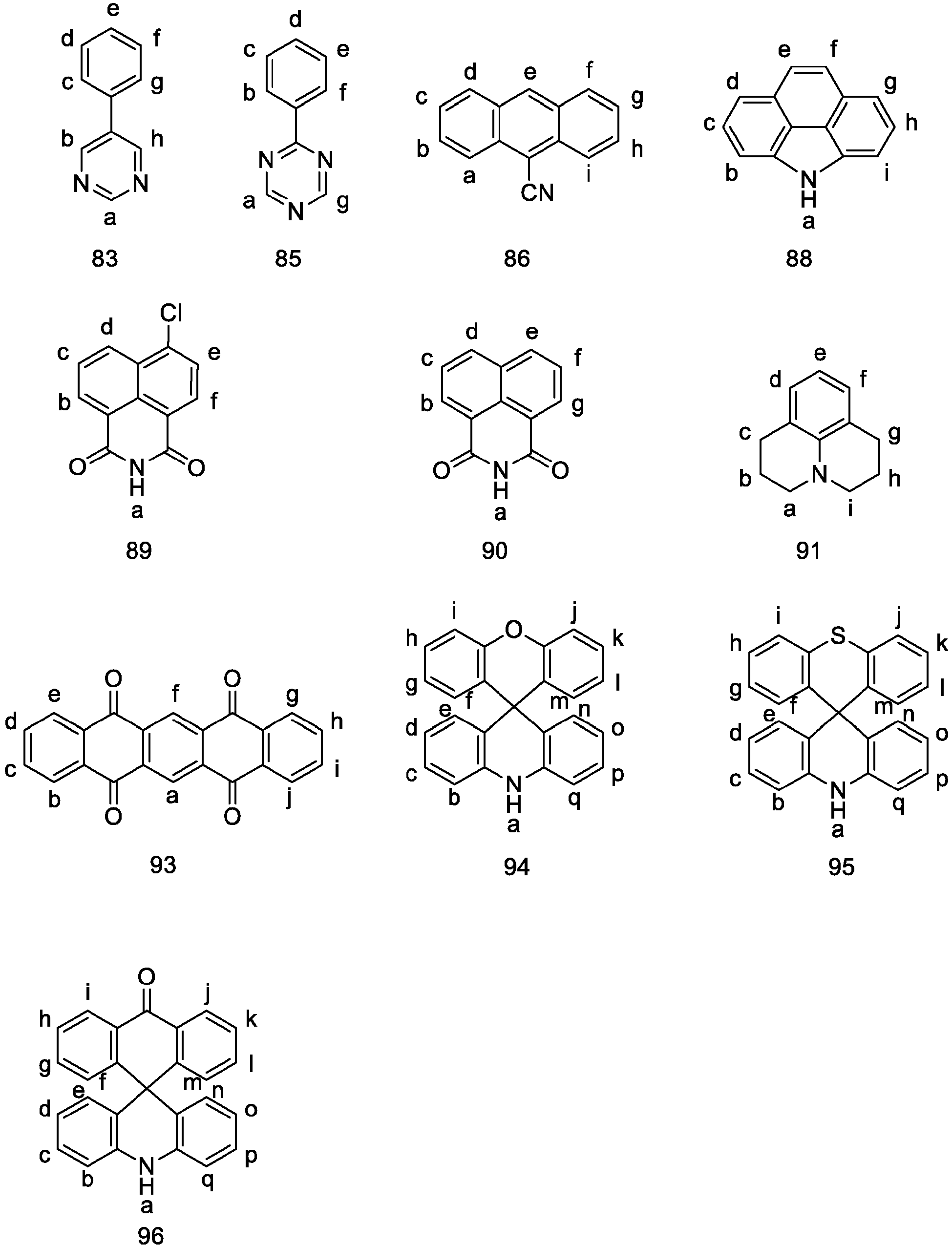

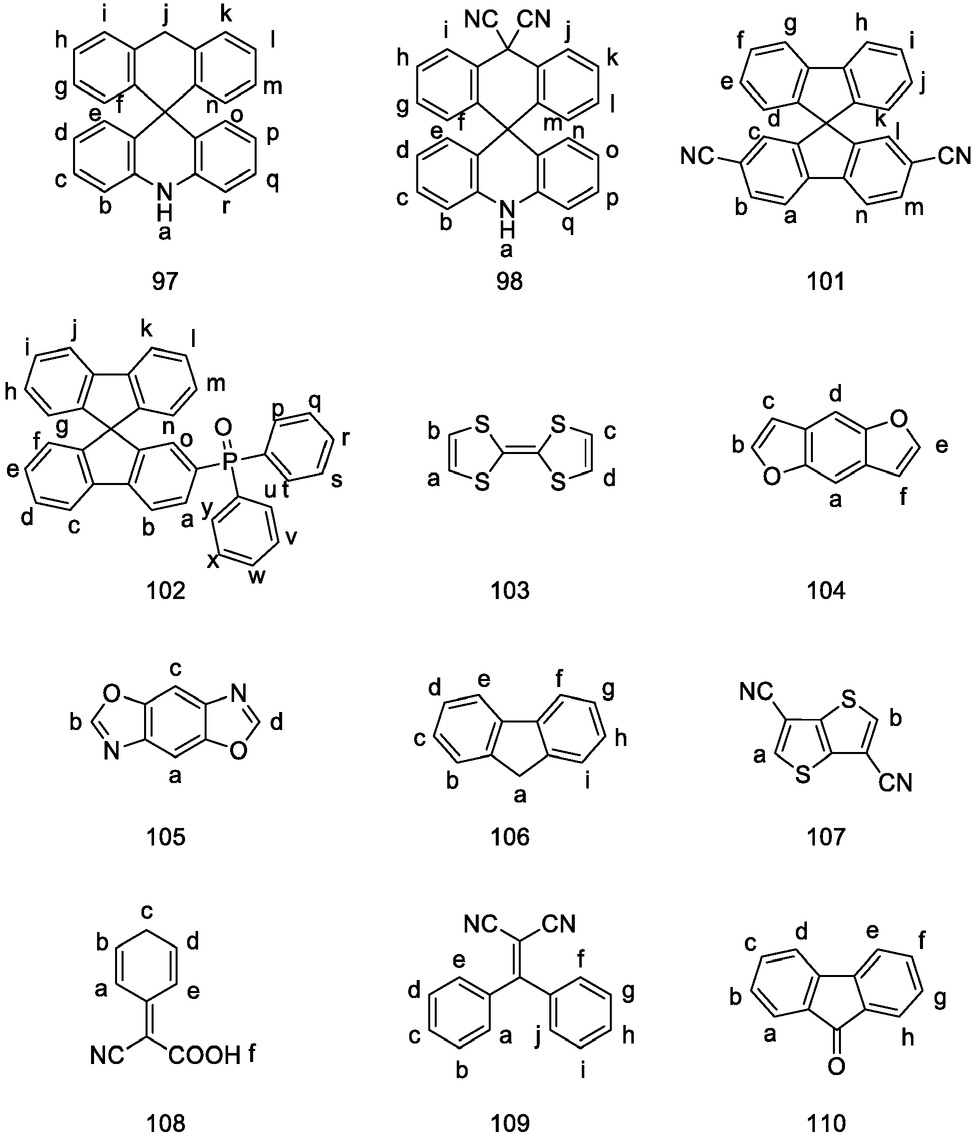

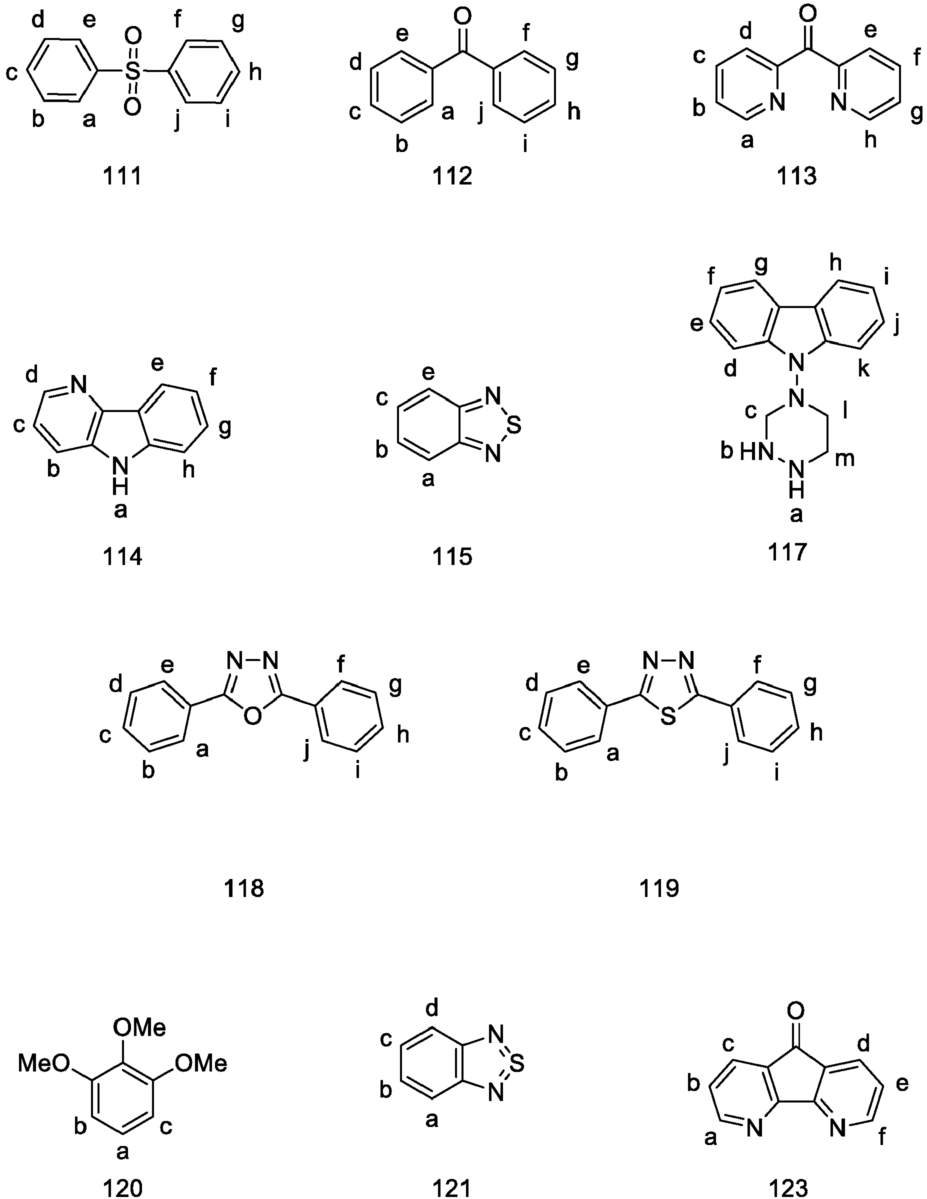

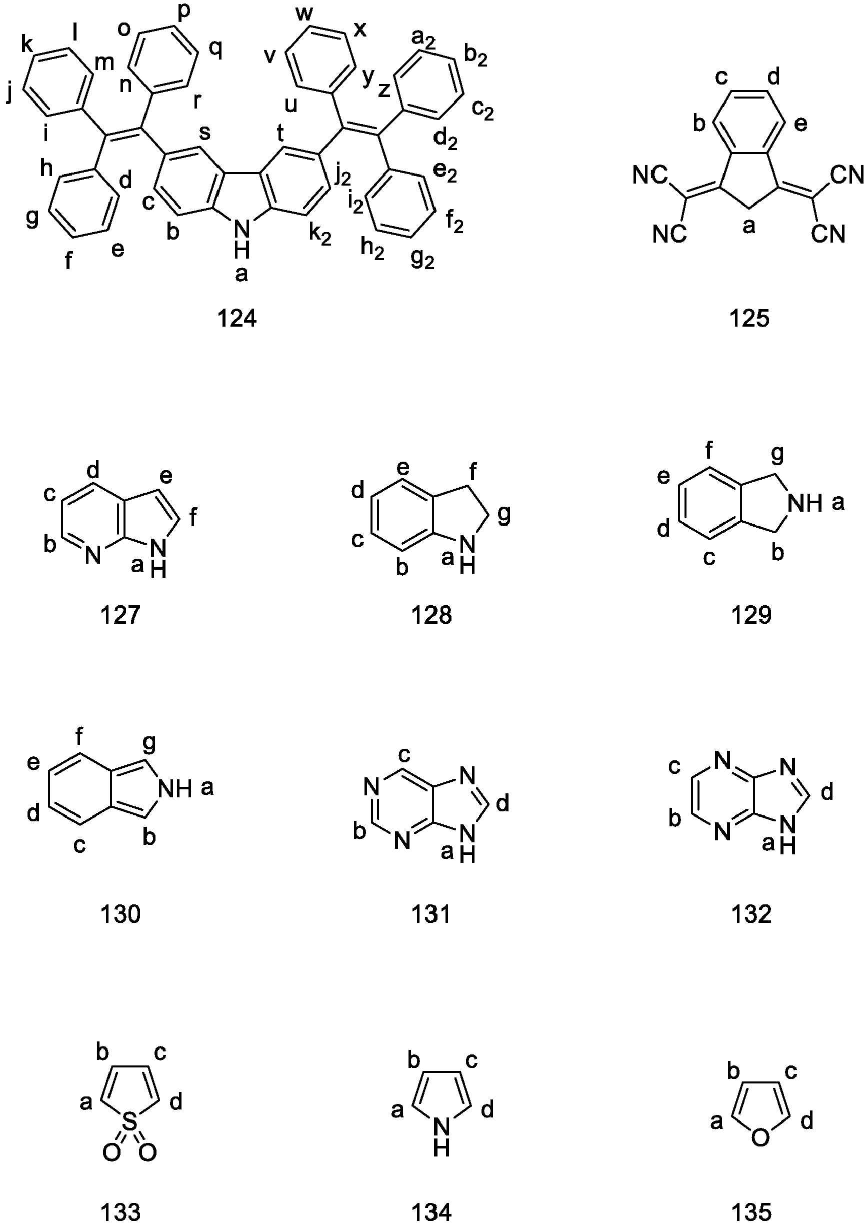

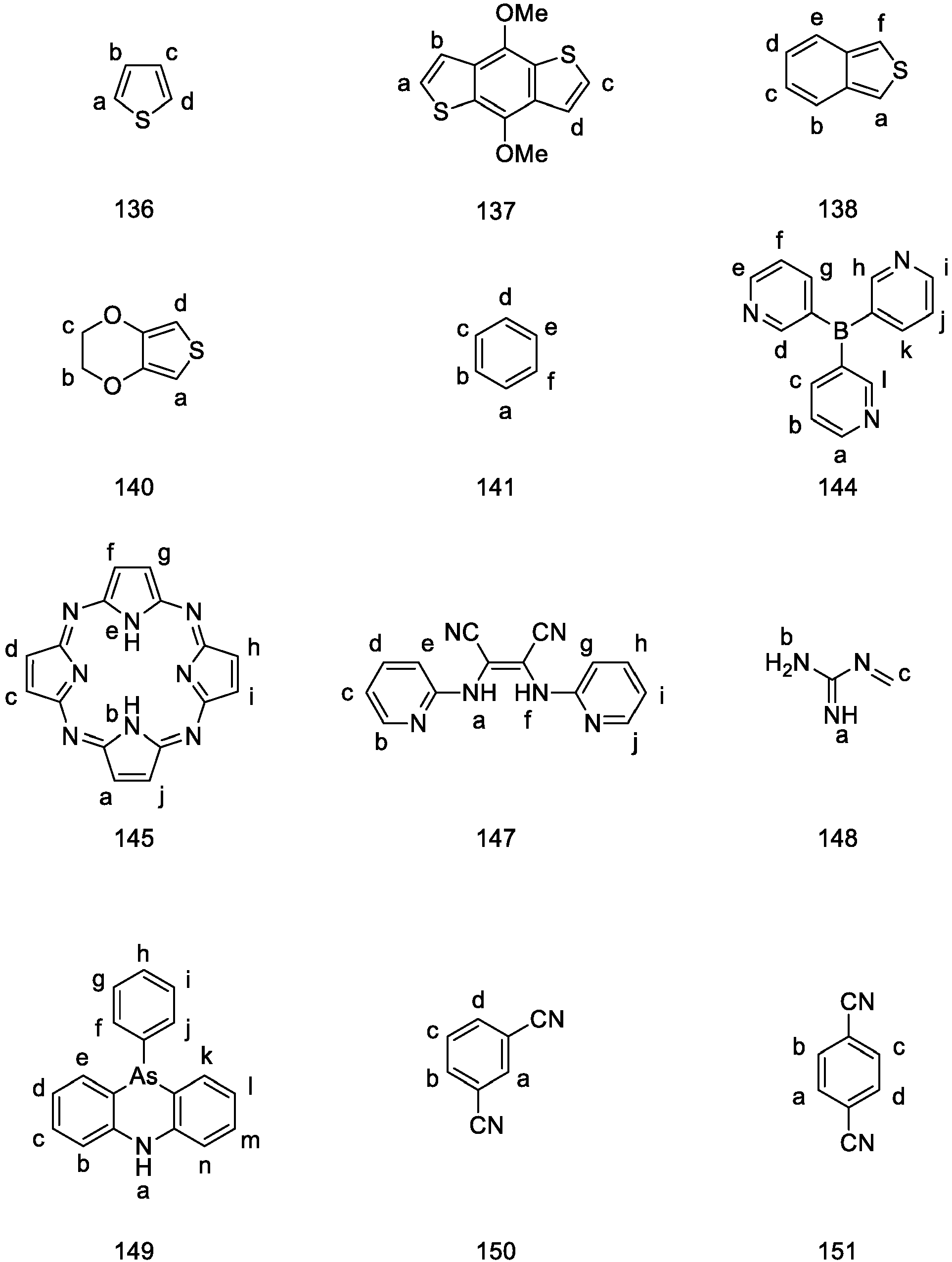

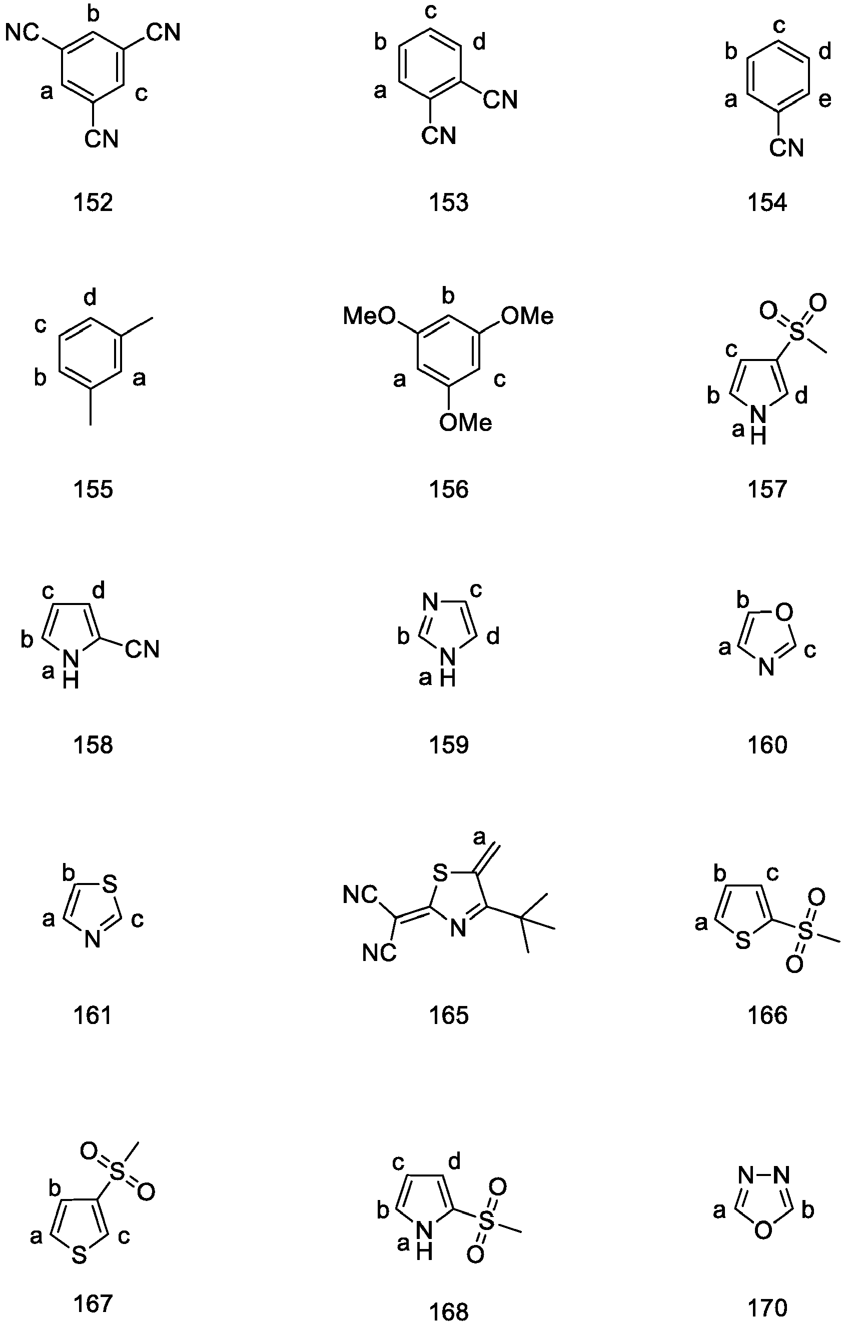

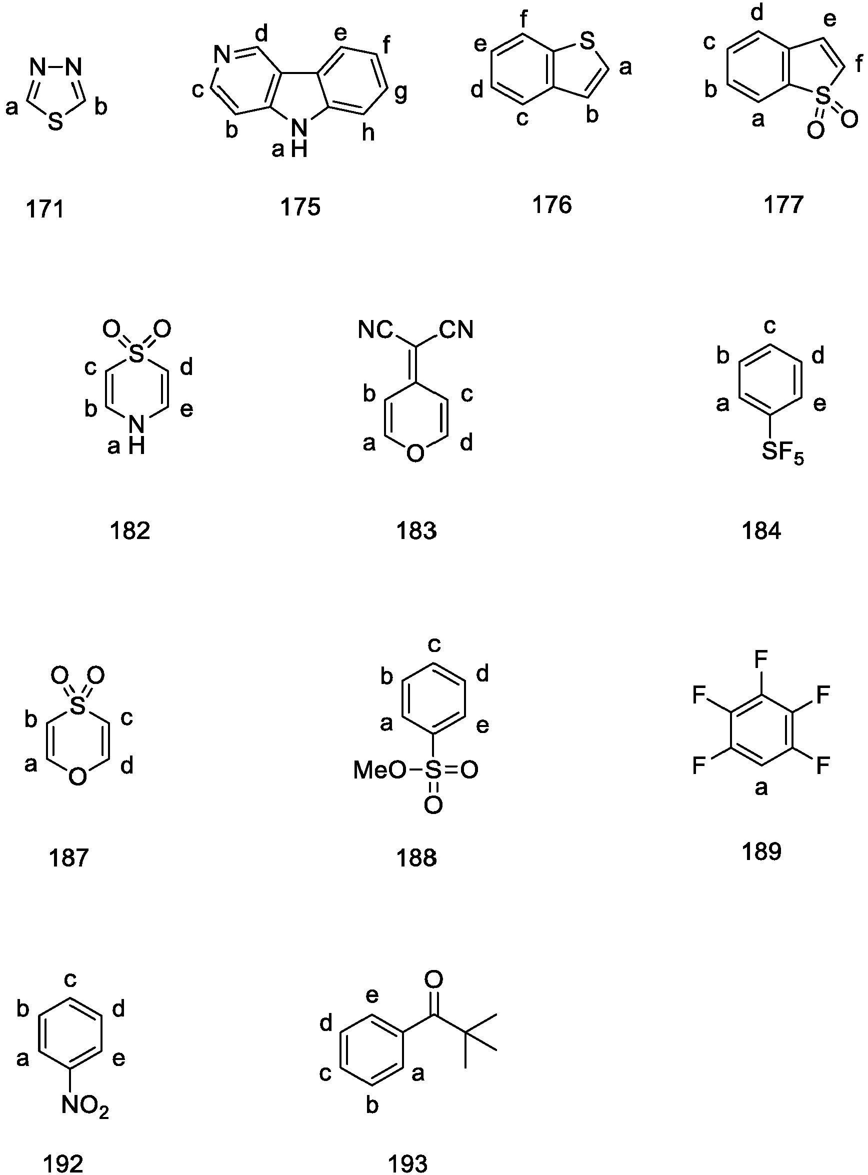

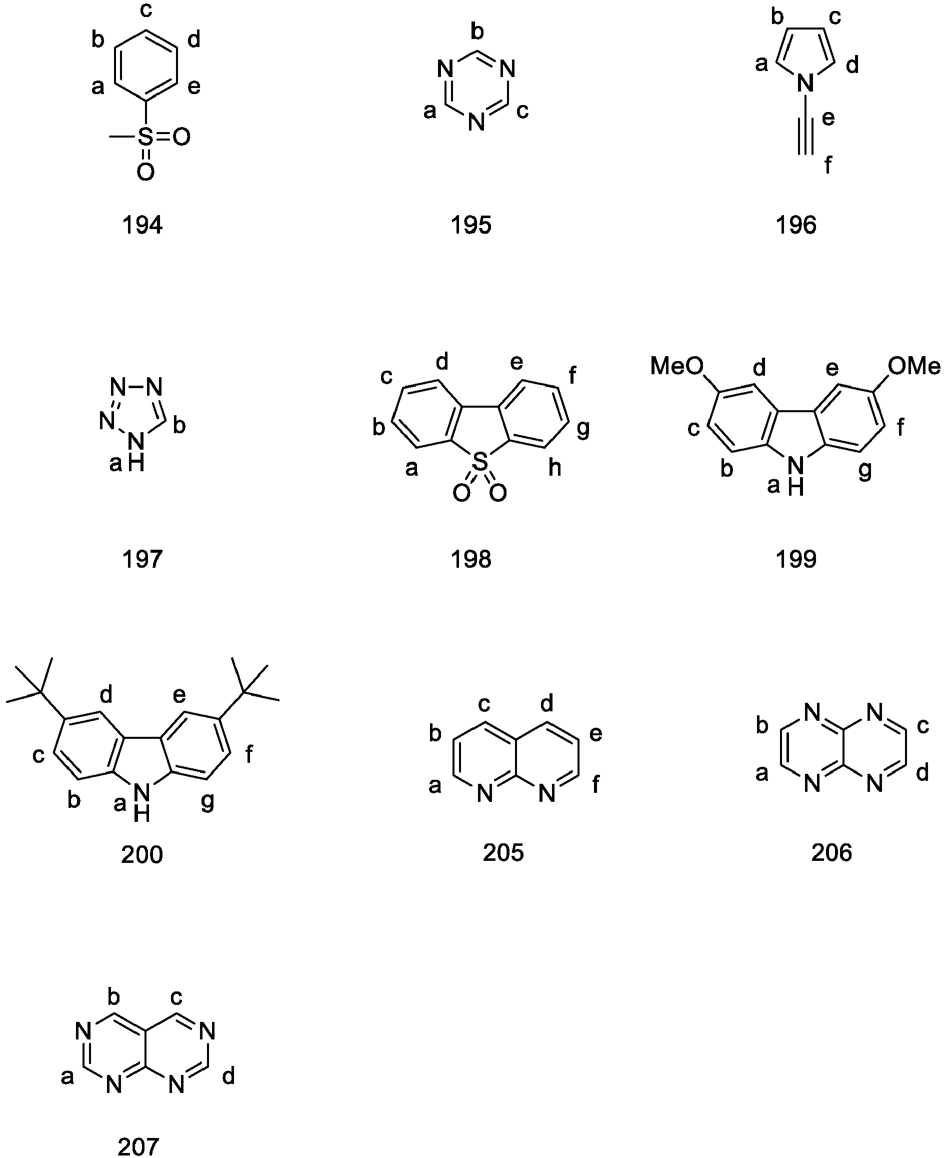

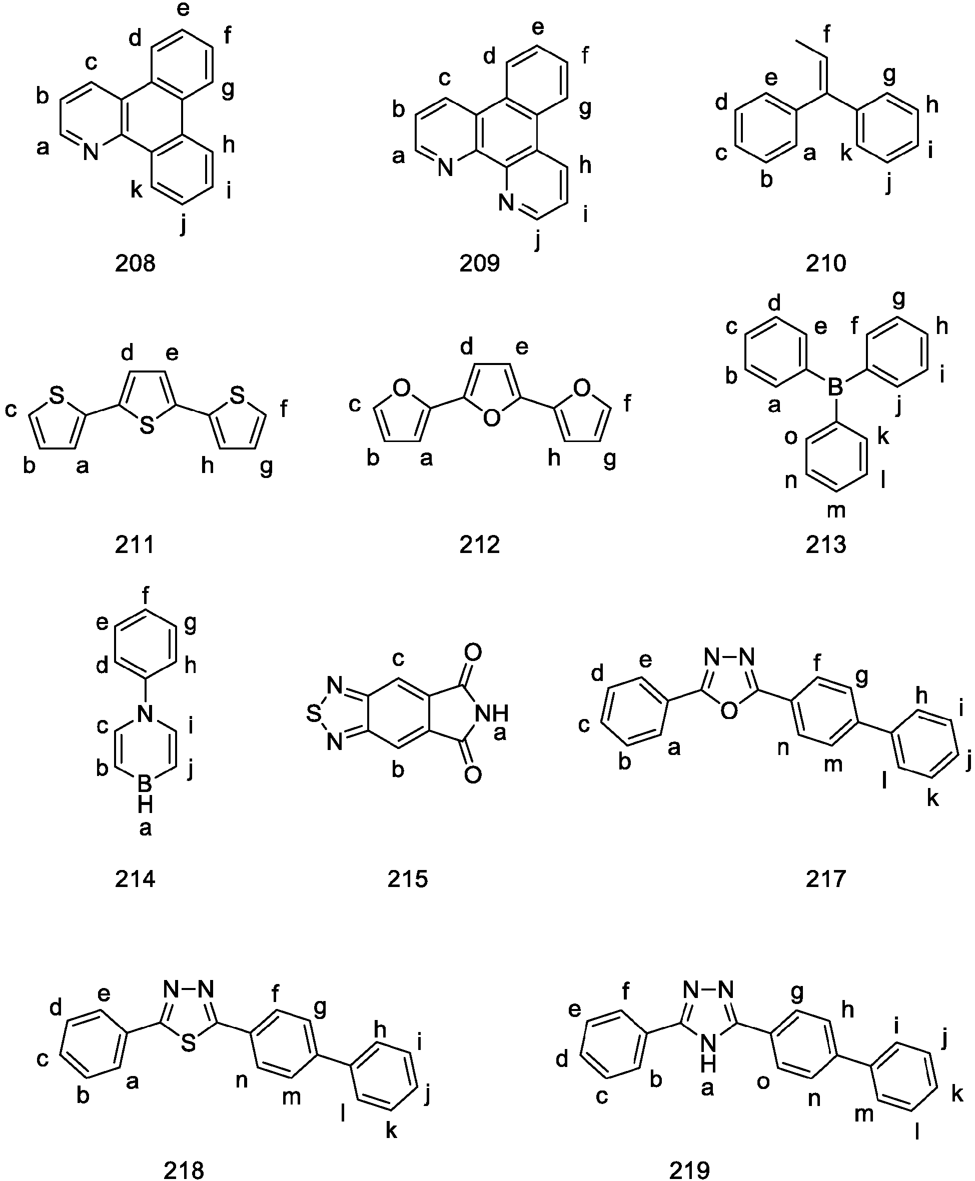

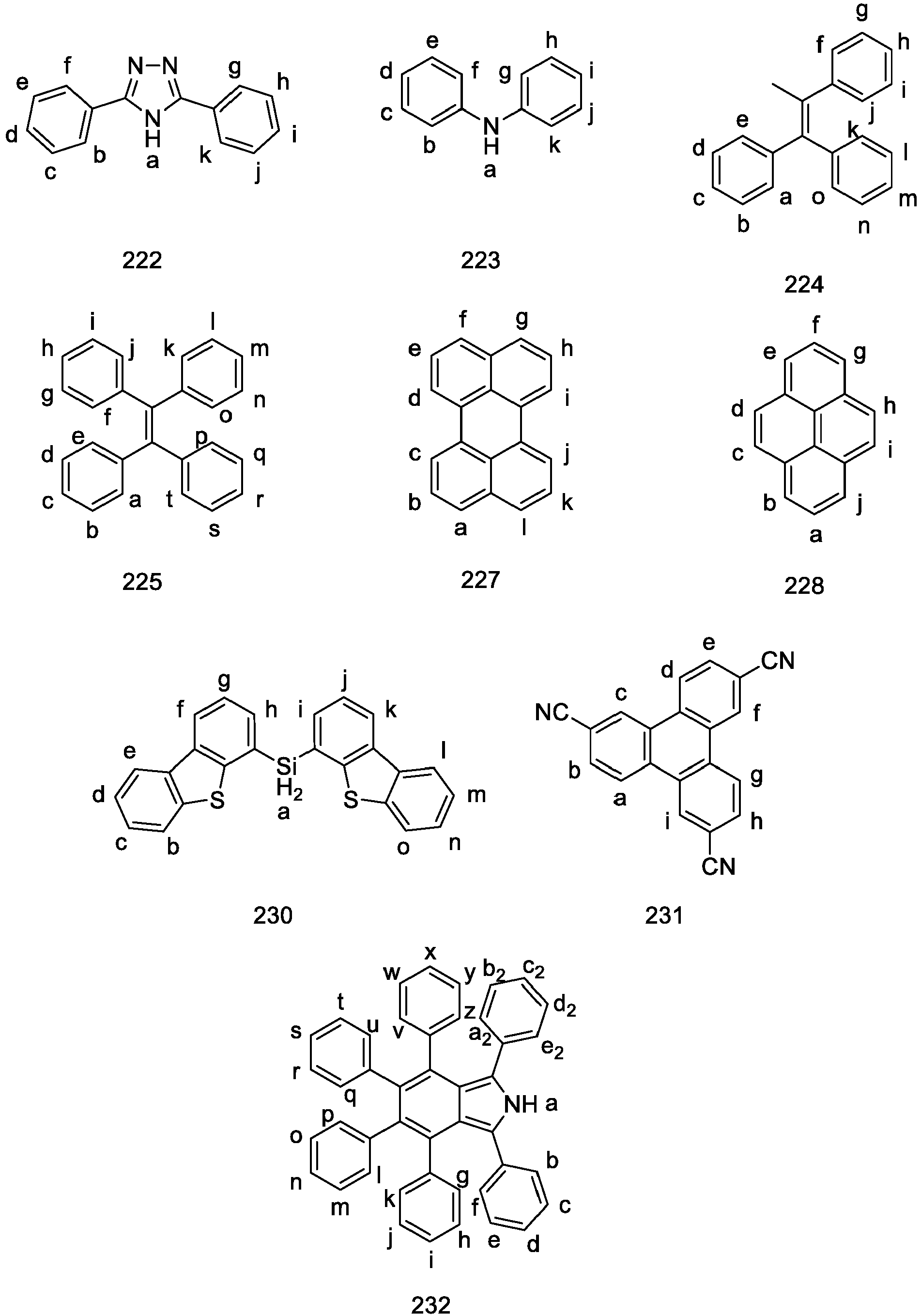

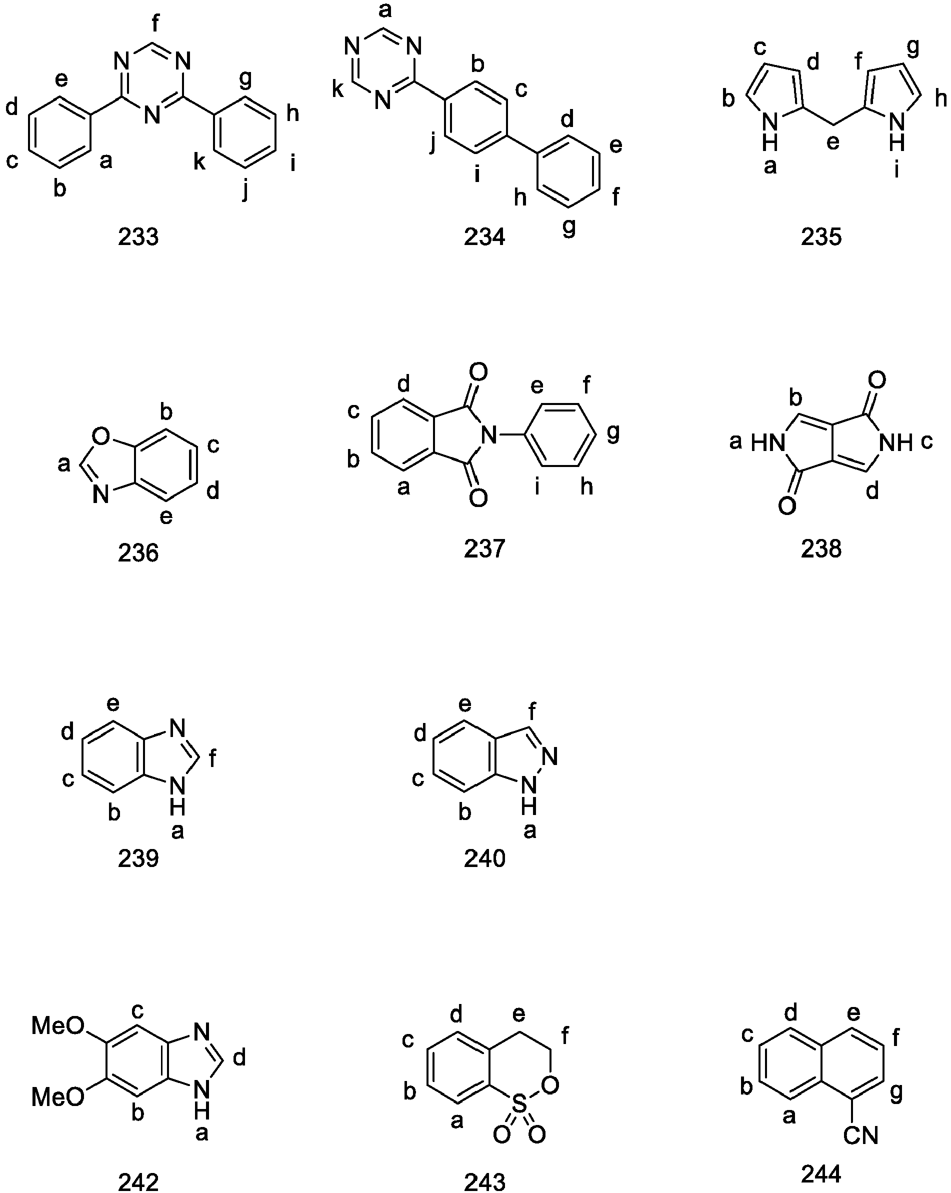

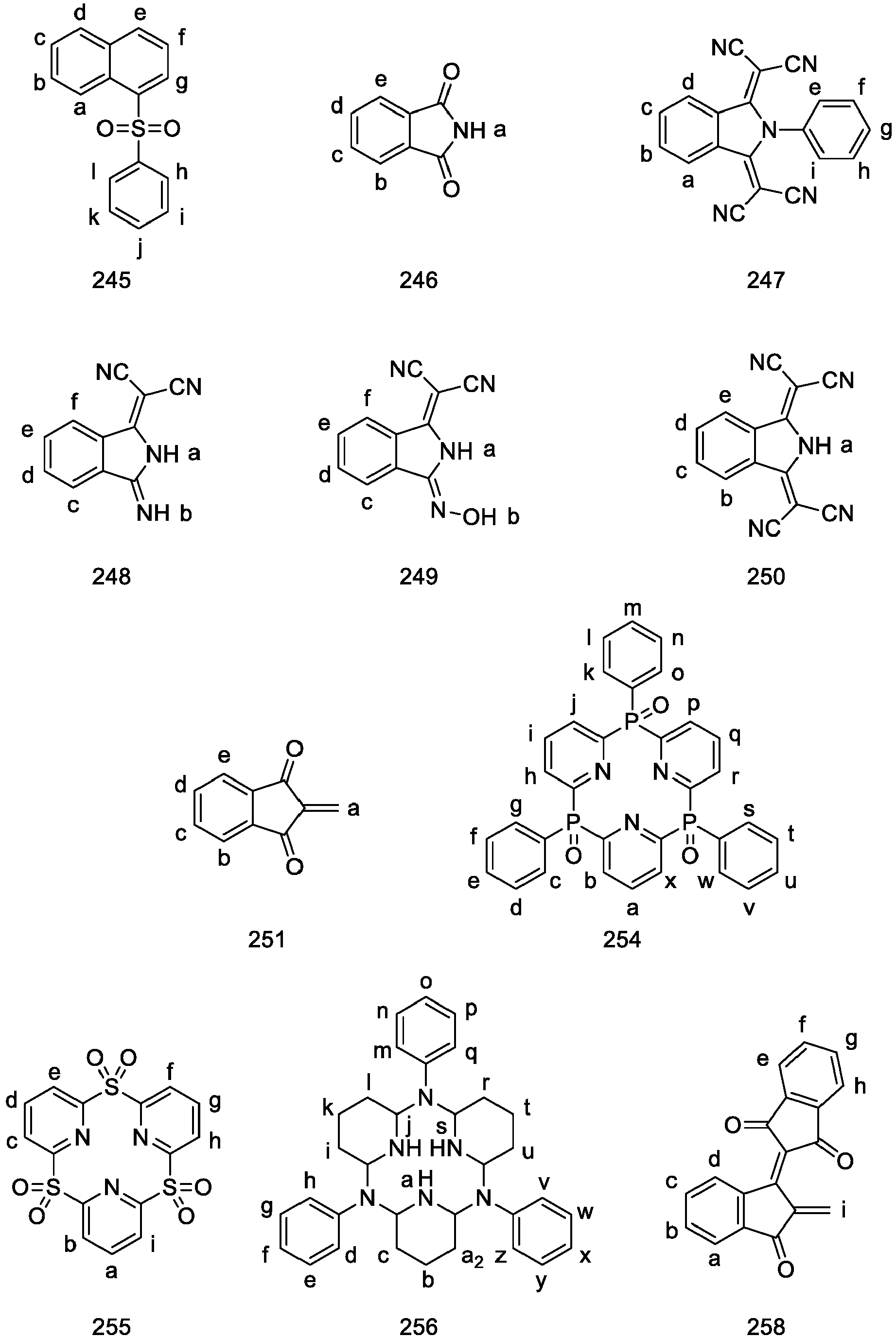

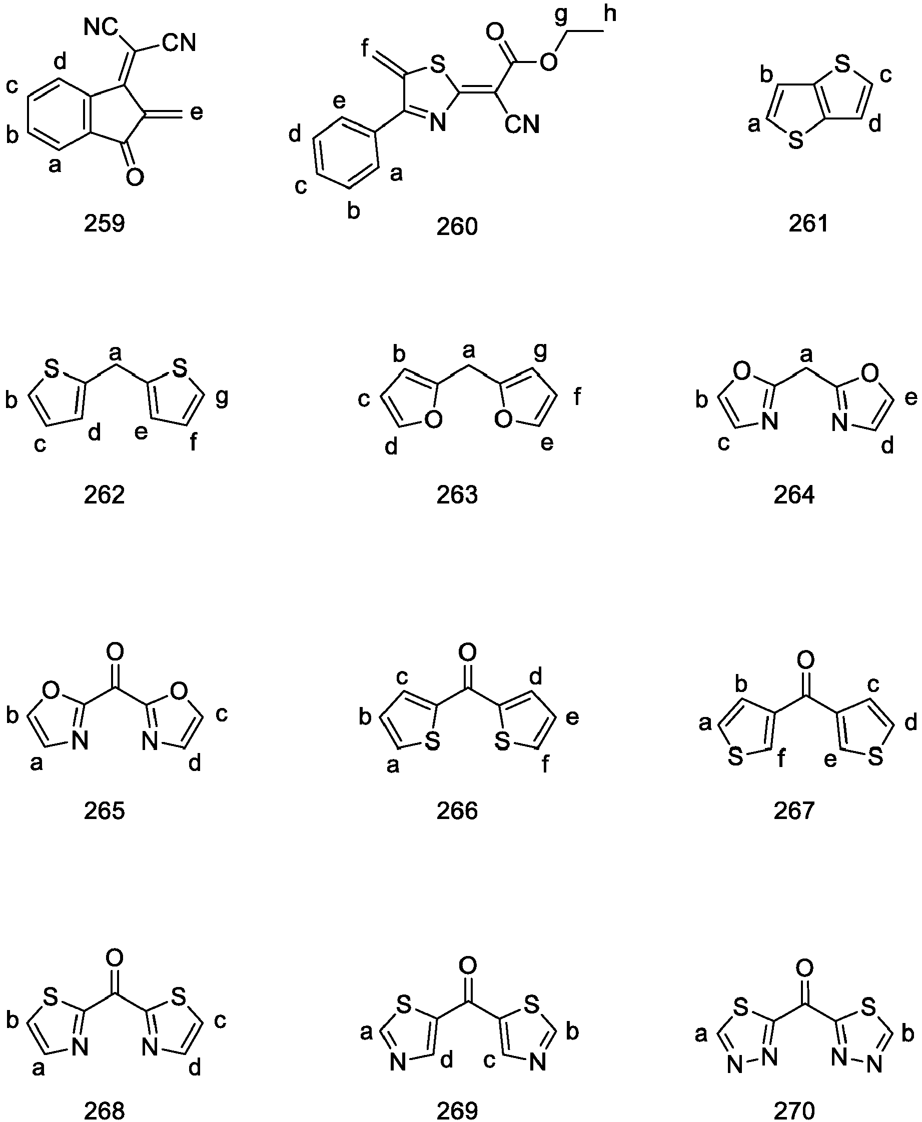

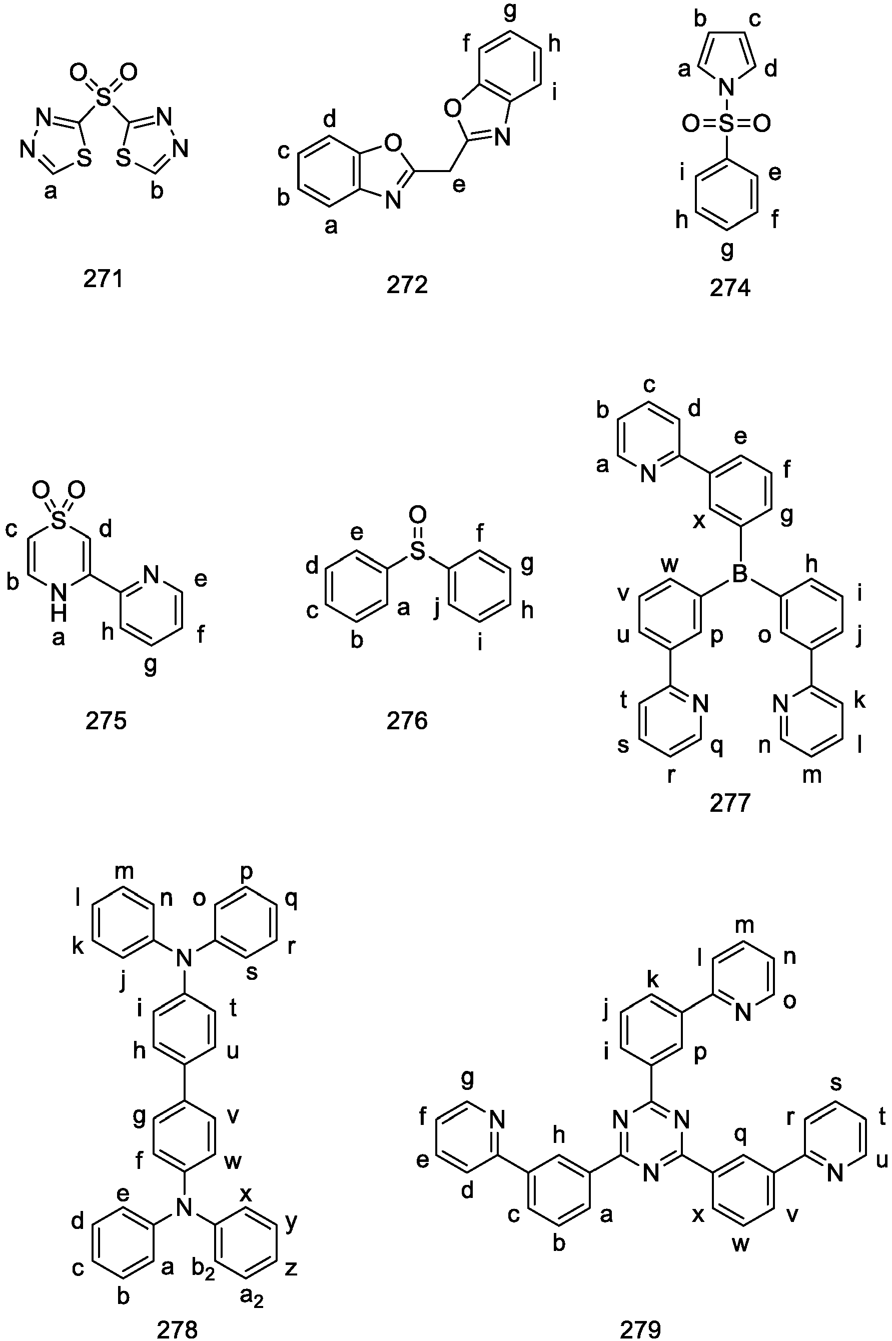

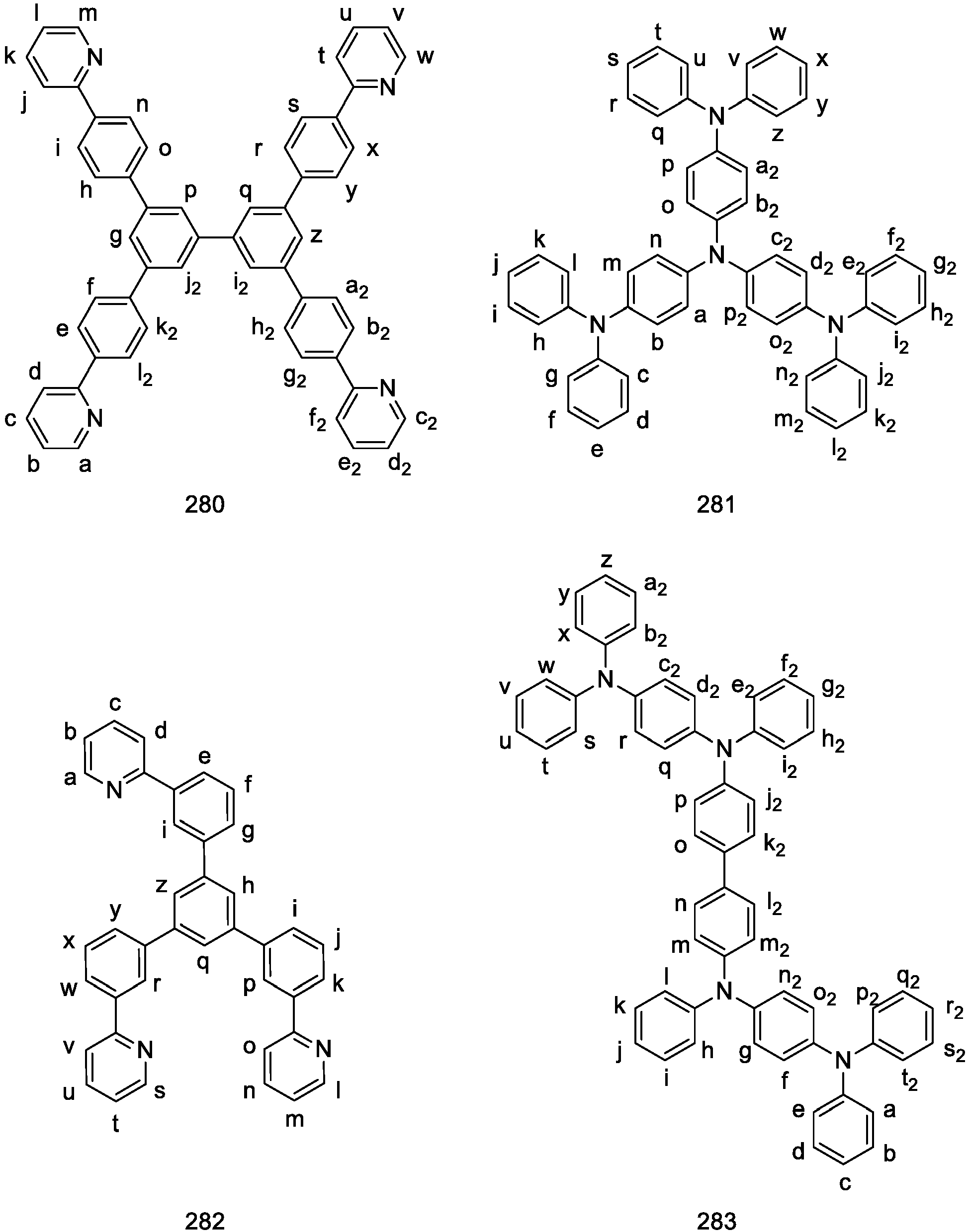

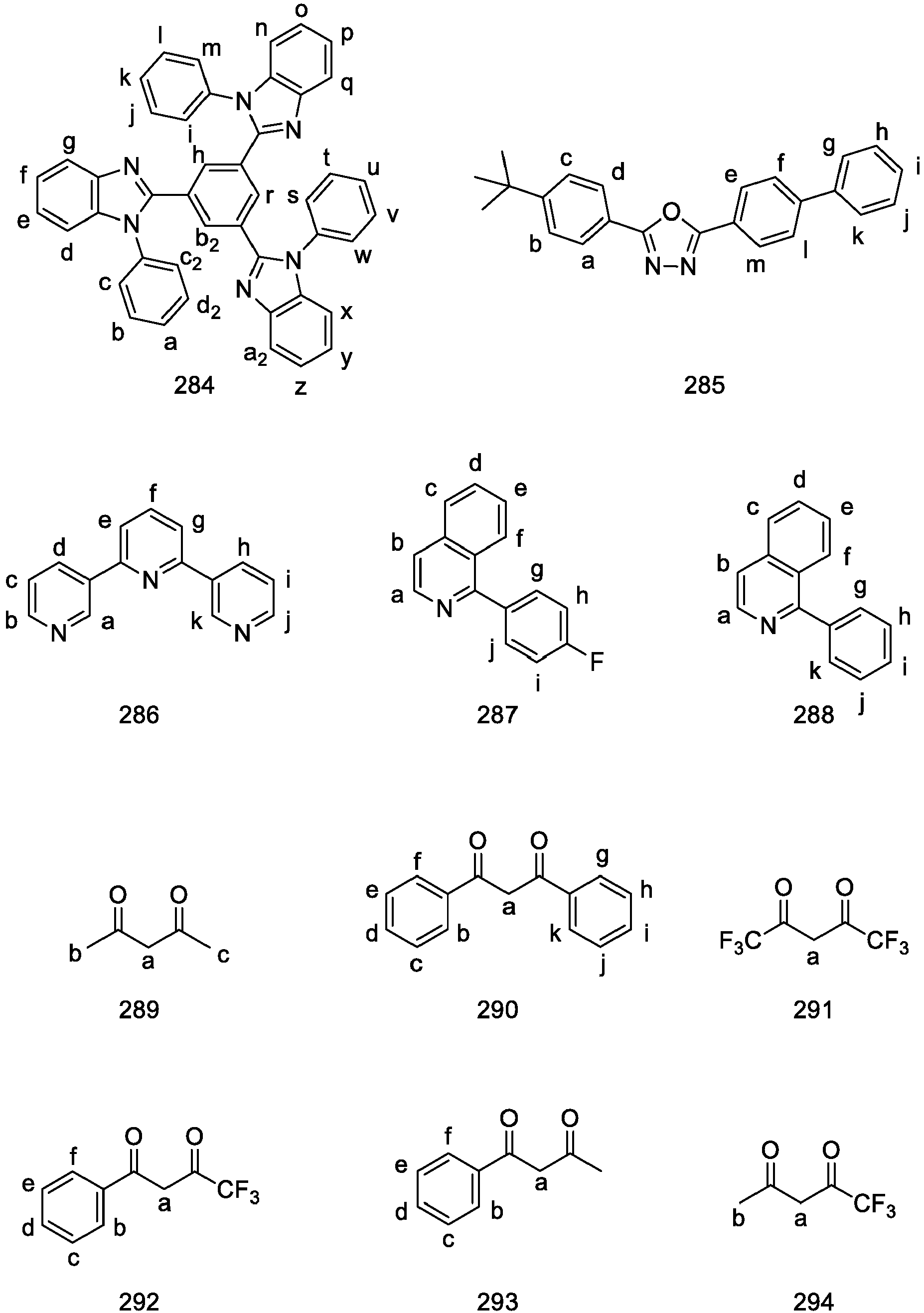

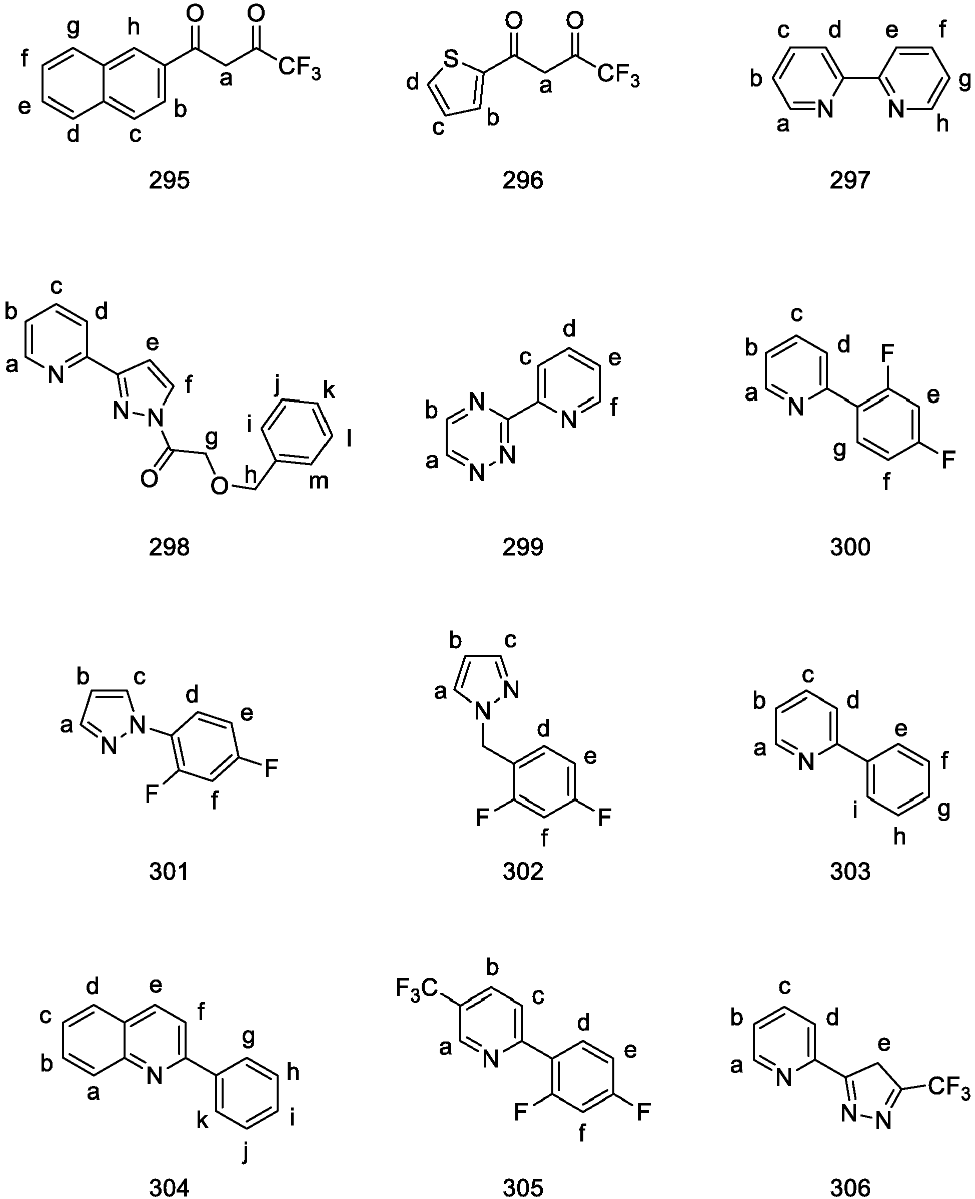

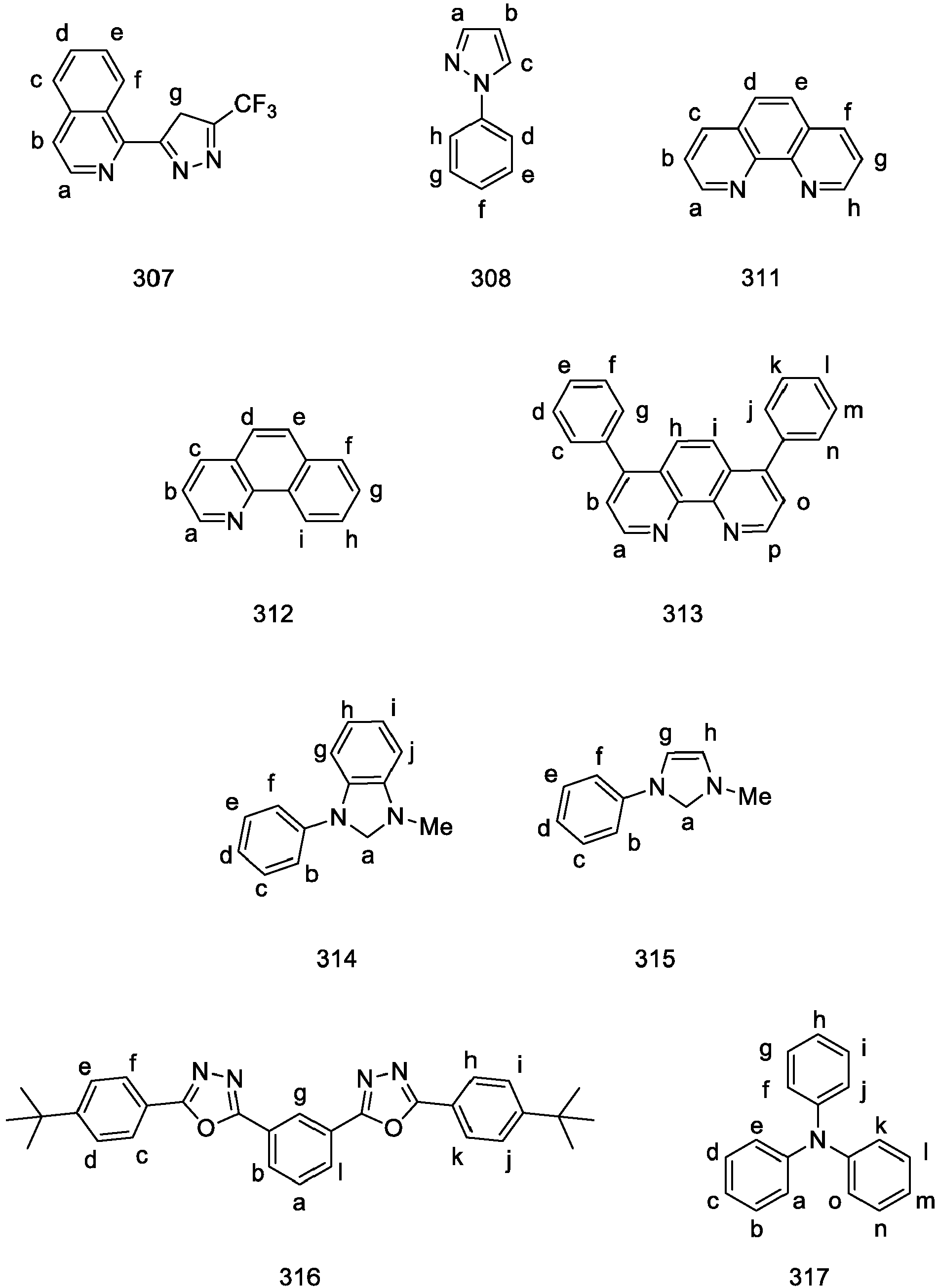

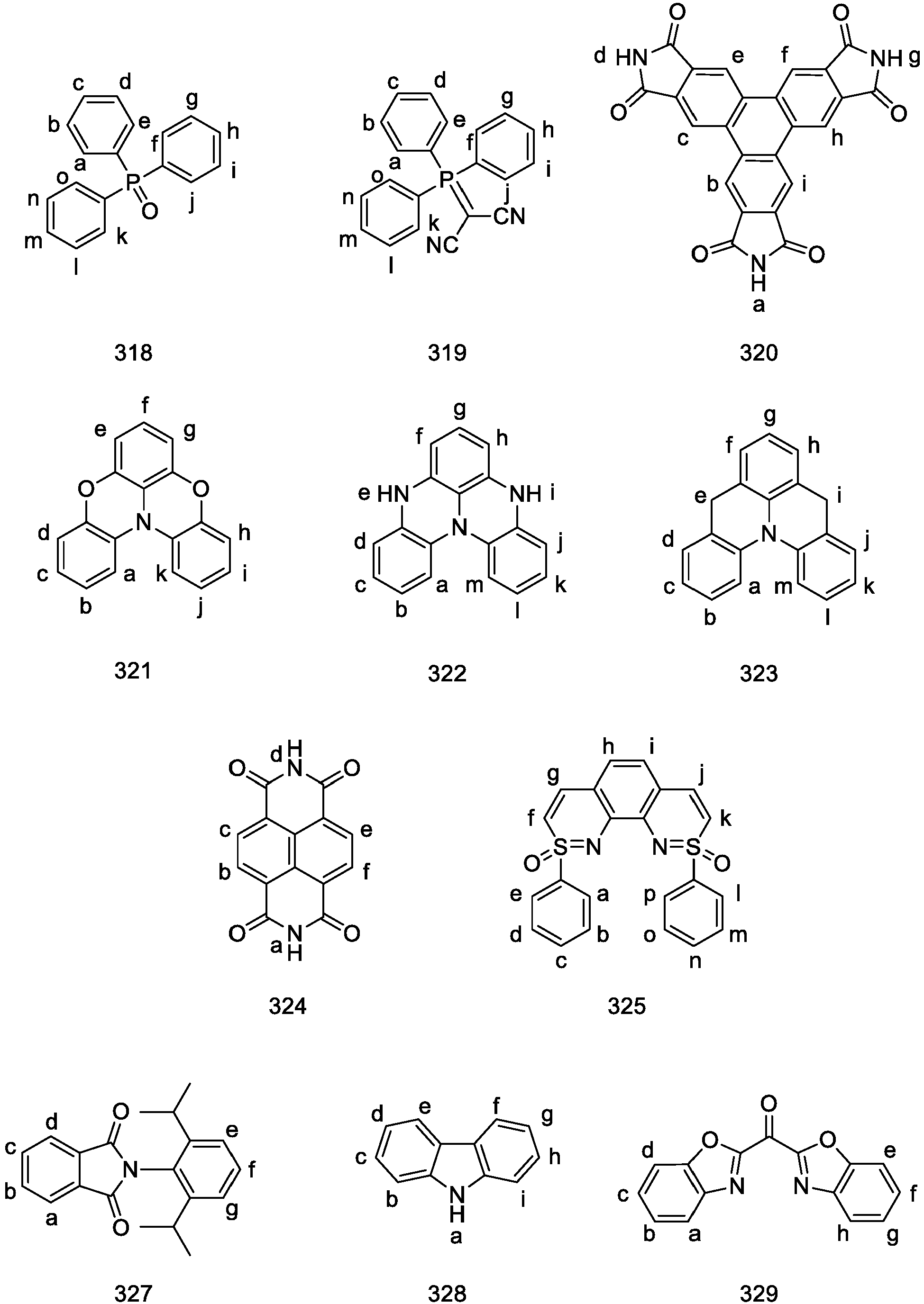

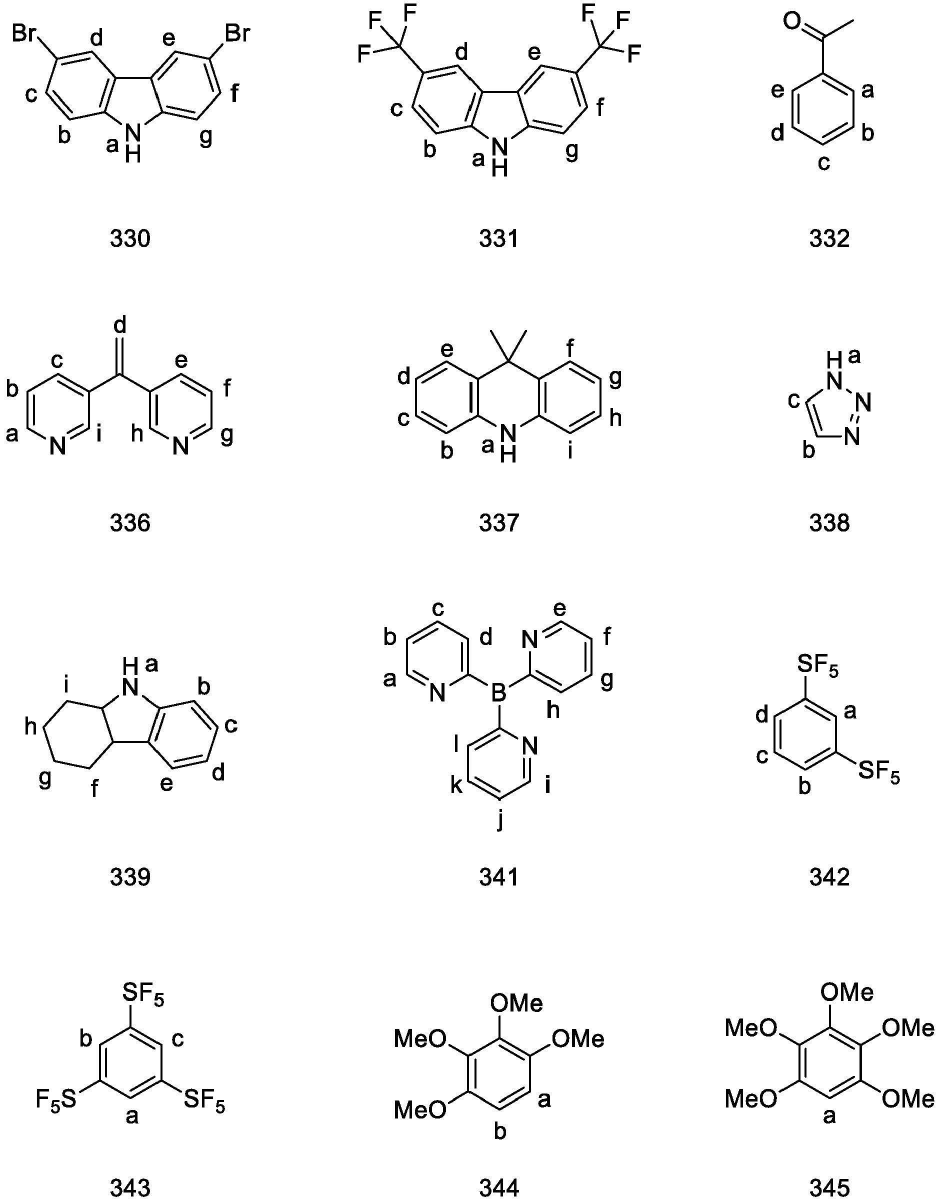

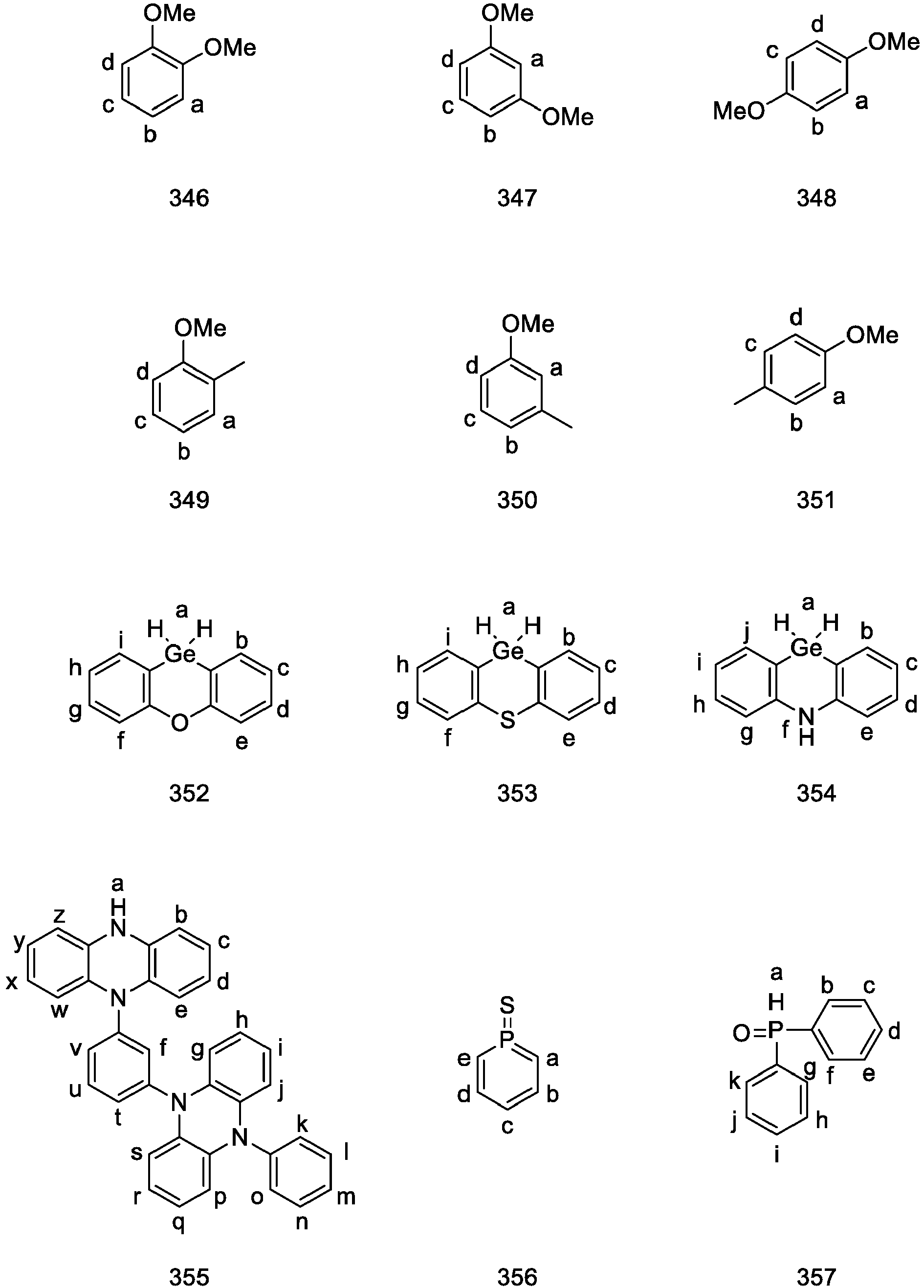

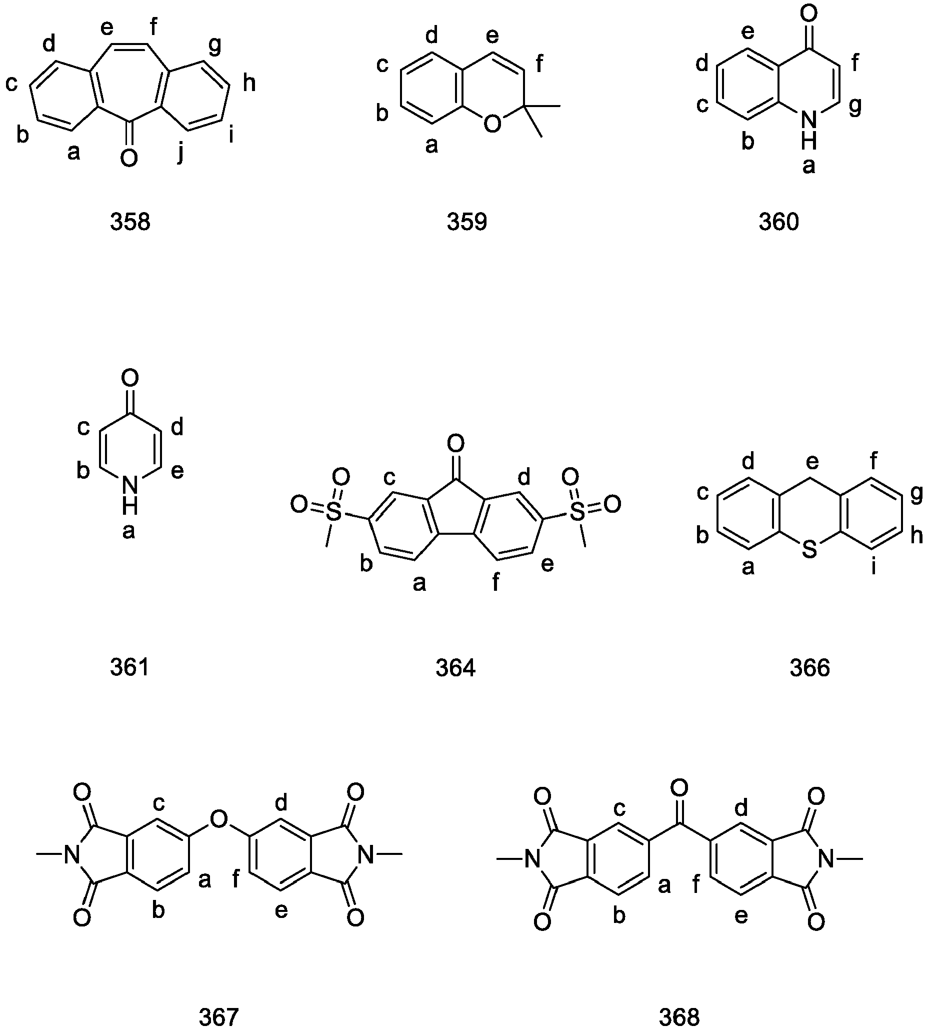

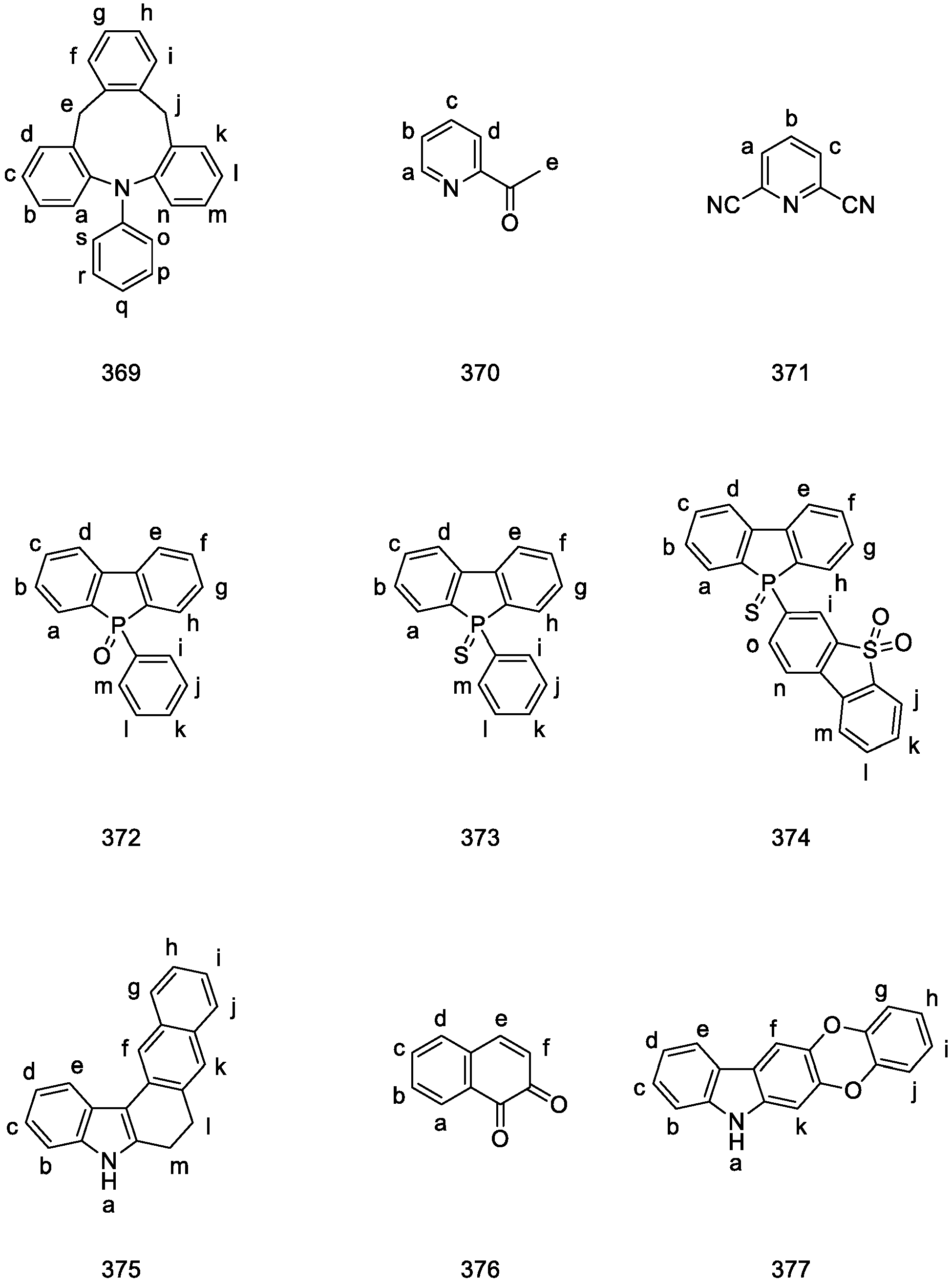

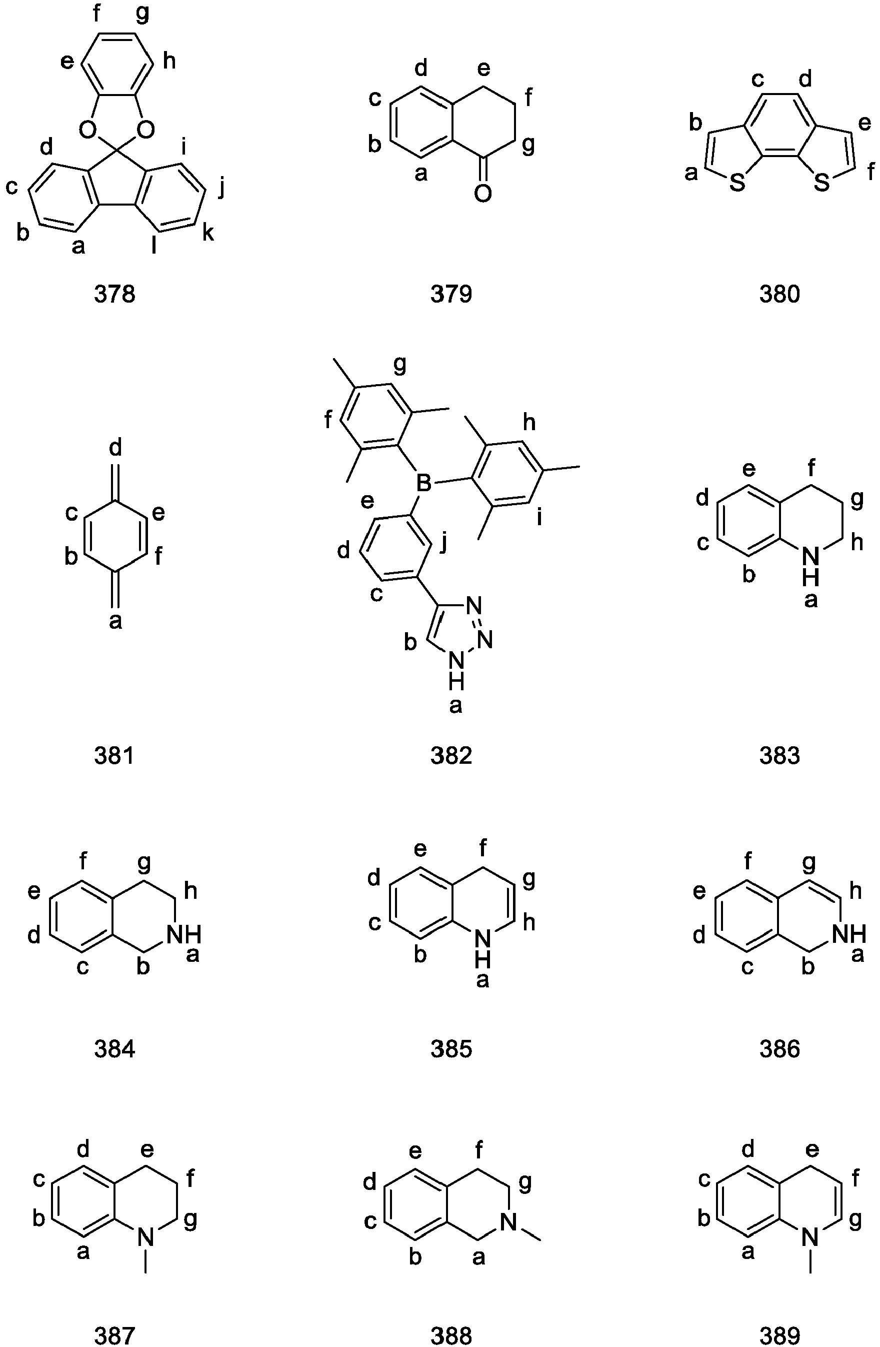

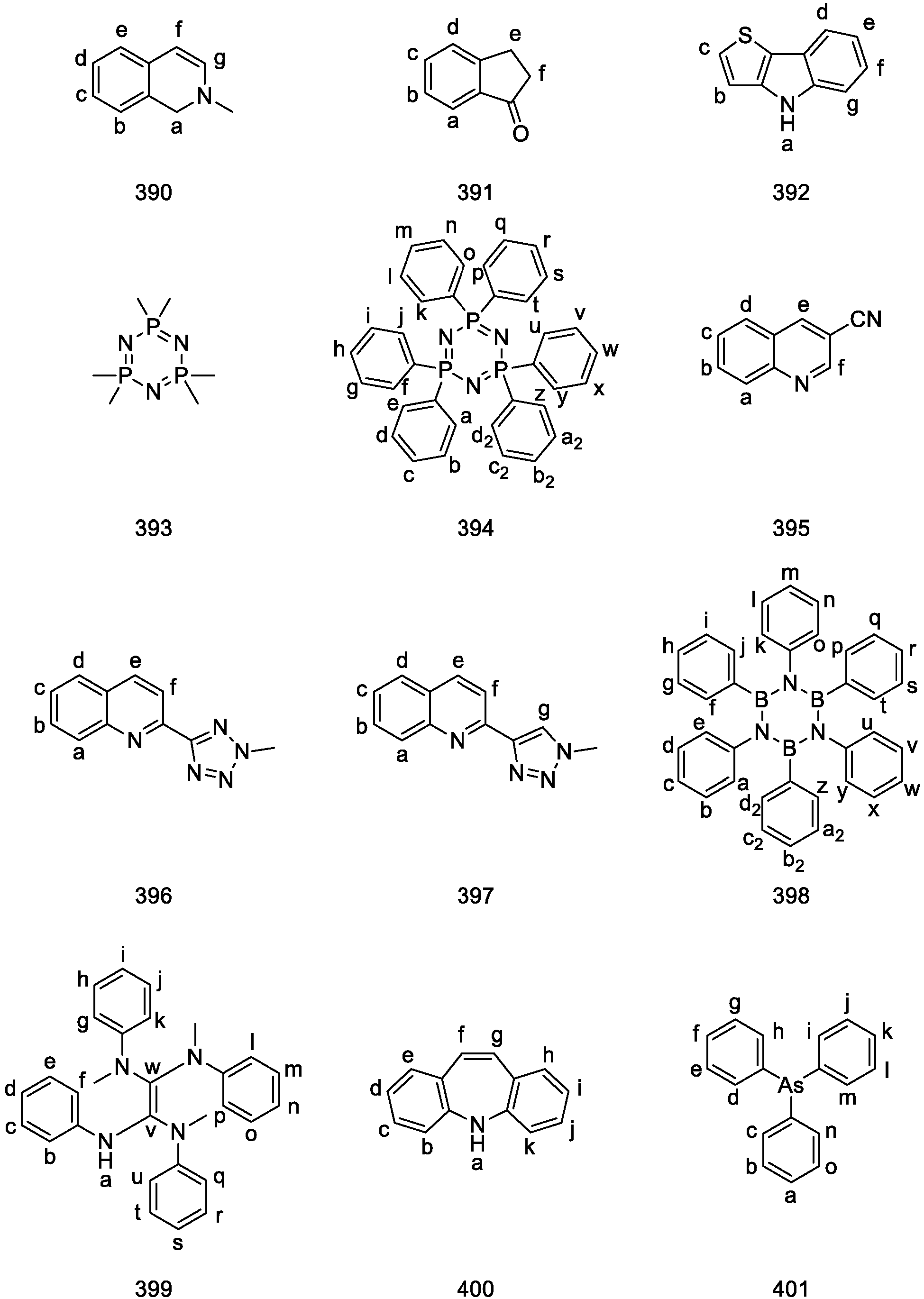

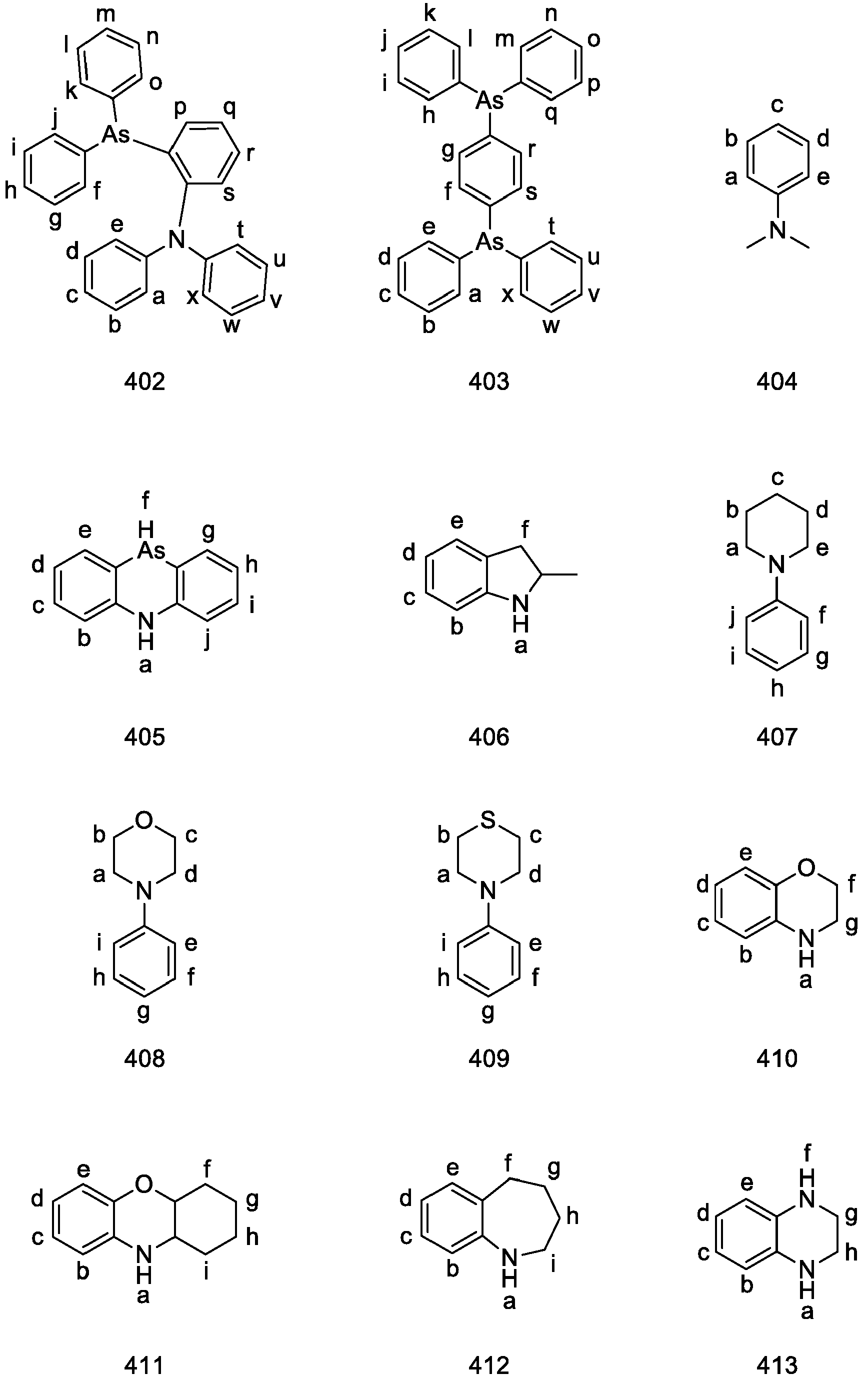

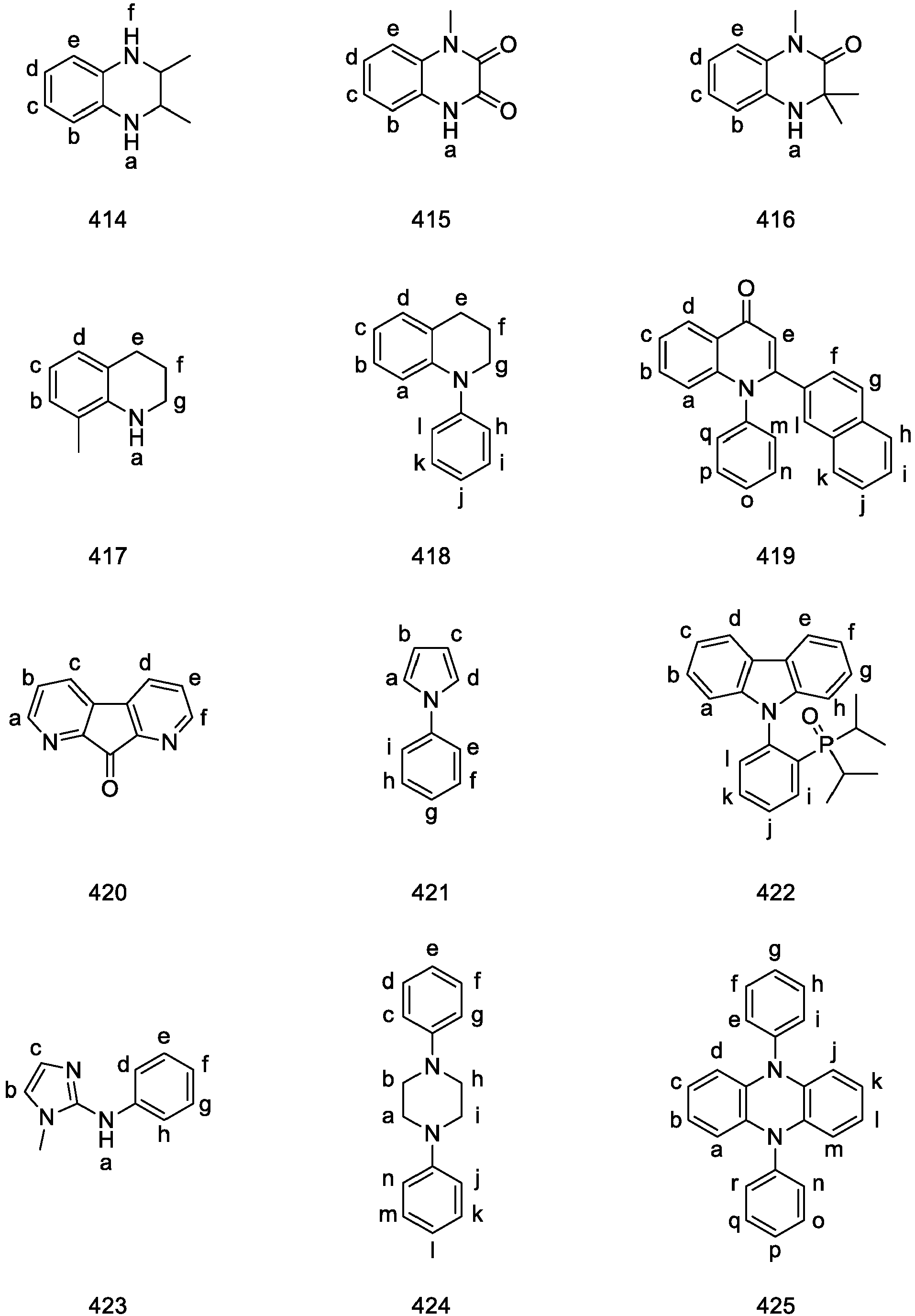

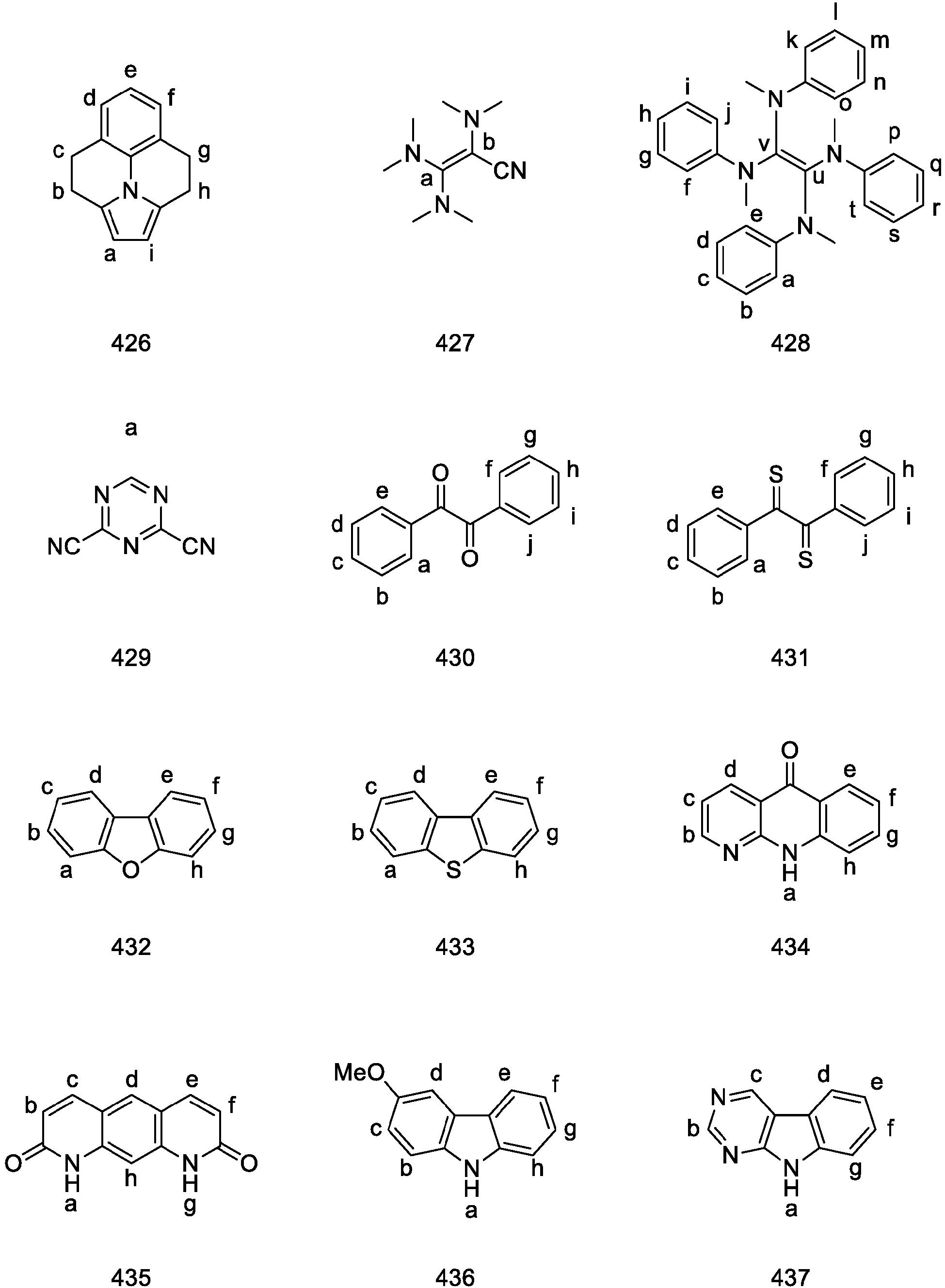

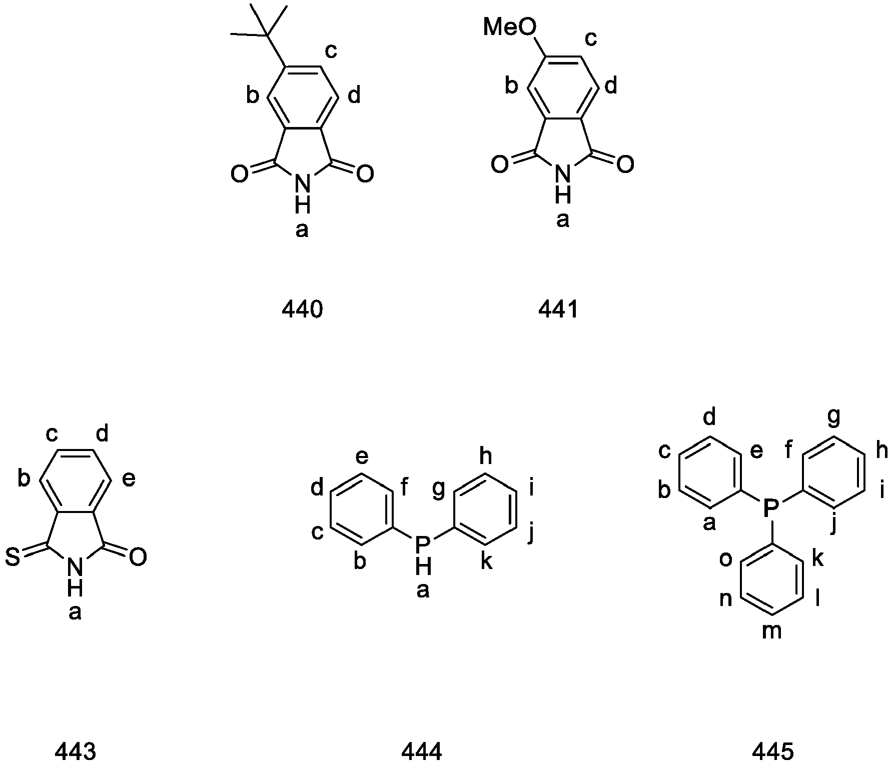

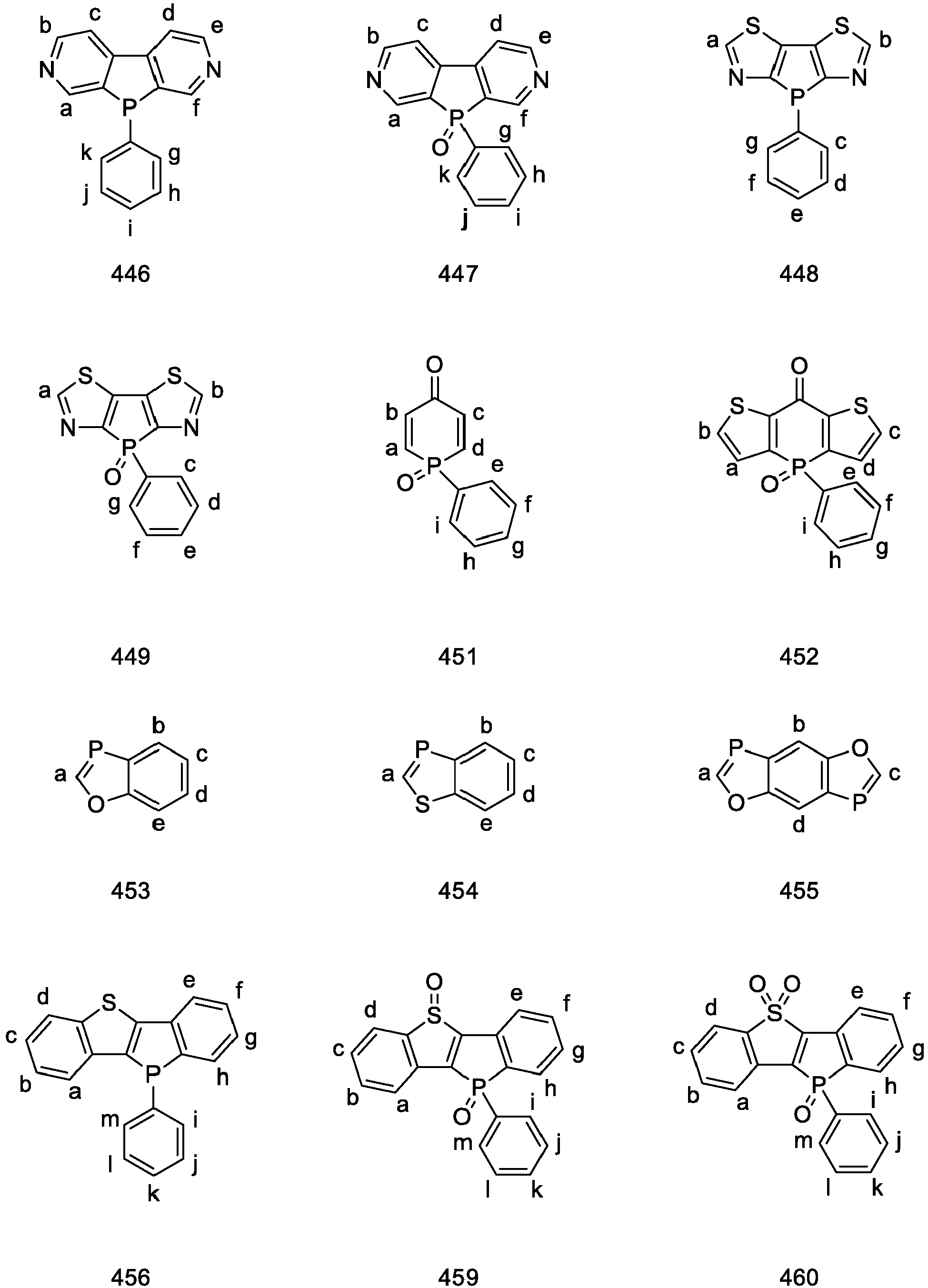

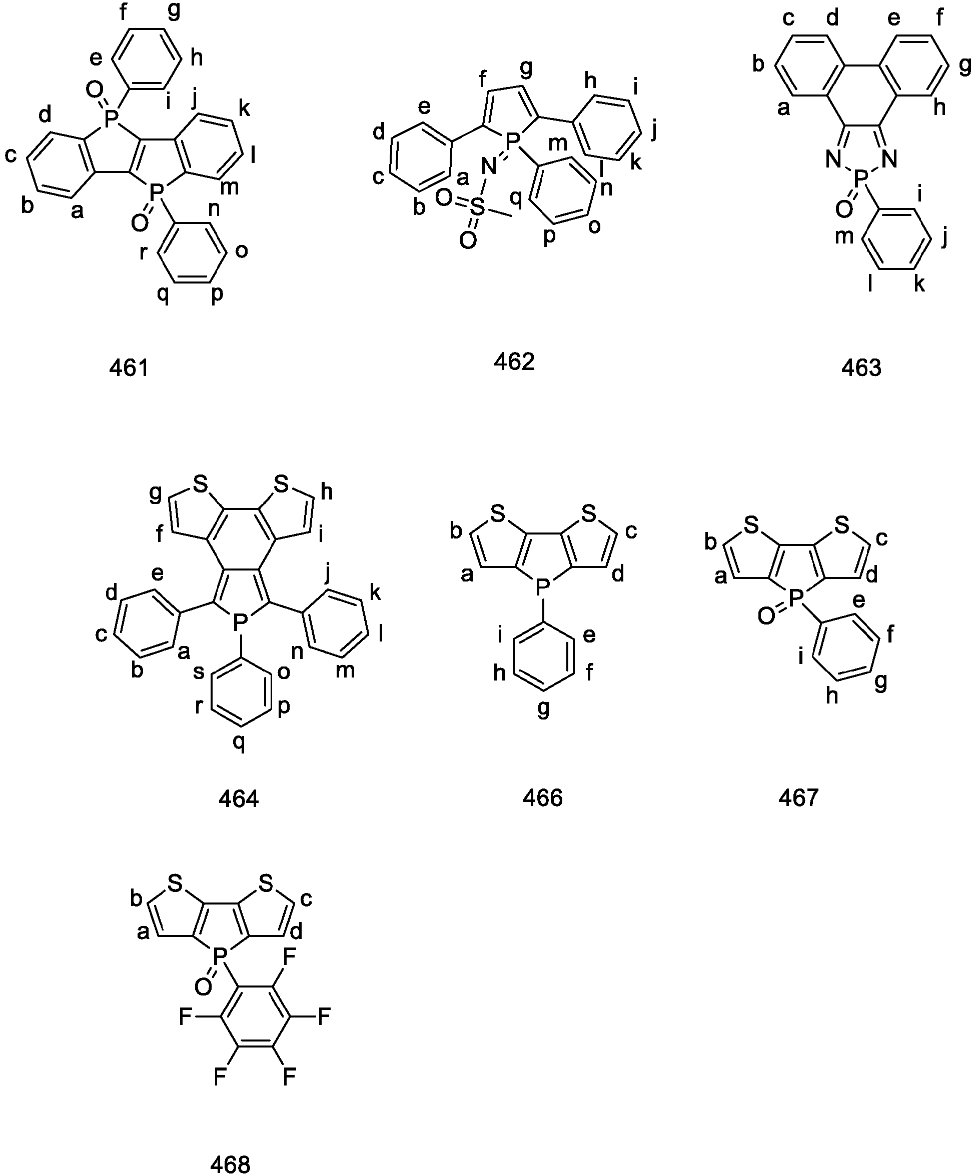

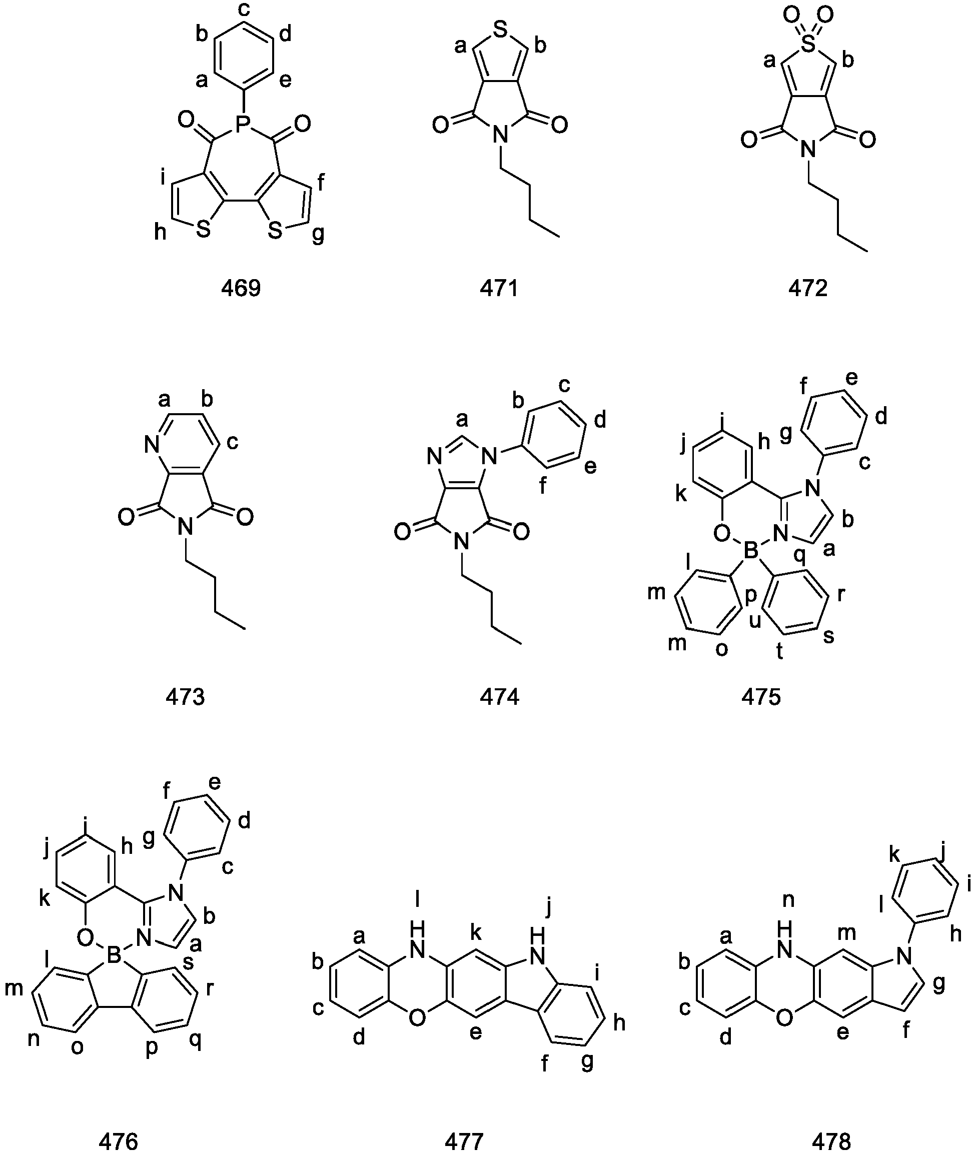

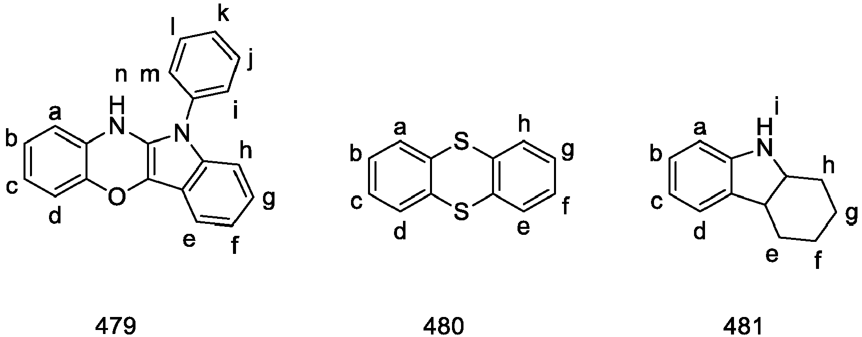

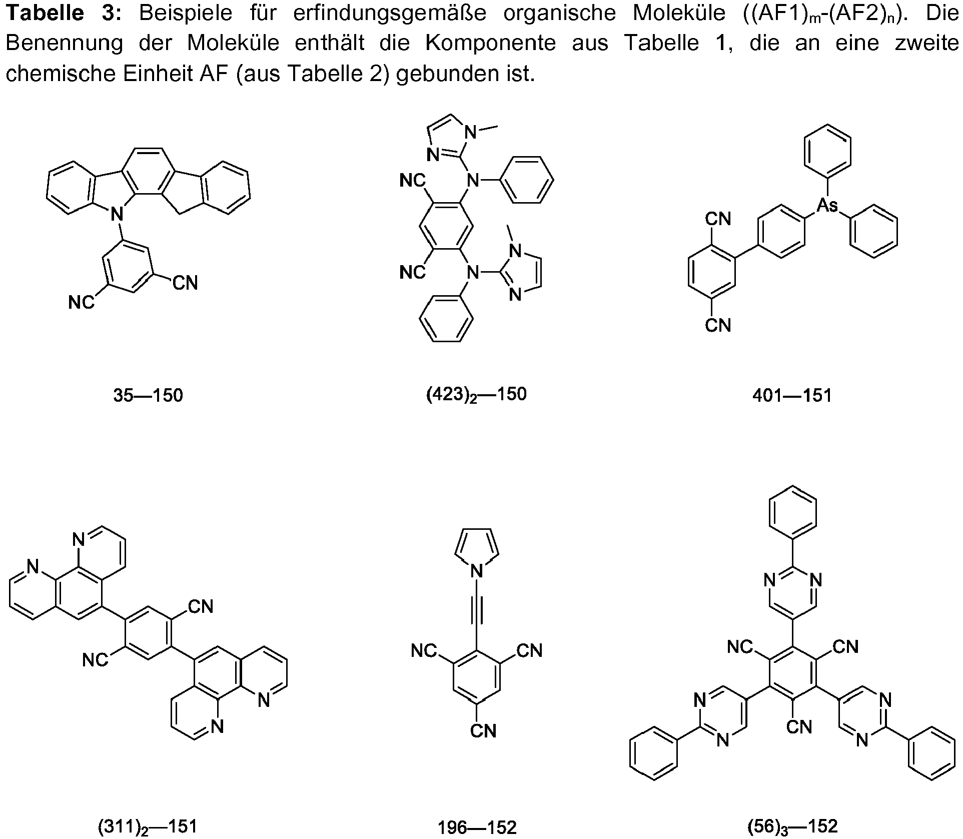

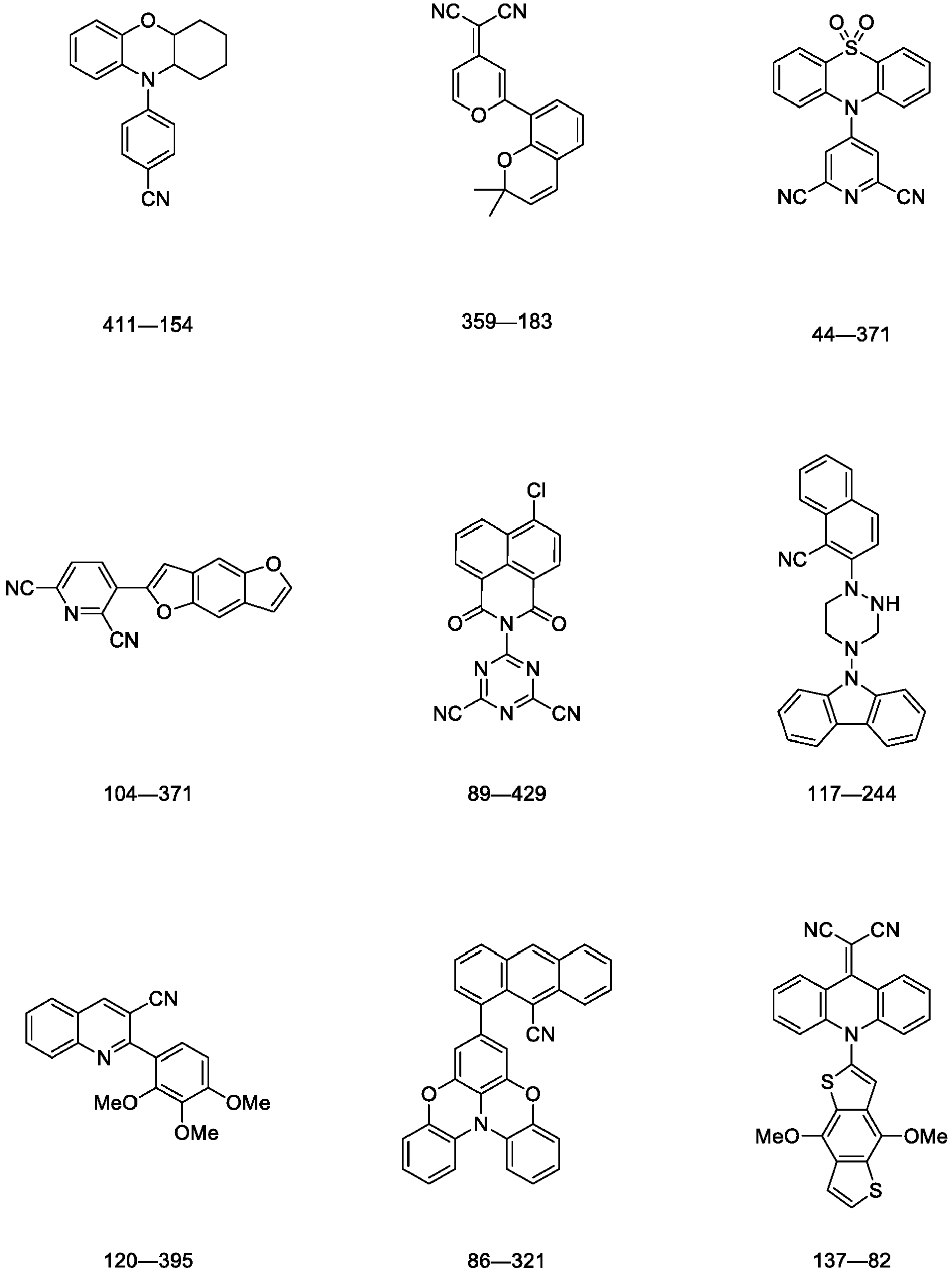

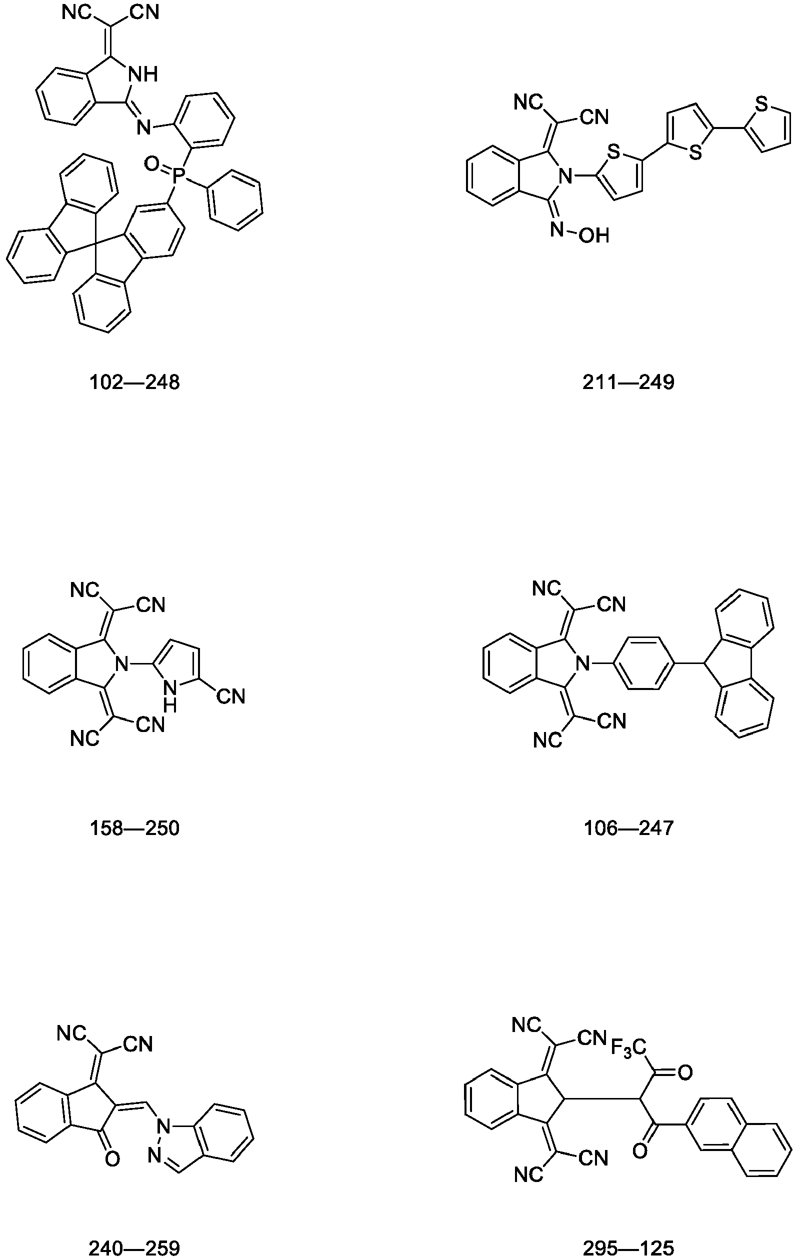

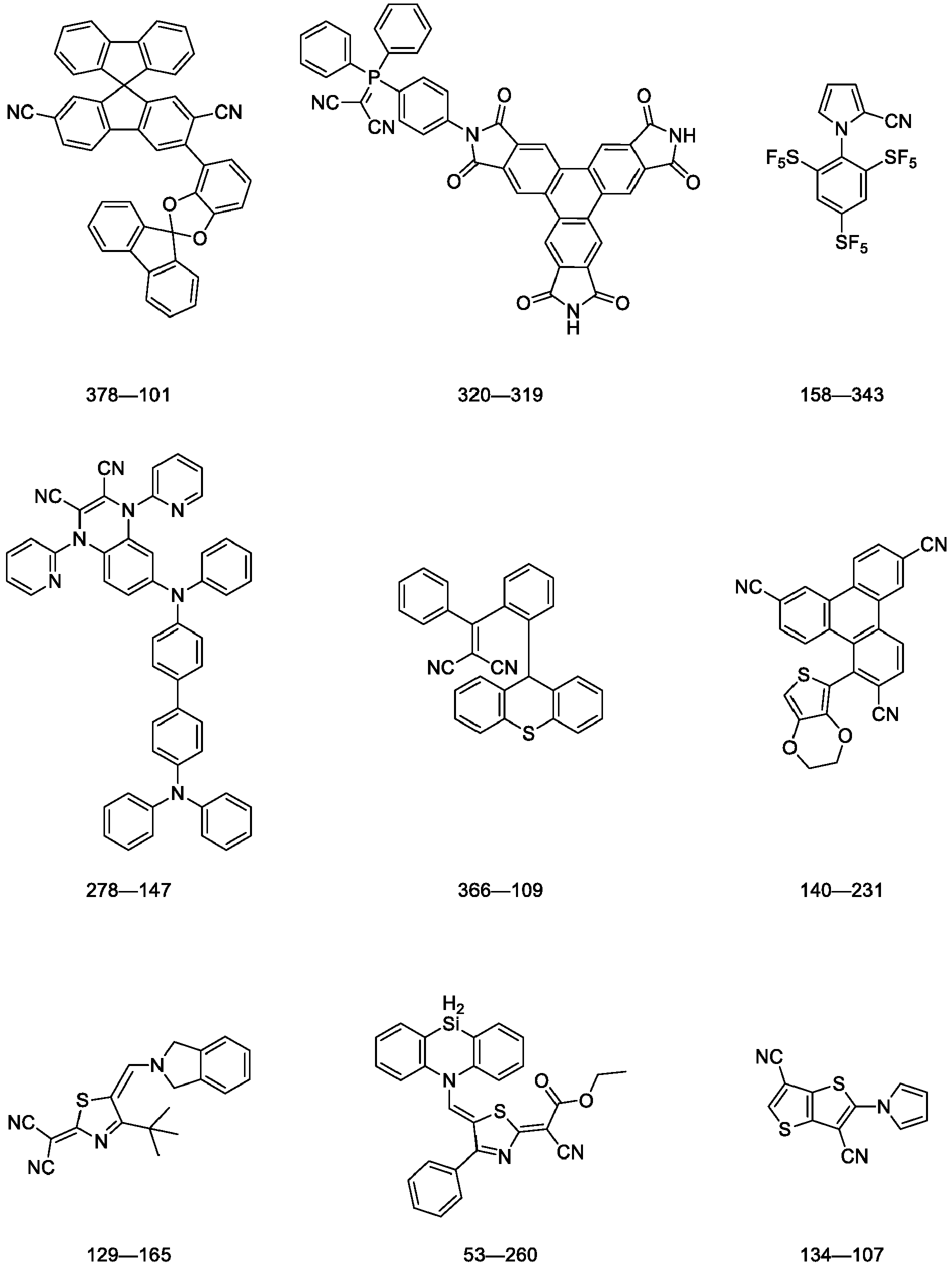

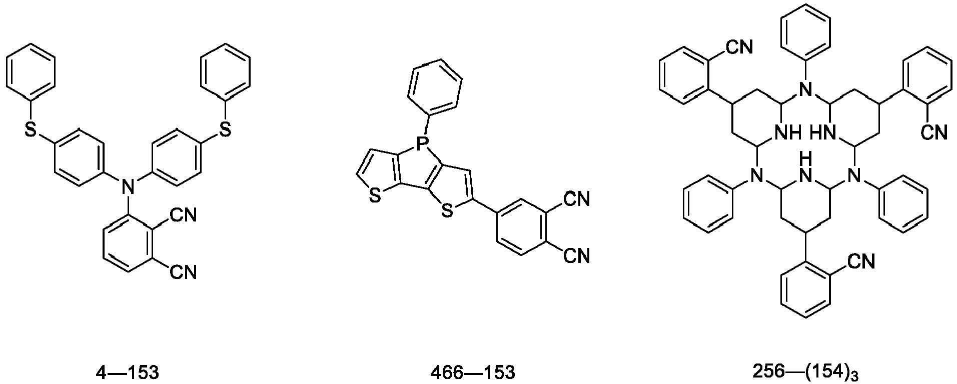

In Tabelle 3 sind mögliche Anknüpfungspositionen sind mit Kleinbuchstaben a bis z bezeichnet. Jede aromatische C-H und N-H Bindung ist optional mit einem löslichkeitsvermittelnden und/oder einem die Polymerisierbarkeit ermöglichenden Rest R substituiert.

Insbesondere die Verwendung chemischen Einheit gemäß einer der Formeln 1a, 2a-2c oder 3a-3e unterscheidet die organischen Moleküle funktionell von Molekülen gemäß dem Stand der Technik da durch den in der einen chemischen Einheit enthaltenen, in ortho-Position zur anderen chemischen Einheit angeknüpften Substituenten die beiden pi-Systeme von AF1 und AF2 durch Verdrillung nicht in Konjugation stehen, aber dennoch in enger räumlicher Nähe fixiert sind.

Anhand spektroskopischer Auswahlregeln (symmetrische Moleküle) oder durch Messung des Extinktionskoeffizienten (UV/VIS-Spektroskopie) oder anhand quantenchemischer Berechnung der Oszillatorstärke kann vorhergesagt werden, ob ein quantenmechanischer Übergang erlaubt ist. Je größer die Oszillatorstärke, desto eher ist ein Übergang erlaubt und desto schneller ist der damit verbundene Prozess (Abklingdauer). Angestrebt sind Abklingdauern von < 300 µs, bevorzugt < 100 µs, besonders bevorzugt von < 50 µs. Bei einer langen Abklingdauer des (organischen) Emitters kommt es bei hohen Stromstärken schnell zu Sättigungseffekten, was die Bauteillebensdauer negativ beeinflusst und die Erreichung hoher Helligkeiten verhindert.

Ein Maß für die Abklingdauer ist der ΔE(S₁-T₁)-Abstand. Dieser wird durch die Überlappung von HOMO und LUMO beeinflusst. Die Größe des quantenmechanischen einen Wert von 0. Die Wahrscheinlichkeit einer effizienten Emission des organischen Moleküls sinkt drastisch. Bei einem Wert von 1 liegt nicht mehr verzögerte Fluoreszenz (TADF) sondern spontane Emission vor.

Erfindungsgemäße organische Moleküle sind in Tabelle 4 dargestellt die sich durch Kombination der oben definierten Paare aus chemischen Einheiten AF1 und AF2 und der Festlegung der Verknüpfung ergeben. Weitere organische Moleküle können durch Kombination der genannten Moleküleinheiten erhalten werden. Die Benennung der Moleküle erfolgt nach dem Schema AF-S-AF, wobei hinsichtlich der Benennung der einen chemischen Einheit AF auf Tabelle 1 und hinsichtlich der Benennung einer zweiten chemischen Einheit auf Tabelle 2 verweisen wird. Der Übersichtlichkeit halber werden die beiden Nummern durch den Buchstaben S getrennt.

**Tabelle 4: Erfindungsgemäße organische Moleküle nach dem Schema AF-S-AF. In Klammern sind die Werte für ΔHOMO, ΔLUMO und Gap angegeben. Der besseren Übersicht halber sind die beiden AF durch den Buchstaben S getrennt.**

| | | | |
|---|---|---|---|
| 82 - S - 247 (0.89 1.06 1.00) | 82 - S - 259 (1.01 0.89 1.17) | 82 - S - 324 (1.06 0.98 1.08) | 86 - S - 57 (1.21 0.85 1.27) |
| 86 - S - 247 (0.92 1.14 0.97) | 86 - S - 259 (1.04 0.97 1.14) | 86 - S - 270 (0.83 0.84 1.28) | 86 - S - 324 (1.09 1.07 1.05) |
| 86 - S - 472 (1.35 0.88 1.23) | 101 **-** S **-** 125 (1.02 1.34 1.24) | 101 **-** S - 429 (1.94 1.35 1.23) | 101 - S - 472 (0.93 0.92 1.65) |
| 107 - S - 343 (1.75 0.81 2.55) | 107 - S - 429 (1.31 1.51 1.86) | 109 - S - 429 (1.52 0.98 1.65) | 147 - S - 57 (1.42 1.28 1.05) |
| 147 - S - 70 (1.22 0.95 1.38) | 147 - S - 152 (2.63 0.94 1.39) | 147 - S - 165 (0.92 1.36 0.97) | 147 - S - 207 (1.01 0.80 1.53) |
| 147 - S - 215 (1.24 1.07 1.26) | 147 - S - 248 (1.07 0.92 1.41) | 147 - S - 259 (1.25 1.41 0.93) | 147 - S - 270 (1.05 1.27 1.07) |
| 147 - S - 320 (1.34 1.01 1.33) | 147 - S - 343 (3.01 1.04 1.30) | 147 - S - 364 (1.19 1.17 1.17) | 147 - S - 368 (1.12 1.03 1.30) |
| 147 - S - 451 (0.91 0.94 1.40) | 147 - S - 463 (0.84 1.04 1.29) | 147 - S - 472 (1.57 1.31 1.02) | 154 - S - 152 (1.09 1.33 2.93) |
| 154 - S - 342 (0.94 0.93 3.33) | 154 - S - 343 (1.47 1.42 2.84) | 154 - S - 429 (1.03 2.12 2.14) | 158 - S - 46 (1.13 1.43 2.69) |
| 158 - S - 57 (0.80 2.45 1.67) | 158 - S - 125 (1.04 2.90 1.22) | 158 - S - 150 (1.46 1.56 2.56) | 158 - S - 151 (1.37 1.82 2.30) |
| 158 - S - 152 (2.01 2.12 2.01) | 158 - S - 153 (1.39 1.66 2.46) | 158 - S - 184 (1.21 1.15 2.97) | 158 - S - 192 (0.82 1.67 2.45) |
| 158 - S - 231 (0.86 1.78 2.34) | 158 - S - 250 (0.81 3.02 1.10) | 158 - S - 271 (1.04 1.85 2.27) | 158 - S - 291 (1.12 1.70 2.42) |
| 158 - S - 342 (1.87 1.72 2.41) | 158 - S - 343 (2.39 2.21 1.91) | 158 - S - 371 (1.55 1.80 2.32) | 158 - S - 429 (1.95 2.91 1.21) |
| 158 - S - 472 (0.95 2.48 1.64) | 183 - S - 125 (0.92 1.22 1.35) | 183 - S - 429 (1.83 1.23 1.34) | 183 - S - 472 (0.83 0.81 1.76) |
| 213 - S - 151 (1.05 0.81 2.63) | 213 - S - 152 (1.69 1.11 2.33) | 213 - S - 343 (2.07 1.20 2.23) | 213 - S - 429 (1.63 1.90 1.54) |
| 244 - S - 125 (0.91 1.70 1.36) | 244 - S - 152 (1.88 0.91 2.14) | 244 - S - 343 (2.26 1.01 2.05) | 244 - S - 429 (1.82 1.71 1.35) |
| 244 - S - 472 (0.82 1.28 1.77) | 248 - S - 429 (1.50 0.82 1.67) | 427 - S - 0 (1.15 1.70 1.72) | 427 - S - 9 (1.26 1.24 2.18) |
| 427 - S - 20 (1.37 1.78 1.63) | 427 - S - 27 (1.28 1.95 1.46) | 427 - S - 28 (1.34 2.22 1.19) | 427 - S - 29 (1.10 1.94 1.47) |
| 427 - S - 40 (0.93 1.94 1.47) | 427 - S - 44 (0.99 1.10 2.31 ) | 427 - S - 46 (2.51 2.11 1.31) | 427 - S - 56 ( 1.27 1.41 2.00) |
| 427 - S - 61 (1.25 1.93 1.49) | 427 - S - 62 (1.13 2.12 1.29) | 427 - S - 65 (1.26 1.72 1.69) | 427 - S - 68 (0.97 1.68 1.73) |
| 427 - S - 79 (1.25 2.10 1.32) | 427 - S - 80 (1.20 2.05 1.36) | 427 - S - 81 (1.28 2.18 1.24) | 427 - S - 82 (1.01 2.36 1.05) |
| 427 - S - 83 (1.31 1.35 2.06) | 427 - S - 85 (1.76 1.91 1.51) | 427 - S - 86 (0.98 2.28 1.14) | 427 - S - 90 (1.65 2.38 1.03) |
| 427 - S - 101 (1.40 2.24 1.18) | 427 - S - 102 (1.02 1.24 2.17) | 427 - S - 105 (1.56 1.33 2.08) | 427 - S - 107 (2.02 2.07 1.34) |
| 427 - S -108 (1.58 2.46 0.95) | 427 - S - 110 (1.31 2.24 1.18) | 427 - S - 111 (2.02 1.36 2.06) | 427 - S - 112 (1.21 1.73 1.68) |
| 427 - S -114 (0.86 1.01 2.41) | 427 - S - 115 (1.69 2.21 1.20) | 427 - S - 118 (1.30 1.63 1.78) | 427 - S - 119 (1.27 1.85 1.57) |
| 427 - S - 123 (0.91 1.97 1.44) | 427 - S - 131 (1.32 1.31 2.11) | 427 - S - 132 (1.24 1.55 1.86) | 427 - S - 133 (2.02 2.36 1.05) |
| 427 - S - 144 (1.36 2.27 1.14) | 427 - S - 148 (1.02 1.14 2.27) | 427 - S - 150 (2.85 2.23 1.18) | 427 - S - 151 (2.75 2.49 0.92) |
| 427 - S - 153 (2.78 2.33 1.08) | 427 - S - 154 (2.31 1.46 1.95) | 427 - S - 166 (2.03 1.29 2.12) | 427 - S - 167 (1.89 0.97 2.44) |
| 427 - S - 171 (1.80 1.29 2.13) | 427 - S - 175 (1.03 0.93 2.49) | 427 - S - 177 (1.86 2.01 1.40) | 427 - S - 182 (1.00 0.85 2.56) |
| 427 - S - 183 (1.51 2.35 1.06) | 427 - S - 184 (2.59 1.83 1.59) | 427 - S - 187 (1.74 1.20 2.22) | 427 - S - 188 (2.17 1.24 2.18) |
| 427 - S - 189 (1.92 0.83 2.59) | 427 - S - 192 (2.20 2.35 1.07) | 427 - S - 193 (1.12 1.51 1.90) | 427 - S - 194 (1.97 0.95 2.46) |
| 427 - S - 195 (2.03 1.67 1.74) | 427 - S - 198 (1.74 1.73 1.68) | 427 - S - 205 (1.26 1.79 1.62) | 427 - S - 208 (1.22 1.26 2.15) |
| 427 - S - 209 (0.97 1.34 2.07) | 427 - S - 213 (1.70 1.69 1.73) | 427 - S - 214 (1.26 0.92 2.49) | 427 - S - 217 (1.13 1.72 1.69) |
| 427 - S - 218 (1.10 1.90 1.52) | 427 - S - 219 (0.90 1.45 1.96) | 427 - S - 222 (1.01 1.30 2.11) | 427 - S - 230 (0.94 1.13 2.29) |
| 427 - S - 231 (2.24 2.46 0.95) | 427 - S - 233 (1.63 1.88 1.53) | 427 - S - 234 (1.45 1.96 1.45) | 427 - S - 236 (1.67 0.85 2.56) |
| 427 - S - 237 (1.46 2.26 1.15) | 427 - S - 243 (2.20 1.09 2.32) | 427 - S - 244 (1.51 1.88 1.54) | 427 - S - 245 (1.43 1.65 1.76) |
| 427 - S - 246 (1.77 2.22 1.20) | 427 - S - 251 (1.28 2.46 0.95) | 427 - S - 254 (1.27 1.57 1.84) | 427 - S - 255 (2.16 2.43 0.98) |
| 427 - S - 265 (1.73 2.36 1.06) | 427 - S - 266 (1.37 1.93 1.48) | 427 - S - 267 (1.17 1.63 1.78) | 427 - S - 268 (1.71 2.51 0.91) |
| 427 - S - 269 (1.67 2.43 0.98) | 427 - S - 272 (1.55 0.98 2.43) | 427 - S - 274 (1.04 1.44 1.98) | 427 - S - 275 (0.84 1.86 1.56) |
| 427 - S - 276 (0.96 0.84 2.57) | 427 - S - 277 (1.25 1.70 1.71) | 427 - S - 279 (1.31 1.76 1.65) | 427 - S - 280 (0.99 1.44 1.97) |
| 427 - S - 282 (1.11 1.27 2.14) | 427 - S - 284 (0.89 1.28 2.14) | 427 - S - 285 (1.03 1.65 1.77) | 427 - S - 286 (1.28 1.55 1.86) |
| 427 - S - 287 (1.09 1.56 1.85) | 427 - S - 288 (1.08 1.50 1.91) | 427 - S - 289 (1.34 1.34 2.07) | 427 - S - 290 (1.37 1.97 1.45) |
| 427 - S - 291 (2.51 2.37 1.04) | 427 - S - 292 (1.84 2.03 1.38) | 427 - S - 293 (1.34 1.84 1.57) | 427 - S - 294 (1.80 1.87 1.54) |
| 427 - S - 295 (1.55 2.12 1.30) | 427 - S - 296 (1.83 2.11 1.30) | 427 - S - 297 (1.14 1.24 2.17) | 427 - S - 298 ( 1.40 1.72 1.70) |
| 427 - S - 300 (1.40 1.12 2.30) | 427 - S - 301 (1.46 0.83 2.59) | 427 - S - 303 (1.16 1.09 2.32) | 427 - S - 304 (1.20 1.56 1.85) |
| 427 - S - 305 (1.72 1.78 1.63) | 427 - S - 306 (1.88 2.43 0.98) | 427 - S - 310 (1.32 1.17 2.24) | 427 - S - 311 (1.04 1.45 1.96) |
| 427 - S - 312 (1.14 1.32 2.09) | 427 - S - 313 (0.92 1.49 1.92) | 427 - S - 316 (1.20 1.78 1.64) | 427 - S - 318 (1.50 0.82 2.59) |
| 427 - S- 327 (1.49 2.18 1.23) | 427 - S - 330 (0.82 1.19 2.23) | 427 - S - 331 (1.35 1.36 2.05) | 427 - S - 332 (1.21 1.53 1.88) |
| 427 - S - 336 (1.31 1.42 1.99) | 427 - S - 341 (0.80 2.05 1.36) | 427 - S - 342 (3.25 2.39 1.02) | 427 - S - 356 (0.97 1.24 2.17) |
| 427 - S - 357 (1.67 0.99 2.43) | 427 - S - 358 (1.15 1.70 1.72) | 427 - S - 367 (1.77 2.33 1.08) | 427 - S - 370 (1.12 1.76 1.65) |
| 427 - S - 371 (2.94 2.48 0.93) | 427 - S - 372 (1.35 1.46 1.95) | 427 - S - 374 (0.96 1.96 1.46) | 427 - S - 379 (1.03 1.46 1.95) |
| 427 - S - 380 (0.87 0.88 2.53) | 427 - S - 382 (1.10 1.64 1.77) | 427 - S - 391 (1.04 1.45 1.97) | 427 - S - 395 (1.97 2.18 1.23) |
| 427 - S - 396 (1.39 1.62 1.80) | 427 - S - 397 (1.20 1.46 1.95) | 427 - S - 415 (1.09 1.34 2.07) | 427 - S - 430 (0.98 2.28 1.13) |
| 427 - S - 432 (1.21 0.95 2.46) | 427 - S - 433 (0.93 0.97 2.45) | 427 - S - 434 (1.04 1.75 1.66) | 427 - S - 435 (1.13 1.92 1.49) |
| 427 - S - 437 (1.31 1.30 2.12) | 427 - S - 440 (1.65 2.09 1.32) | 427 - S - 441 (1.58 2.02 1.40) | 427 - S - 447 (1.59 2.20 1.21) |
| 427 - S - 449 (1.33 2.17 1.24) | 427 - S - 450 (0.96 1.84 1.58) | 427 - S - 452 (1.73 2.44 0.98) | 427 - S - 453 (1.22 1.14 2.27) |
| 427 - S - 454 ( 1.05 1.31 2.11) | 427 - S - 455 (1.03 1.58 1.84) | 427 - S - 457 (1.18 2.08 1.33) | 427 - S - 459 (0.97 2.32 1.10) |
| 427 - S - 461 (1.17 2.27 1.14) | 427 - S - 462 (0.87 2.29 1.12) | 427 - S - 467 (0.86 1.68 1.73) | 427 - S - 468 (1.06 1.99 1.42) |
| 427 - S - 469 (1.15 2.42 1.00) | 427 - S - 471 (1.76 1.75 1.67) | 427 - S - 473 (1.80 2.36 1.05) | 427 - S - 474 (1.66 2.25 1.16) |
| | | | |
| 12 - S - 82 (0.89 1.02 1.84) | 12 - S - 154 (2.19 0.80 2.06) | 12 - S - 429 (3.21 1.11 1.75) | 19 - S - 109 (0.99 0.98 3.27) |
| 19 - S -125 (1.59 1.20 3.04) | 19 - S -150 (2.01 0.90 3.35) | 19 - S - 152 (2.57 1.24 3.01) | 19 -S - 153 (1.95 0.96 3.28) |
| 19 - S - 154 (1.48 1.48 2.77) | 19 - S - 248 (1.00 1.07 3.18) | 19 - S - 429 (2.51 1*.*78 2.46) | 22 - S - 82 (1.26 0.88 1.46) |
| 22 - S - 429 (3.59 0.97 1.38) | 23 - S - 82 (1.15 0.80 1.57) | 23 - S - 429 (3.48 0.89 1.49) | 24 - S - 82 (0.89 1.26 1.83) |
| 24 - S - 154 (2.19 1.04 2.05) | 24 - S - 429 (3.22 1.34 1.75) | 26 - S - 429 (3.58 0.81 1.38) | 28 - S - 429 (1.99 0.88 2.97) |
| 29 - S - 429 (2.23 0.89 2.73) | 30 - S - 429 (2.82 1.01 2.15) | 32 - S - 429 (2.72 0.82 2.25) | 35 - S - 154 (1.73 0.97 2.51) |
| 35 - S - 429 (2.76 1.27 2.21) | 36 - S - 429 (1.84 0.91 3.13) | 37 - S - 429 (1.84 1.06 3.13) | 38 -S - 429 (2.62 0.88 2.35) |
| 39 - S - 82 (0.86 0.90 1.86) | 39 - S - 429 (3.19 0.99 1.78) | 44 - S - 429 (2.34 0.91 2.62) | 45 -S - 154 (1.92 0.95 2.33) |
| 45 - S - 429 (2.94 1.25 2.02) | 46 - S - 152 (0.88 0.85 4.69) | 46 - S - 429 (0.82 1.40 4.14) | 47 -S - 429 (3.09 0.80 1.88) |
| 48 - S - 101 (1.08 0.82 2.84) | 48 - S - 109 (1.50 0.90 2.76) | 48 - S - 125 (2.10 1.13 2.53) | 48 -S - 150 (2.52 0.82 2.84) |
| 48 - S - 152 (3.08 1.16 2.50) | 48 - S - 153 (2.46 0.89 2.77) | 48 - S - 154 (1.99 1.41 2.25) | 48 -S - 158 (1.06 1.13 2.52) |
| 48 - S - 183 (1.19 0.80 2.86) | 48 - S - 244 (1.19 0.83 2.83) | 48 - S - 248 (1.51 0.99 2.66) | 48 -S - 429 (3.02 1.71 1.95) |
| 57 - S - 429 (1.15 0.82 3.82) | 60 - S - 429 (2.78 0.84 2.19) | 64 - S - 125 (1.94 1.00 2.69) | 64 -S - 152 (2.92 1.04 2.66) |
| 64 - S - 154 (1.83 1.28 2.41) | 64 - S - 158 (0.90 1.01 2.69) | 64 - S - 248 (1.35 0.87 2.83) | 64 -S - 429 (2.86 1.58 2.11) |
| 66 - S - 82 (1.54 1.34 1.19) | 66 - S - 125 (2.95 0.85 1.69) | 66 - S - 152 (3.92 0.88 1.65) | 66 - S - 154 (2.84 1.13 1.41) |
| 66 - S -158 (1.91 0.86 1.68) | 66 - S - 429 (3.86 1.43 1.10) | 75 - S - 82 (1.21 1.50 1.51) | 75 - S - 125 (2.63 1.01 2.01) |
| 75 - S - 152 (3.60 1.04 1.97) | 75 - S - 154 (2.51 1.281.73) | 75 - S - 158 (1.59 1.01 2.00) | 75 - S - 248 (2.04 0.87 2.14) |
| 75 - S - 429 (3.54 1.59 1.43) | 76 - S - 82 (1.55 1.06 1.18) | 76 - S - 154 (2.85 0.84 1.40) | 76 - S - 429 (3.87 1.15 1.09) |
| 77 - S - 82 (1.06 0.80 1.67) | 77 - S - 429 (3.39 0.89 1.58) | 78 - S - 82 (1.24 1.00 1.49) | 78 - S - 429 (3.56 1.08 1.40) |
| 88 - S - 154 (1.83 1.03 2.41) | 88 - S - 429 (2.86 1.34 2.11) | 89 - S - 429 (1.60 0.85 3.37) | 91 - S - 82 (1.29 1.32 1.44) |
| 91 - S - 125 (2.70 0.82 1.94) | 91 - S - 152 (3.67 0.86 1.90) | 91 - S - 154 (2.59 1.10 1.66) | 91 - S - 158 (1.66 0.83 1.93) |
| 91 - S - 429 (3.61 1.401.35) | 95 - S - 429 (3.12 0.86 1.85) | 96 - S - 109 (1.47 0.81 2.78) | 96 - S - 125 (2.08 1.03 2.56) |
| 96 - S - 152 (3.05 1.07 2.52) | 96 - S - 154 (1.97 1.31 2.28) | 96 - S - 158 (1.04 1.04 2.55) | 96 - S - 248 (1.49 0.90 2.69) |
| 96 - S - 429 (2.99 1.62 1.97) | 97 - S - 82 (0.88 1.56 1.84) | 97 - S - 109 (1.69 0.84 2.57) | 97 -S - 125 (2.30 1.07 2.34) |
| 97 - S - 152 (3.27 1.102.30) | 97 - S - 153 (2.65 0.83 2.58) | 97 - S - 154 (2.18 1.34 2.06) | 97 - S - 158 (1.26 1.07 2.33) |
| 97 - S - 248 (1.71 0.93 2.47) | 97 - S **-** 429 (3.21 1.65 1.76) | 102 **-** S **-** 154 (1.29 0.88 2.96) | 102 - S - 429 (2.31 1.19 2.65) |
| 103 - S - 82 (1.44 0.99 1.28) | 103 - S - 429 (3.77 1.07 1.20) | 104 - S - 154 (1.63 0.82 2.61) | 104 - S - 429 (2.66 1.13 2.31) |
| 115 - S - 150 (1.16 1.25 4.21) | 115 - S - 151 (1.06 1.12 4.33) | 115 - S - 152 (1.71 1.59 3.86) | 115 - S - 153 (1.09 1.31 4.14) |
| 115 - S - 429 (1.65 2.13 3.32) | 118 - S - 154 (1.01 0.88 3.23) | 118 - S - 429 (2.04 1.18 2.93) | 119 - S - 429 (2.07 1.07 2.90) |
| 123 - S - 154 (1.40 1.00 2.84) | 123 - S - 429 (2.43 1.31 2.54) | 128 - S - 82 (0.93 1.08 1.79) | 128 - S - 154 (2.23 0.86 2.01) |
| 128 - S - 429 (3.26 1.16 1.71) | 129 - S - 429 (2.64 0.97 2.33) | 131 - S - 429 (2.01 1.01 2.95) | 144 - S - 154 (0.95 0.92 3.29) |
| 144 - S - 429 (1.98 1.23 2.99) | 148 - S - 109 (0.80 1.23 3.46) | 148 - S - 125 (1.41 1.46 3.23) | 148 - S - 150 (1.83 1.15 3.54) |
| 148 - S - 151 (1.74 1.03 3.66) | 148 - S - 152 (2.38 1.49 3.19) | 148 - S - 153 (1.76 1.22 3.47) | 148 - S - 154 (1.29 1.74 2.95) |
| 148 - S - 247 (0.89 1.05 3.64) | 148 - S - 248 (0.82 1.33 3.36) | 148 - S - 429 (2.32 2.04 2.65) | 154 - S - 152 (1.09 1.11 4.49) |
| 154 - S - 429 (1.03 1.65 3.94) | 155 - S - 152 (2.10 0.81 3.47) | 155 - S - 154 (1.02 1.05 3.23) | 155 - S - 429 (2.04 1.35 2.92) |
| 159 - S - 107 (0.91 1.64 4.09) | 159 - S - 125 (1.31 2.40 3.33) | 159 - S - 150 (1.73 2.10 3.63) | 159 - S - 151 (1.64 1.97 3.76) |
| 159 - S - 152 (2.28 2.44 3.29) | 159 - S - 153 (1.66 2.17 3.56) | 159 - S - 154 (1.20 2.68 3.05) | 159 - S - 429 (2.22 2.99 2.74) |
| 160 - S - 150 (1.04 1.25 4.33) | 160 - S - 151 (0.95 1.13 4.45) | 160 - S - 152 (1.59 1.59 3.98) | 160 - S - 153 (0.97 1.32 4.26) |
| 160 - S - 429 (1.53 2.14 3.44) | 161 - S - 152 (1.58 0.99 4.00) | 161 - S - 429 (1.52 1.54 3.45) | 165 - S - 429 (1.65 1.11 3.32) |
| 167 - S - 150 (0.95 0.89 4.41) | 167 - S - 152 (1.50 1.24 4.07) | 167 - S - 153 (0.88 0.96 4.34) | 167 - S - 429 (1.44 1.78 3.52) |
| 168 - S - 125 (0.97 1.12 3.67) | 168 - S - 150 (1.39 0.81 3.97) | 168 - S - 152 (1.94 1.16 3.63) | 168 - S - 153 (1.32 0.88 3.91) |
| 168 - S - 154 (0.85 1.40 3.39) | 168 - S - 429 (1.88 1.70 3.09) | 177 - S - 429 (1.48 1.31 3.49) | 189 - S - 150 (0.93 0.91 4.43) |
| 189 - S - 152 (1.48 1.25 4.09) | 189 - S - 153 (0.86 0.98 4.37) | 189 - S - 429 (1.42 1.79 3.55) | 192 - S - 152 (1.20 1.89 4.38) |
| 192 - S - 429 (1.14 2.44 3.83) | 193 - S - 125 (1.31 1.45 3.33) | 193 - S - 150 (1.73 1.15 3.64) | 193 - S - 151 (1.64 1.02 3.76) |
| 193 - S - 152 (2.28 1.49 3.29) | 193 - S - 153 (1.66 1.21 3.57) | 193 - S - 154 (1.19 1.73 3.05) | 193 - S - 429 (2.22 2.03 2.75) |
| 194 - S - 429 (1.37 1.11 3.60) | 195 - S - 150 (0.82 1.64 4.54) | 195 - S - 152 (1.37 1.98 4.20) | 195 - S - 429 (1.31 2.53 3.65) |
| 196 - S - 125 (1.36 1.03 3.28) | 196 - S - 152 (2.33 1.06 3.24) | 196 - S - 154 (1.24 1.30 3.00) | 196 - S - 429 (2.27 1.61 2.70) |
| 197 - S - 429 (1.17 1.02 3.80) | 200 - S - 429 (2.91 0.81 2.06) | 205 - S - 125 (1.17 0.94 3.47) | 205 - S - 152 (2.14 0.98 3.43) |
| 205 - S - 154 (1.05 1.22 3.19) | 205 - S - 429 (2.08 1.52 2.89) | 206 - S - 429 (2.09 1.08 2.88) | 208 - S - 429 (2.12 0.86 2.85) |
| 209 - S - 154 (1.34 0.80 2.90) | 209 - S - 429 (2.37 1.10 2.60) | 211 - S - 429 (3.02 0.93 1.95) | 217 - S - 429 (2.21 0.96 2.76) |
| 222 - S - 429 (2.33 0.83 2.64) | 224 - S - 109 (1.17 0.92 3.08) | 224 - S - 125 (1.78 1.15 2.86) | 224 - S - 150 (2.20 0.84 3.16) |
| 224 - S - 152 (2.75 1.18 2.82) | 224 - S - 153 (2.13 0.91 3.09) | 224 - S - 154 (1.67 1.42 2.58) | 224 - S - 183 (0.86 0.82 3.18) |
| 224 - S - 244 (0.87 0.85 3.15) | 224 - S - 248 (1.19 1.01 2.99) | 224 - S - 429 (2.69 1.73 2.27) | 225 - S - 429 (2.83 1.05 2.13) |
| 228 - S - 154 (1.67 0.87 2.57) | 228 - S - 429 (2.70 1.17 2.27) | 230 - S - 154 (1.37 0.91 2.87) | 230 - S - 429 (2.40 1.21 2.57) |
| 236 - S - 429 (1.67 1.04 3.30) | 240 - S - 429 (2.28 0.86 2.69) | 247 - S - 152 (1.49 0.99 4.08) | 247 - S - 429 (1.43 1.54 3.53) |
| 248 - S - 429 (1.50 0.81 3.46) | 260 - S - 125 (1.14 2.17 3.50) | 260 - S - 150 (1.56 1.86 3.80) | 260 - S - 151 (1.47 1.74 3.93) |
| 260 - S - 152 (2.11 2.21 3.46) | 260 - S - 153 (1.49 1.93 3.73) | 260 - S - 154 (1.03 2.45 3.22) | 260 - S - 429 (2.05 2.75 2.92) |
| 261 - S - 429 (2.44 0.93 2.53) | 264 - S - 152 (1.85 0.82 3.73) | 264 - S - 429 (1.79 1.36 3.18) | 265 - S - 429 (1.61 1.04 3.36) |
| 266 - S - 125 (1.06 0.98 3.58) | 266 - S - 152 (2.03 1.02 3.54) | 266 - S - 154 (0.94 1.26 3.30) | 266 - S - 429 (1.97 1.56 3.00) |
| 279 - S - 125 (1.12 0.94 3.52) | 279 - S - 152 (2.09 0.97 3.48) | 279 - S - 154 (1.00 1.21 3.24) | 279 - S - 429 (2.03 1.52 2.94) |
| 283 - S - 82 (1.25 1.04 1.47) | 283 - S - 154 (2.55 0.82 1.69) | 283 - S - 429 (3.58 1.13 1.39) | 284 - S - 125 (1.54 0.86 3.10) |
| 284 - S - 152 (2.51 0.90 3.06) | 284 - S - 154 (1.43 1.14 2.82) | 284 - S - 429 (2.45 1.45 2.52) | 285 - S - 125 (1.39 1.46 3.24) |
| 285 - S - 150 (1.81 1.15 3.55) | 285 - S - 151 (1.72 1.03 3.68) | 285 - S - 152 (2.37 1.49 3.21) | 285 - S - 153 (1.75 1.22 3.48) |
| 285 - S - 154 (1.28 1.74 2.97) | 285 - S - 247 (0.87 1.05 3.65) | 285 - S - 248 (0.80 1.33 3.38) | 285 - S - 429 (2.31 2.04 2.66) |
| 286 - S - 125 (1.14 0.90 3.50) | 286 - S - 152 (2.11 0.94 3.46) | 286 - S - 154 (1.03 1.18 3.22) | 286 - S - 429 (2.05 1.49 2.91) |
| 294 - S - 429 (1.54 0.80 3.43) | 297 - S - 429 (2.20 0.94 2.77) | 299 - S - 429 (2.68 0.91 2.29) | 302 - S - 429 (1.76 1.14 3.21) |
| 304 - S - 429 (2.13 0.91 2.83) | 323 - S - 82 (0.87 0.87 1.85) | 323 - S - 429 (3.20 0.96 1.77) | 324 - S - 429 (1.26 1.11 3.70) |
| 331 - S - 429 (1.99 1.06 2.98) | 339 - S - 82 (1.01 1.54 1.72) | 339 - S - 109 (1.81 0.82 2.44) | 339 - S - 125 (2.42 1.05 2.22) |
| 339 - S - 152 (3.39 1.08 2.18) | 339 - S - 153 (2.77 0.81 2.45) | 339 - S - 154 (2.31 1.33 1.94) | 339 - S - 158 (1.38 1.06 2.21) |
| 339 - S - 248 (1.83 0.92 2.35) | 339 - S - 429 (3.33 1.63 1.63) | 341 - S - 109 (1.02 1.09 3.24) | 341 - S - 125 (1.62 1.31 3.01) |
| 341 - S - 150 (2.04 1.01 3.32) | 341 - S - 151 (1.95 0.88 3.45) | 341 - S - 152 (2.60 1.35 2.98) | 341 - S - 153 (1.98 1.08 3.25) |
| 341 - S - 154 (1.51 1.59 2.74) | 341 - S - 213 (0.90 0.84 3.49) | 341 - S - 247 (1.10 0.91 3.42) | 341 - S - 248 (1.03 1.18 3.15) |
| 341 - S - 429 (2.54 1.90 2.43) | 344 - S - 429 (2.98 0.96 1.99) | 347 - S - 154 (1.69 0.83 2.55) | 347 - S - 429 (2.72 1.13 2.25) |
| 348 - S - 125 (2.16 0.86 2.48) | 348 - S - 152 (3.13 0.89 2.44) | 348 - S - 154 (2.04 1.14 2.20) | 348 - S - 158 (1.11 0.87 2.47) |
| 348 - S - 429 (3.07 1.44 1.90) | 349 - S - 152 (2.64 0.80 2.93) | 349 - S - 154 (1.56 1.04 2.69) | 349 - S - 429 (2.58 1.35 2.38) |
| 353 - S - 429 (2.50 1.10 2.47) | 359 - S - 109 (1.26 0.81 2.99) | 359 - S - 125 (1.87 1.03 2.77) | 359 - S - 152 (2.84 1.07 2.73) |
| 359 - S - 154 (1.75 1.31 2.49) | 359 - S - 158 (0.83 1.04 2.76) | 359 - S - 248 (1.28 0.90 2.90) | 359 - S - 429 (2.78 1.61 2.19) |
| 360 - S - 125 (1.83 1.01 2.81) | 360 - S - 152 (2.80 1.05 2.77) | 360 - S - 154 (1.71 1.29 2.53) | 360 - S - 248 (1.24 0.88 2.94) |
| 360 - S - 429 (2.74 1.59 2.23) | 361 - S - 101 (1.09 1.09 2.83) | 361 - S - 109 (1.51 1.17 2.75) | 361 - S - 125 (2.11 1.40 2.53) |
| 361 - S - 150 (2.53 1.09 2.83) | 361 - S - 151 (2.44 0.97 2.96) | 361 - S - 152 (3.08 1.43 2.49) | 361 - S - 153 (2.46 1.16 2.76) |
| 361 - S - 154 (2.00 1.68 2.25) | 361 - S - 158 (1.07 1.41 2.52) | 361 - S - 183 (1.19 1.07 2.85) | 361 - S - 213 (1.39 0.92 3.00) |
| 361 - S - 244 (1.20 1.10 2.82) | 361 - S - 247 (1.59 0.99 2.93) | 361 - S - 248 (1.52 1.27 2.66) | 361 - S - 429 (3.02 1.98 1.94) |
| 368 - S - 150 (0.96 1.20 4.40) | 368 - S - 151 (0.87 1.08 4.53) | 368 - S - 152 (1.51 1.55 4.06) | 368 - S - 153 (0.89 1.27 4.34) |
| 368 - S - 429 (1.45 2.09 3.52) | 369 - S - 82 (0.81 1.86 1.92) | 369 - S - 101 (1.20 1.05 2.72) | 369 - S - 109 (1.62 1.13 2.64) |
| 369 - S - 125 (2.22 1.36 2.42) | 369 - S - 150 (2.64 1.05 2.72) | 369 - S - 151 (2.55 0.93 2.85) | 369 - S - 152 (3.19 1.40 2.38) |
| 369 - S - 153 (2.57 1.12 2.65) | 369 - S - 154 (2.11 1.64 2.14) | 369 - S - 158 (1.18 1.37 2.41) | 369 - S - 183 (1.30 1.04 2.74) |
| 369 - S - 213 (1.50 0.88 2.89) | 369 - S - 244 (1.31 1.06 2.71) | 369 - S - 247 (1.70 0.95 2.82) | 369 - S - 248 (1.63 1.23 2.55) |
| 369 - S - 429 (3.13 1.94 1.83) | 370 - S - 125 (1.31 1.34 3.33) | 370 - S - 150 (1.73 1.04 3.64) | 370 - S - 151 (1.64 0.91 3.76) |
| 370 - S - 152 (2.28 1.38 3.29) | 370 - S - 153 (1.66 1.11 3.57) | 370 - S - 154 (1.19 1.62 3.05) | 370 - S - 429 (2.22 1.93 2.75) |
| 372 **-** S - 125 (1.08 1.19 3.56) | 372 - S - 150 (1.50 0.89 3.86) | 372 - S - 152 (2.05 1.23 3.52) | 372 - S - 153 (1.43 0.96 3.80) |
| 372 - S - 154 (0.96 1.47 3.28) | 372 - S - 429 (1.99 1.77 2.98) | 373 - S - 109 (1.08 1.10 3.18) | 373 - S - 125 (1.69 1.33 2.95) |
| 373 - S - 150 (2.11 1.02 3.26) | 373 - S - 151 (2.02 0.90 3.38) | 373 - S - 152 (2.66 1.37 2.91) | 373 - S - 153 (2.04 1.09 3.19) |
| 373 - S - 154 (1.57 1.61 2.67) | 373 - S - 213 (0.97 0.85 3.43) | 373 - S - 247 (1.17 0.92 3.36) | 373 - S - 248 (1.10 1.20 3.08) |
| 373 - S - 429 (2.60 1.91 2.37) | 374 - S - 109 (0.86 1.01 3.39) | 374 - S - 125 (1.47 1.24 3.17) | 374 - S - 150 (1.89 0.93 3.47) |
| 374 - S - 151 (1.80 0.81 3.60) | 374 - S - 152 (2.44 1.28 3.13) | 374 - S - 153 (1.82 1.00 3.41) | 374 - S - 154 (1.35 1.52 2.89) |
| 374 - S - 247 (0.95 0.84 3.57) | 374 - S - 248 (0.88 1.11 3.30) | 374 - S - 429 (2.38 1.82 2.59) | 375 - S - 101 (1.18 0.93 2.73) |
| 375 - S - 109 (1.60 1.01 2.65) | 375 - S - 125 (2.21 1.24 2.43) | 375 - S - 150 (2.63 0.93 2.73) | 375 - S - 151 (2.54 0.81 2.86) |
| 375 - S - 152 (3.18 1.28 2.39) | 375 - S - 153 (2.56 1.00 2.67) | 375 - S - 154 (2.09 1.52 2.15) | 375 - S - 158 (1.17 1.25 2.42) |
| 375 - S - 183 (1.29 0.92 2.75) | 375 - S - 244 (1.30 0.95 2.72) | 375 - S - 247 (1.69 0.83 2.83) | 375 - S - 248 (1.62 1.11 2.56) |
| 375 - S - 429 (3.12 1.82 1.85) | 377 - S - 82 (0.85 1.04 1.88) | 377 - S - 154 (2.15 0.82 2.09) | 377 - S - 429 (3.18 1.12 1.79) |
| 378 - S - 429 (2.86 1.08 2.10) | 379 - S - 154 (1.28 0.95 2.96) | 379 - S - 429 (2.31 1.26 2.66) | 380 - S - 429 (2.47 0.97 2.50) |
| 384 - S - 429 (2.82 0.99 2.15) | 386 - S - 82 (1.17 1.30 1.55) | 386 - S - 152 (3.56 0.84 2.01) | 386 - S - 154 (2.47 1.08 1.77) |
| 386 - S - 158 (1.55 0.81 2.04) | 386 - S - 429 (3.50 1.38 1.47) | 388 - S - 125 (2.03 0.85 2.60) | 388 - S - 152 (3.00 0.89 2.57) |
| 388 - S - 154 (1.92 1.13 2.33) | 388 - S - 158 (0.99 0.86 2.60) | 388 - S - 429 (2.95 1.43 2.02) | 391 - S - 154 (1.27 0.96 2.97) |
| 391 - S - 429 (2.30 1.27 2.67) | 397 - S - 429 (2.14 0.95 2.82) | 398 - S - 429 (2.24 0.99 2.73) | 401 - S - 429 (2.30 0.90 2.67) |
| 404 - S - 82 (0.98 1.95 1.74) | 404 - S - 86 (0.95 1.06 2.62) | 404 - S - 101 (1.37 1.14 2.54) | 404 - S - 109 (1.79 1.22 2.46) |
| 404 - S - 125 (2.40 1.45 2.24) | 404 - S - 150 (2.82 1.14 2.54) | 404 - S - 151 (2.73 1.02 2.67) | 404 - S - 152 (3.37 1.49 2.20) |
| 404 - S - 153 (2.75 1.21 2.48) | 404 - S - 154 (2.28 1.73 1.96) | 404 - S - 158 (1.36 1.46 2.23) | 404 - S - 183 (1.48 1.13 2.56) |
| 404 - S - 213 (1.68 0.97 2.72) | 404 - S - 244 (1.49 1.16 2.53) | 404 - S - 247 (1.88 1.04 2.64) | 404 - S - 248 (1.81 1.32 2.37) |
| 404 - S - 429 (3.31 2.03 1.66) | 405 - S - 101 (1.03 0.89 2.89) | 405 - S - 109 (1.45 0.97 2.81) | 405 - S - 125 (2.05 1.20 2.58) |
| 405 - S - 150 (2.47 0.89 2.89) | 405 - S - 152 (3.03 1.23 2.55) | 405 - S - 153 (2.41 0.96 2.82) | 405 - S - 154 (1.94 1.48 2.31) |
| 405 - S - 158 (1.01 1.21 2.58) | 405 - S - 183 (1.13 0.87 2.91) | 405 - S - 244 (1.14 0.90 2.88) | 405 - S - 248 (1.46 1.07 2.72) |
| 405 - S - 429 (2.97 1.78 2.00) | 406 - S - 82 (0.92 1.73 1.80) | 406 - S - 86 (0.89 0.85 2.69) | 406 - S - 101 (1.31 0.93 2.61) |
| 406 - S - 109 (1.73 1.01 2.53) | 406 - S - 125 (2.34 1.23 2.30) | 406 - S - 150 (2.76 0.93 2.61) | 406 - S - 151 (2.66 0.80 2.73) |
| 406 - S - 152 (3.31 1.27 2.26) | 406 - S - 153 (2.69 1.00 2.54) | 406 - S - 154 (2.22 1.51 2.02) | 406 - S - 158 (1.29 1.24 2.29) |
| 406 - S - 183 (1.42 0.91 2.63) | 406 - S - 244 (1.43 0.94 2.60) | 406 - S - 247 (1.81 0.83 2.71) | 406 - S - 248 (1.75 1.10 2.43) |
| 406 - S - 429 (3.25 1.82 1.72) | 407 - S - 82 (1.05 1.41 1.67) | 407 - S - 125 (2.47 0.91 2.17) | 407 - S - 152 (3.44 0.95 2.13) |
| 407 - S - 154 (2.35 1.19 1.89) | 407 - S - 158 (1.43 0.92 2.16) | 407 - S - 429 (3.38 1.49 1.59) | 408 - S - 101 (1.12 1.16 2.79) |
| 408 - S - 109 (1.54 1.24 2.71) | 408 - S - 125 (2.15 1.46 2.49) | 408 - S - 150 (2.57 1.16 2.79) | 408 - S - 151 (2.48 1.03 2.92) |
| 408 - S - 152 (3.12 1.50 2.45) | 408 - S - 153 (2.50 1.23 2.73) | 408 - S - 154 (2.03 1.74 2.21) | 408 - S - 158 (1.11 1.47 2.48) |
| 408 - S - 183 (1.23 1.14 2.81) | 408 - S - 213 (1.43 0.99 2.97) | 408 - S - 244 (1.24 1.17 2.78) | 408 - S - 247 (1.63 1.06 2.89) |
| 408 - S - 248 (1.56 1.33 2.62) | 408 - S - 429 (3.06 2.04 1.91) | 409 - S - 82 (0.88 1.65 1.84) | 409 - S - 101 (1.27 0.85 2.65) |
| 409 - S - 109 (1.69 0.93 2.57) | 409 - S - 125 (2.30 1.15 2.34) | 409 - S - 150 (2.72 0.85 2.65) | 409 - S - 152 (3.27 1.19 2.30) |
| 409 - S - 153 (2.65 0.91 2.58) | 409 - S - 154 (2.18 1.43 2.06) | 409 - S - 158 (1.25 1.16 2.33) | 409 - S - 183 (1.38 0.83 2.66) |
| 409 - S - 244 (1.39 0.86 2.64) | 409 - S - 248 (1.71 1.02 2.47) | 409 - S - 429 (3.21 1.73 1.76) | 410 - S - 82 (0.99 1.75 1.74) |
| 410 - S - 86 (0.96 0.87 2.62) | 410 - S - 101 (1.38 0.95 2.54) | 410 - S - 109 (1.80 1.03 2.46) | 410 - S - 125 (2.40 1.25 2.24) |
| 410 - S - 150 (2.82 0.95 2.54) | 410 - S - 151 (2.73 0.82 2.67) | 410 - S - 152 (3.37 1.29 2.20) | 410 - S - 153 (2.75 1.02 2.47) |
| 410 - S - 154 (2.29 1.53 1.96) | 410 - S - 158 (1.36 1.26 2.23) | 410 - S - 183 (1.48 0.93 2.56) | 410 - S - 244 (1.49 0.96 2.53) |
| 410 - S - 247 (1.88 0.85 2.64) | 410 - S - 248 (1.81 1.12 2.37) | 410 - S - 429 (3.31 1.84 1.65) | 411 - S - 82 (1.06 1.40 1.67) |
| 411 - S - 125 (2.47 0.90 2.16) | 411 - S - 152 (3.45 0.94 2.13) | 411 - S - 154 (2.36 1.18 1.88) | 411 - S - 158 (1.43 0.91 2.15) |
| 411 - S - 429 (3.39 1.48 1.58) | 413 - S - 82 (1.47 1.08 1.26) | 413 - S - 154 (2.77 0.86 1.47) | 413 - S - 429 (3.80 1.17 1.17) |
| 414 - S - 82 (1.48 3.39 1.24) | 414 - S - 86 (1.45 2.50 2.13) | 414 - S - 101 (1.87 2.59 2.05) | 414 - S - 107 (2.49 2.13 2.50) |
| 414 - S - 109 (2.29 2.67 1.97) | 414 - S - 125 (2.90 2.89 1.74) | 414 - S - 147 (1.24 2.60 2.03) | 414 - S - 150 (3.32 2.59 2.05) |
| 414 - S - 151 (3.22 2.46 2.17) | 414 - S - 152 (3.87 2.93 1.70) | 414 - S - 153 (3.25 2.65 1.98) | 414 - S - 154 (2.78 3.17 1.46) |
| 414 - S - 158 (1.85 2.90 1.73) | 414 - S - 165 (2.16 2.21 2.43) | 414 - S - 183 (1.98 2.57 2.06) | 414 - S - 213 (2.17 2.41 2.22) |
| 414 - S - 244 (1.99 2.60 2.04) | 414 - S - 247 (2.37 2.49 2.15) | 414 - S - 248 (2.31 2.76 1.87) | 414 - S - 429 (3.81 3.47 1.16) |
| 415 - S - 429 (2.25 1.04 2.72) | 419 - S - 154 (1.83 0.89 2.41) | 419 - S - 429 (2.86 1.19 2.11) | 420 - S - 125 (1.28 0.82 3.36) |
| 420 - S - 152 (2.25 0.85 3.32) | 420 - S - 154 (1.16 1.09 3.08) | 420 - S - 429 (2.19 1.40 2.78) | 422 - S - 154 (1.92 0.98 2.32) |
| 422 - S - 429 (2.95 1.29 2.02) | 423 - S - 429 (2.97 1.10 2.00) | 424 - S - 429 (3.05 1.07 1.92) | 425 - S - 82 (1.72 1.97 1.00) |
| 425 - S - 86 (1.69 1.08 1.89) | 425 - S - 101 (2.11 1.16 1.81) | 425 - S - 109 (2.53 1.24 1.72) | 425 - S - 125 (3.14 1.47 1.50) |
| 425 - S - 147 (1.48 1.18 1.79) | 425 - S - 150 (3.56 1.16 1.80) | 425 - S - 151 (3.47 1.04 1.93) | 425 - S - 152 (4.11 1.51 1.46) |
| 425 - S - 153 (3.49 1.23 1.74) | 425 - S - 154 (3.02 1.75 1.22) | 425 - S - 158 (2.10 1.48 1.49) | 425 - S - 183 (2.22 1.15 1.82) |
| 425 - S - 213 (2.42 0.99 1.98) | 425 - S - 244 (2.23 1.18 1.79) | 425 - S - 247 (2.62 1.06 1.90) | 425 - S - 248 (2.55 1.34 1.63) |
| 425 - S - 429 (4.05 2.05 0.92) | 426 - S - 152 (3.09 0.83 2.49) | 426 - S - 154 (2.00 1.07 2.25) | 426 - S - 158 (1.07 0.80 2.52) |
| 426 - S - 429 (3.03 1.38 1.94) | 427 - S - 82 (1.01 1.87 1.72) | 427 - S - 86 (0.98 0.99 2.60) | 427 - S - 101 (1.40 1.07 2.52) |
| 427 - S - 109 (1.82 1.15 2.44) | 427 - S - 125 (2.42 1.38 2.21) | 427 - S - 150 (2.85 1.07 2.52) | 427 - S - 151 (2.75 0.95 2.64) |
| 427 - S - 152 (3.40 1.41 2.18) | 427 - S - 153 (2.78 1.14 2.45) | 427 - S - 154 (2.31 1.66 1.93) | 427 - S - 158 (1.38 1.39 2.20) |
| 427 - S - 183 (1.51 1.05 2.54) | 427 - S - 213 (1.70 0.90 2.69) | 427 - S - 244 (1.51 1.08 2.51) | 427 - S - 247 (1.90 0.97 2.62) |
| 427 - S - 248 (1.84 1.25 2.34) | 427 - S - 429 (3.34 1.96 1.63) | 428 - S - 82 (1.47 1.91 1.26) | 428 - S - 86 (1.44 1.03 2.14) |
| 428 - S - 101 (1.86 1.11 2.06) | 428 - S - 109 (2.28 1.19 1.98) | 428 - S - 125 (2.88 1.41 1.75) | 428 - S - 147 (1.22 1.12 2.05) |
| 428 - S - 150 (3.30 1.11 2.06) | 428 - S - 151 (3.21 0.98 2.18) | 428 - S - 152 (3.86 1.45 1.72) | 428 - S - 153 (3.24 1.18 1.99) |
| 428 - S - 154 (2.77 1.69 1.47) | 428 - S - 158 (1.84 1.42 1.74) | 428 - S - 183 (1.97 1.09 2.08) | 428 - S - 213 (2.16 0.94 2.23) |
| 428 - S - 244 (1.97 1.12 2.05) | 428 - S - 247 (2.36 1.01 2.16) | 428 - S - 248 (2.29 1.28 1.88) | 428 - S - 429 (3.80 2.00 1.17) |
| 430 - S - 109 (0.84 1.01 3.42) | 430 - S - 125 (1.45 1.24 3.19) | 430 - S - 150 (1.87 0.93 3.50) | 430 - S - 151 (1.78 0.81 3.62) |
| 430 - S - 152 (2.42 1.27 3.15) | 430 - S - 153 (1.80 1.00 3.43) | 430 - S - 154 (1.33 1.52 2.91) | 430 - S - 247 (0.93 0.83 3.60) |
| 430 - S - 248 (0.86 1.11 3.32) | 430 - S - 429 (2.36 1.82 2.61) | 431 - S - 109 (1.15 1.32 3.11) | 431 - S - 125 (1.76 1.55 2.88) |
| 431 - S - 150 (2.18 1.24 3.19) | 431 - S - 151 (2.09 1.11 3.31) | 431 - S - 152 (2.73 1.58 2.84) | 431 - S - 153 (2.11 1.31 3.12) |
| 431 - S - 154 (1.64 1.82 2.60) | 431 - S - 165 (1.02 0.86 3.57) | 431 - S - 183 (0.84 1.22 3.20) | 431 - S - 213 (1.04 1.07 3.36) |
| 431 - S - 244 (0.85 1.25 3.17) | 431 - S - 247 (1.24 1.14 3.28) | 431 - S - 248 (1.17 1.41 3.01) | 431 - S - 429 (2.67 2.13 2.30) |
| 432 - S - 107 (0.81 0.85 4.19) | 432 - S - 125 (1.21 1.61 3.43) | 432 - S - 150 (1.63 1.30 3.73) | 432 - S - 151 (1.54 1.18 3.86) |
| 432 - S - 152 (2.18 1.65 3.39) | 432 - S - 153 (1.56 1.37 3.66) | 432 - S - 154 (1.10 1.89 3.15) | 432 - S - 429 (2.12 2.19 2.84) |
| 433 - S - 109 (0.89 1.15 3.37) | 433 - S - 125 (1.50 1.38 3.14) | 433 - S - 150 (1.92 1.07 3.45) | 433 - S - 151 (1.82 0.95 3.57) |
| 433 - S - 152 (2.47 1.41 3.10) | 433 - S - 153 (1.85 1.14 3.38) | 433 - S - 154 (1.38 1.66 2.86) | 433 - S - 247 (0.97 0.97 3.55) |
| 433 - S - 248 (0.91 1.25 3.27) | 433 - S - 429 (2.41 1.96 2.56) | 434 - S - 107 (0.98 1.03 4.02) | 434 - S - 125 (1.38 1.80 3.25) |
| 434 - S - 150 (1.80 1.49 3.56) | 434 - S - 151 (1.71 1.36 3.69) | 434 - S - 152 (2.36 1.83 3.22) | 434 - S - 153 (1.74 1.56 3.49) |
| 434 - S - 154 (1.27 2.07 2.98) | 434 - S - 247 (0.86 1.39 3.66) | 434 - S - 429 (2.30 2.38 2.67) | 435 - S - 107 (0.90 1.05 4.10) |
| 435 - S - 125 (1.30 1.81 3.34) | 435 - S - 150 (1.72 1.51 3.65) | 435 - S - 151 (1.63 1.38 3.77) | 435 - S - 152 (2.27 1.85 3.30) |
| 435 - S - 153 (1.65 1.58 3.58) | 435 - S - 154 (1.18 2.09 3.06) | 435 - S - 429 (2.21 2.40 2.76) | 437 - S - 125 (1.12 0.96 3.52) |
| 437 - S - 152 (2.09 1.00 3.48) | 437 - S - 154 (1.00 1.24 3.24) | 437 - S - 429 (2.03 1.55 2.94) | 440 - S - 150 (1.19 1.24 4.17) |
| 440 - S - 151 (1.10 1.12 4.30) | 440 - S - 152 (1.75 1.59 3.83) | 440 - S - 153 (1.13 1.31 4.10) | 440 - S - 429 (1.69 2.13 3.28) |
| 441 - S - 125 (0.84 0.91 3.79) | 441 - S - 152 (1.82 0.94 3.76) | 441 - S - 429 (1.76 1.49 3.21) | 445 - S - 154 (1.52 1.00 2.72) |
| 445 - S - 429 (2.55 1.30 2.42) | 447 - S - 125 (0.83 1.09 3.81) | 447 - S - 152 (1.80 1.13 3.77) | 447 - S - 153 (1.18 0.85 4.04) |
| 447 - S - 429 (1.74 1.67 3.22) | 448 - S - 101 (0.87 0.90 3.05) | 448 - S - 109 (1.29 0.98 2.97) | 448 - S - 125 (1.89 1.20 2.75) |
| 448 - S - 150 (2.31 0.90 3.05) | 448 - S - 152 (2.86 1.24 2.71) | 448 - S - 153 (2.24 0.97 2.98) | 448 - S - 154 (1.78 1.48 2.47) |
| 448 - S - 158 (0.85 1.21 2.74) | 448 - S - 183 (0.97 0.88 3.07) | 448 - S - 244 (0.98 0.91 3.04) | 448 - S - 248 (1.30 1.07 2.88) |
| 448 - S - 429 (2.80 1.79 2.16) | 449 - S - 154 (0.98 0.97 3.27) | 449 - S - 429 (2.00 1.27 2.96) | 450 - S - 125 (1.47 0.92 3.17) |
| 450 - S - 152 (2.44 0.96 3.13) | 450 - S - 154 (1.35 1.20 2.89) | 450 - S - 429 (2.38 1.50 2.59) | 451 - S - 150 (1.17 0.90 4.19) |
| 451 - S - 152 (1.72 1.24 3.85) | 451 - S - 153 (1.10 0.96 4.13) | 451 - S - 429 (1.66 1.78 3.31) | 452 - S - 429 (1.61 1.28 3.36) |
| 456 - S - 429 (2.61 0.83 2.36) | 457 - S - 429 (2.16 0.82 2.81) | 460 - S - 154 (0.85 0.84 3.39) | 460 - S - 429 (1.88 1.14 3.09) |
| 466 - S - 154 (1.76 0.87 2.49) | 466 - S - 429 (2.78 1.18 2.18) | 467 - S - 154 (1.45 0.91 2.79) | 467 - S - 429 (2.48 1.22 2.49) |
| 468 - S - 154 (1.25 0.91 2.99) | 468 - S - 429 (2.28 1.22 2.69) | 471 - S - 429 (1.58 0.83 3.39) | |

In einer Ausführungsform werden an die chemisch substituierbaren Positionen der so erhaltenen organischen Moleküle weitere Reste R angefügt, um die Löslichkeit der Emitter zu steigern und/oder die Polymerisierbarkeit zu ermöglichen ohne dabei die elektronischen Eigenscahften des Moleküls signifikant zu verändern, sodass auch bei Verwendung von R ein Emitter vorliegt, wobei
jedes R und bei jedem Auftreten gleich oder verschieden ist H, Deuterium, F, Cl, Br, I, N(R²)₂, CN, CF₃, NO₂, OH, COOH, COOR², CO(NR²)₂, Si(R²)₃, B(OR²)₂, C(=O)R², P(=O)(R²)₂, S(=O)R², S(=O)₂R², OSO₂R², eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination dieser Systeme; dabei können zwei oder mehrere dieser Substituenten R¹ auch miteinander ein mono- oder polycyclisches, aliphatisches, aromatisches und/oder benzoannelliertes Ringsystem bilden;
R² bei jedem Auftreten gleich oder verschieden ist H, Deuterium, F, Cl, Br, I, N(R³)₂, CN, CF₃, NO₂, OH, COOH, COOR³, CO(NR³)₂, Si(R³)₃, B(OR³)₂, C(=O)R³, P(=O)(R³)₂, S(=O)R³, S(=O)₂R³, OSO₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C=C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination dieser Systeme; dabei können zwei oder mehrere dieser Substituenten R² auch miteinander ein mono- oder polycyclisches, aliphatisches, aromatisches und/oder benzoannelliertes Ringsystem bilden;
R³ bei jedem Auftreten gleich oder verschieden ist H, Deuterium, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R³ auch miteinander ein mono- oder polycyclisches, aliphatisches Ringsystem bilden.

Polymerisierbare Reste sind solche Reste, die polymerisierbare funktionelle Einheiten tragen, die mit sich selbst homopolymerisiert oder mit anderen Monomeren copolymiersiert werden können. Somit können die erfindungsgemäßen Moleküle als Polymer mit folgenden Wiederholungseinheiten der Formeln 4 und 5 erhalten werden, die als Polymere in der lichtemittierenden Schicht des optoelektronischen Bauelements Verwendung finden können.

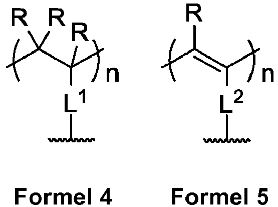

In Formel 4 und 5 stehen L1 und L2 für gleiche oder verschiedene Linkergruppen, die 0 bis 20, bevorzugt 1 bis 15, stärker bevorzugt 2 bis 10 Kohlenstoffatome aufweist, und wobei die gewellte Linie die Position kennzeichnet, über die die Linkergruppe an das organische Molekül der Formel 1 angebunden ist. In einer bevorzugten Ausführungsform weist die Linkergruppe L1 und/oder L2 eine Form -X-L3- auf, wobei X für O oder S steht und L3 für eine Linkergruppe ausgewählt aus der Gruppe aus einer substituierten und unsubstituierten Alkylengruppe (linear, verzweigt oder cyclisch) und einer substituierten und unsubstituierten Arylengruppe, bevorzugt eine substituierte oder unsubstituierte Alkylengruppe mit 1 bis 10 Kohlenstoffatomen oder eine substituierte oder unsubstituierte Phenylengruppe, wobei auch Kombinationen möglich sind. In einer weiteren Ausführungsform weist die Linkergruppe L1 und/oder L2 eine Form -C(=O)O- auf.

Vorteilhafte Ausführungsformen der Wiederholungseinheiten sind Strukturen der Formeln 6 bis 11:

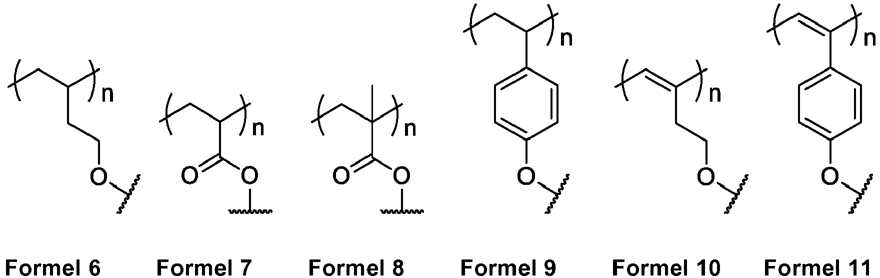

Zur Herstellung der Polymere, die die bevorzugten Wiederholungseinheiten gemäß Formel 6 bis 11 aufweisen, werden die polymerisierbaren funktionellen Einheiten über eine Linkergruppe der Formeln 12 bis 17, die eine Hydroxyleinheit aufweisen, an das organische Molekül der Formel 1 oder 2 angebunden und die daraus resultierenden Verbindungen mit sich selbst homopolymerisiert oder mit anderen geeigneten Monomeren copolymerisiert.

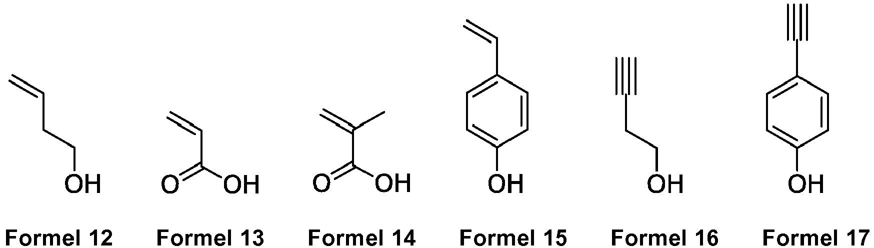

Polymere, die eine Einheit gemäß Formel 4 oder Formel 5 aufweisen, können dabei entweder ausschließlich Wiederholungseinheiten mit einer Struktur der allgemeinen Formel 4 oder 5, oder Wiederholungseinheiten mit einer anderen Struktur aufweisen. Beispiele von Wiederholungseinheiten mit anderen Strukturen weisen Einheiten auf, die sich aus entsprechenden Monomeren ergeben, die typischerweise in Copolymerisationen eingesetzt oder verwendet werden. Beispiele für derartige Wiederholungseinheiten, die sich aus Monomeren ergeben, sind Wiederholungseinheiten, die ungesättigte Einheiten wie Ethylen oder Styrol aufweisen.

Eine Ausführungsform der Erfindung betrifft organischen Moleküle, welche
- einen ΔE(S₁-T₁)-Wert zwischen dem untersten angeregten Singulett (S₁)- und dem darunter liegenden Triplett (T₁)-Zustand von kleiner als 5000 cm⁻¹, bevorzugt kleiner als 3000 cm⁻¹ aufweisen und/oder
- eine Emissionslebensdauer von höchstens 50 µs aufweisen.

Die Erfindung betrifft in einem Aspekt die Verwendung eines erfindungsgemäßen organischen Moleküls als Emitter oder Absorber in einem optoelektronischen Bauelement, das insbesondere durch ein Vakuumverdampfungsverfahren oder aus Lösung hergestellt wird, wobei das optoelektronische Bauelement insbesondere ausgewählt ist aus der Gruppe bestehend aus:
- Organischen Licht-emittierenden Bauteilen (OLEDs),
- Licht-emittierenden elektrochemischen Zellen,
- OLED-Sensoren, insbesondere in nicht hermetisch nach außen abgeschirmten Gas- und Dampf-Sensoren,
- organischen Solarzellen,
- Organischen Feldeffekttransistoren,
- Organischen Lasern und
- Down-Konversions-Elementen.

Der Anteil des erfindungsgemäßen organischen Moleküls am Emitter oder Absorber beträgt in einer Ausführungsform 1 % bis 99 % (Gew%), insbesondere beträgt der Anteil am Emitter in optischen Licht emittierenden Bauelementen, in OLEDs insbesondere zwischen 5 % und 80 %. In einer alternativen Ausführungsform beträgt der Anteil des organischen Moleküls am Emitter oder Absorber 100 %.

Die Erfindung betrifft in einem weiteren Aspekt optoelektronische Bauelemente, aufweisend ein erfindungsgemäßes organisches Molekül, wobei das optoelektronische Bauelement insbesondere ausgeformt ist als ein Bauelement ausgewählt aus der Gruppe bestehend aus organischem lichtemittierendem Bauelement, organischer Diode, organischer Solarzelle, organischem Transistor, organischer lichtemittierender Diode, Licht-emittierender elektrochemischer Zelle, organischem Feldeffekttransistor und organischem Laser.

In einer Ausführungsform des Bauelements wird das organische Molekül als Emissionsmaterial in einer Emissionsschicht eingesetzt wird, wobei sie in Kombination mit mindestens einem Matrixmaterial oder insbesondere als Reinschicht eingesetzt werden kann. In einer Ausführungsform beträgt dabei der Anteil des organischen Moleküls als Emissionsmaterial in einer Emissionsschicht in optischen Licht emittierenden Bauelementen, insbesondere in OLEDs, zwischen 5 % und 80 % (Gew%).

In einer weiteren Ausführungsform des erfindungsgemäßen Bauelements ist die ein erfindungsgemäßes organisches Molekül aufweisende lichtemittierende Schicht auf ein Substrat aufgebracht.

Eine Ausführungsform des optoelektronischen Bauelements weist ein erfindungsgemäßes organisches Molekül auf, wobei das Bauelement ein Substrat, eine Anode und eine Kathode aufweist, wobei die Anode und die Kathode auf das Substrat aufgebracht ist, und die lichtemittierende Schicht zwischen Anode und Kathode angeordnet ist.

In einer Ausführungsform betrifft die Erfindung ein optoelektronisches Bauelement, bei dem die lichtemittierende Schicht ausschließlich ein erfindungsgemäßes organisches Molekül in 100 % Konzentration aufweist, wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement neben dem erfindungsgemäßen organischen Molekül auch ein Hostmaterial auf, dessen Triplett (T1)-und Singulett (S1)-Energieniveaus energetisch höher liegen als die Triplett (T1)- und Singulett (S1)-Energieniveaus des organischen Moleküls, wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement ein Substrat, eine Anode, eine Kathode und mindestens je eine löcherinjizierende und eine elektroneninjizierende Schicht und mindestens eine lichtemittierende Schicht auf, wobei die mindestens eine lichtemittierende Schicht ein erfindungsgemäßes organisches Molekül und ein Hostmaterial aufweist, dessen Triplett (T1)- und Singulett (S1)-Energieniveaus energetisch höher liegen als die Triplett (T1)- und Singulett (S1)-Energieniveaus des organischen Moleküls, und wobei die Anode und die Kathode auf das Substrat aufgebracht ist, und die löcher- und elektroneninjizierende Schicht zwischen Anode und Kathode aufgebracht ist und die lichtemittierende Schicht zwischen löcher- und elektroneninjizierende Schicht aufgebracht ist.

In einer weiteren Ausführungsform weist das optoelektronische Bauelement ein Substrat, eine Anode, eine Kathode und mindestens je eine löcherinjizierende und eine elektroneninjizierende Schicht, und mindestens je eine löchertransportierende und eine elektronentransportierende Schicht, und mindestens eine lichtemittierende Schicht auf, wobei die mindestens eine lichtemittierende Schicht ein erfindungsgemäßes organisches Molekül sowie ein Hostmaterial aufweist, dessen Triplett (T1)- und Singulett (S1)-Energieniveaus energetisch höher liegen als die Triplett (T1)- und Singulett (S1)-Energieniveaus des organischen Moleküls, und wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

Die Erfindung betrifft in einem Aspekt ein lichtemittierendes Material, aufweisend ein erfindungsgemäßes organisches Molekül und ein Hostmaterial, wobei die Triplett (T1)- und Singulett (S1)-Energieniveaus des Hostmaterials energetisch höher liegen als die Triplett (T1)- und Singulett (S1)-Energieniveaus des organischen Moleküls, und wobei das organische Molekül Fluoreszenz oder thermisch aktivierte verzögerte Fluoreszenz (TADF) emittiert, und einen deltaE(S1-T1)-Wert zwischen dem untersten angeregten Singulett (S1)-und dem darunter liegenden Triplett (T1)-Zustand von kleiner als 3000 cm⁻¹ aufweist.

Ein Aspekt der Erfindung betrifft ein Verfahren zur Herstellung eines optoelektronischen Bauelements aufweisend ein erfindungsgemäßes organisches Molekül. In einer Ausführungsform ist das Verfahren gekennzeichnet durch das Aufbringen des organischen Moleküls auf einen Träger, wobei das Aufbringen insbesondere nass-chemisch, mittels kolloidaler Suspension oder mittels Sublimation erfolgt.

In einer weiteren bevorzugten des Verfahrens wird mindestens eine Schicht
- mit einem Sublimationsverfahren beschichtet
- mit einem OVPD (Organic Vapor Phase Deposition) Verfahren beschichtet
- mit Hilfe einer Trägergassublimation beschichtet oder
- aus Lösung oder mit einem beliebigen Druckverfahren hergestellt.

Ein Aspekt der Erfindung betrifft ein Verfahren zur Veränderung der Emissions- und/oder Absorptionseigenschaften eines elektronischen Bauelements, wobei ein erfindungsgemäßes organisches Molekül in ein Matrixmaterial zur Leitung von Elektronen oder Löchern in einem optoelektronischen Bauelement eingebracht wird.

Die Erfindung betrifft zudem in einem weiteren Aspekt die Verwendung eines erfindungsgemäßen Moleküls zur Umwandlung von UV-Strahlung oder von blauem Licht in sichtbares Licht, insbesondere in grünes, gelbes oder rotes Licht (down-Konversion), insbesondere in einem optoelektronischen Bauelement der hier beschriebenen Art.

In einer weiteren Ausführungsform des optoelektronischen Bauelements bilden ein organisches Molekül gemäß Formel 1 und ein funktionelles Material, beispielsweise in Form eines weiteren Emitter-Materials, eines Host-Materials, oder eines weiteren organisches Moleküls, welches zur Bildung eines Exciplex mit dem Molekül gemäß Formel 1 befähigt ist, einen Exciplex. Funktionelle Materialien sind beispielsweise Hostmaterialien wie MCP, Elektronentransportmaterialien wie TPBI und Lochtransportmaterialien wie NPD oder MTDATA. Exciplexe sind Addukte aus elektronisch angeregten Molekülen und solchen im elektronischen Grundzustand, die zur Lichtemission fähig sind.

### Figuren

Es zeigen
- Figur 1:: Schematische Darstellung des Aufbaus einer organischen lichtemittierenden Diode (OLED).

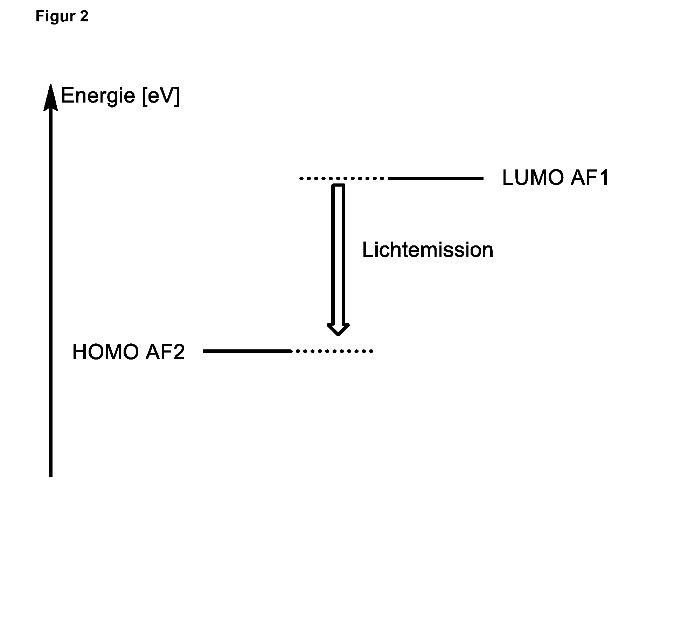

- Figur 2:: Schematische Darstellung des Energieniveaudiagrammes (relative Energie in eV) eines erfindungsgemäßen Emittermoleküls (Lichtemission resultiert aus dem Übergang LUMO AF1 hin zu HOMO AF2).

## Patentansprüche

**1.** Organisches Molekül, aufweisend eine Struktur der Formel 1 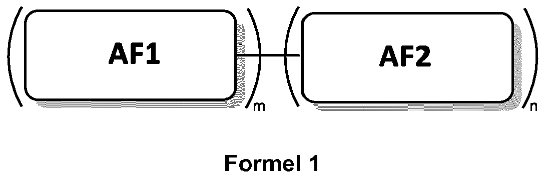 wobei gilt:
n und m sind unabhängig voneinander ausgewählt aus einer ganzzahligen Zahl von 1 bis 3,
AF1 ist eine erste chemische Einheit, aufweisend ein konjugiertes System, insbesondere mindestens sechs in Konjugation stehende π-Elektronen (z. B. in Form mindestens eines aromatischen Systems);
AF2 ist eine zweite chemische Einheit, aufweisend ein konjugiertes System, insbesondere mindestens sechs in Konjugation stehende π-Elektronen (z. B. in Form mindestens eines aromatischen Systems);
AF1 # AF2;
und wobei mindestens eine der beiden chemischen Einheiten AF1 oder AF2 eine Struktur
gemäß Formel 1a aufweist 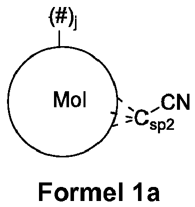
und wobei bevorzugt nur eine der beiden chemischen Einheiten AF1 oder AF2 ausgewählt ist aus einer der durch die Formel 1a beschriebenen Struktur;
und wobei bevorzugt die jeweils andere chemische Einheit AF ausgewählt ist aus einer der in Tabelle 2 aufgeführten Strukturen; und wobei diese andere chemische Einheit AF ein Substituent eines Atoms ist, welches direkt benachbart mindestens ein Atom mit einem Substituenten ungleich H oder D aufweist;
wobei in Formel 1a bedeutet:
# Anknüpfungspunkte an weitere chemische Einheit AF;
j ist eine ganze Zahl von 1 bis 5, bevorzugt von 1 bis 3;
Cₛₚ₂ ist ein aromatisches oder olefinisches Kohlenstoffatom und Bestandteil eines olefinischen, aromatischen oder heteroaromatischen Molekülfragments Mol;
und wobei insbesondere
- die Differenz der Energie des HOMO der zweiten chemischen Einheit AF2 und der Energie des HOMO der ersten chemischen Einheit AF1 > 0,8 eV ist (Δ HOMO = HOMO(AF2) - HOMO(AF1) > 0,8 eV);
- die Differenz der Energie des LUMO der zweiten chemischen Einheit AF2 und der Energie des LUMO der ersten chemischen Einheit AF1 > 0,8 eV ist (Δ LUMO = LUMO(AF2) - LUMO(AF1) > 0,8 eV); und/oder
- die Differenz der Energie des LUMO der ersten chemischen Einheit AF1 und der Energie des HOMO der zweiten chemischen Einheit AF2 > 0,9 eV ist (Δ Gap = LUMO (AF1) - HOMO(AF2)> 0,9 eV).

**2.** Organisches Molekül nach Anspruch 1, **dadurch gekennzeichnet dass** eine der beiden chemischen Einheiten AF1 oder AF2 ausgewählt ist aus einer der durch die Formeln 2a bis 2c beschriebenen Strukturen 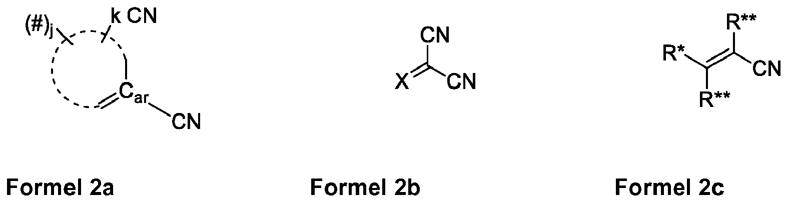 wobei gilt:
# Anknüpfungspunkte an weitere chemische Einheit AF;
k ist eine ganze Zahl von 0 bis 2;
Cₐᵣ ist ein aromatisches Kohlenstoffatom und Bestandteil eines aromatischen oder heteroaromatischen mono-, oder di- oder tri- oder tetrazyklischen annelierten Ringsystems, bevorzugt ausgewählt aus der Gruppe von Phenyl, Naphthyl, Anthracenyl, Pyrrolyl, Thienothienyl, Triphenylenyl und Spirobiphenylenyl;
X ist ausgewählt aus der Gruppe von PPh₃, 4H-Pyran, 9,10-Dihydroacridin, Diphenylmethylen oder 2-Methylen-2,5-dihydrothiazol, wobei weitere chemischen Einheiten AF über diesen Teil des Moleküls angeknüpft sind;
R** ist entweder ein Rest R* oder kennzeichnet die Anknüpfungsposition an eine weitere chemische Einheit AF oder ist eine Nitrilgruppe, wobei ein Rest R** zwingend eine Anknüpfungsposition an eine chemische Einheit AF kennzeichnet; und wobei optional zwei oder mehrere Substituenten R** auch miteinander ein mono- oder polycyclisches, aliphatisches Ringsystem bilden;
R* ist bei jedem Auftreten unabhängig voneinander ausgewählt aus der Gruppe bestehend aus H, Deuterium, Phenyl, Naphthyl, N(R²)₂, -CN, -NC, -SCN, -CF₃, -NO₂, C(=O)OH, C(=O)OR³, C(=O)N(R³)₂, C(=O)SR³, C(=S)SR³, Si(R⁴)₃, B(OR⁵)₂, B(N(R⁶)₂)₂, C(=O)R³, P(=O)(R⁷)₂, As(=O)(R⁷)₂, P(=S)(R⁷)₂, As(=S)(R⁷)₂, S(=O)R³, S=NR³, S(=O)NR³, S(=O)₂NR³, S(=O)₂R³, O-S(=O)₂R³, SF₅, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C=C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, - C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R* auch miteinander ein monozyklisches aliphatisches Ringsystem mit insgesamt fünf oder sechs Ringgliedern bilden;
R² ist bei jedem Auftreten unabhängig voneinander ausgewählt aus der Gruppe bestehend aus H, Deuterium, Phenyl, Naphthyl, CF₃, C(=O)OR³, C(=O)N(R²)₂, Si(R⁴)₃, C(=O)R³, P(=O)(R⁷)₂, As(=O)(R⁷)₂ P(=S)(R⁷)₂, As(=S)(R⁷)₂, S(=O)R³, S(=O)₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C=C-, bzw. eine benachbarte CH₂-Gruppe durch -Si(R⁴)₂-, - Ge(R⁴)₂-, -Sn(R⁴)₂, -C(=O)-, -C(=S)-, -C(=Se)-, -C=N-, -C(=O)O-, -C(=O)N(R³)-, -P(=O)(R⁷)-, - As(=O)(R⁷)-, -P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R² auch miteinander ein mono- oder polycyclisches, aliphatisches, aromatisches und/oder benzoannelliertes Ringsystem bilden;
R³ ist bei jedem Auftreten unabhängig voneinander ausgewählt aus der Gruppe bestehend aus H, Deuterium, Phenyl, Naphthyl, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R³ auch miteinander ein mono- oder polycyclisches, aliphatisches Ringsystem bilden;
R⁴ ist bei jedem Auftreten unabhängig voneinander ausgewählt aus der Gruppe bestehend aus H, Deuterium, Phenyl, Naphthyl, N(R²)₂, CN, CF₃, OH, C(=O)OR³, C(=O)N(R³)₂, C(=O)R³, P(=O)(R⁷)₂, As(=O)(R⁷)₂, P(=S)(R⁷)₂, As(=S)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch - R⁹C=CR⁹-, -C=C-, bzw. eine benachbarte CH₂-Gruppe durch -Si(R⁴)₂-, -Ge(R⁴)₂-, -Sn(R⁴)₂, - C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁴ auch miteinander ein mono- oder polycyclisches, aliphatisches, aromatisches und/oder benzoannelliertes Ringsystem bilden;
R⁵ ist bei jedem Auftreten unabhängig voneinander ausgewählt aus der Gruppe bestehend aus Phenyl, Naphthyl, CF₃, C(=O)R³ P(=O)(R⁷)₂, As(=O)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch - R⁹C=CR⁹-, -C=C-, bzw. eine benachbarte CH₂-Gruppe durch -Si(R⁴)₂-, -Ge(R⁴)₂-, -Sn(R⁴)₂, - C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷)-, -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁵ auch miteinander ein mono- oder polycyclisches, aliphatisches, aromatisches und/oder benzoannelliertes Ringsystem bilden;
R⁶ ist bei jedem Auftreten unabhängig voneinander ausgewählt aus der Gruppe bestehend aus Phenyl, Naphthyl, CF₃, Si(R⁴)₃, C(=O)R³, P(=O)(R⁷)₂, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch - R⁹C=CR⁹-, -C=C-, bzw. eine benachbarte CH₂-Gruppe durch-Si(R⁴)₂-, -Ge(R⁴)₂-, -Sn(R⁴)₂, - C(=O)-, -C(=S)-, -C(=Se)-, -C=N-, -C(=O)O-, -C(=O)N(R³)-, -P(=O)(R⁷)-, -As(=O)(R⁷)-, - P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R* substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R⁶ auch miteinander ein mono- oder polycyclisches, aliphatisches, aromatisches und/oder benzoannelliertes Ringsystem bilden;
R⁷ ist bei jedem Auftreten unabhängig voneinander ausgewählt aus der Gruppe bestehend aus, Phenyl, Naphthyl, N(R²)₂, CN, CF₃, C(=O)OR³, C(=O)N(R³)₂, Si(R⁴)₃, C(=O)R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl- , Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C=C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, -C=N-, -C(=O)O-, - C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, - O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁹ substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination dieser Systeme; dabei können zwei oder mehrere dieser Substituenten R⁷ auch miteinander ein mono- oder polycyclisches, aliphatisches, aromatisches und/oder benzoannelliertes Ringsystem bilden;
R⁸ ist bei jedem Auftreten unabhängig voneinander ausgewählt aus der Gruppe bestehend aus H, Deuterium, Phenyl, Naphthyl, F, CF₃ oder ein aliphatischer, aromatischer und/oder heteroaromatischer Kohlenwasserstoffrest mit 1 bis 20 C-Atomen, in dem auch ein oder mehrere H-Atome durch F oder CF₃ ersetzt sein können; dabei können zwei oder mehrere Substituenten R⁸ auch miteinander ein mono- oder polycyclisches, aliphatisches Ringsystem bilden;
R⁹ ist bei jedem Auftreten unabhängig voneinander ausgewählt aus der Gruppe bestehend aus H, Deuterium, Phenyl, Naphthyl, N(R²)₂, CN, CF₃, NO₂, OH, COOH, C(=O)OR³, C(=O)N(R³)₂, Si(R⁴)₃, B(OR⁵)₂, C(=O)R³, P(=O)(R⁷)₂, P(=S)(R⁷)₂, As(=O)(R⁷)₂, P(=S)(R⁷)₂, S(=O)R³, S(=O)₂R³, OSO₂R³, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁸ substituiert sein kann, wobei eine oder mehrere nicht benachbarte CH₂-Gruppen durch -R³C=CR³-, -C=C-, bzw. eine benachbarte CH₂-Gruppe durch -Si(R⁴)₂-, -Ge(R⁴)₂-, -Sn(R⁴)₂, -C(=O)-, -C(=S)-, -C(=Se)-, - C=N-, -C(=O)O-, -C(=O)N(R³)-, -P(=O)(R⁷)-, -As(=O)(R⁷)-, -P(=S)(R⁷), -As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R⁸ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁸ substituiert sein kann, oder eine Kombination dieser Systeme; dabei können zwei oder mehrere dieser Substituenten R⁹ auch miteinander ein mono- oder polycyclisches, aliphatisches, aromatisches und/oder benzoannelliertes Ringsystem bilden.

**3.** Organisches Molekül nach Anspruch 1 oder 2, **dadurch gekennzeichnet dass** eine der beiden chemischen Einheiten AF1 oder AF2 ausgewählt ist aus einer der durch die Formeln 3a bis 3e beschriebenen Strukturen; 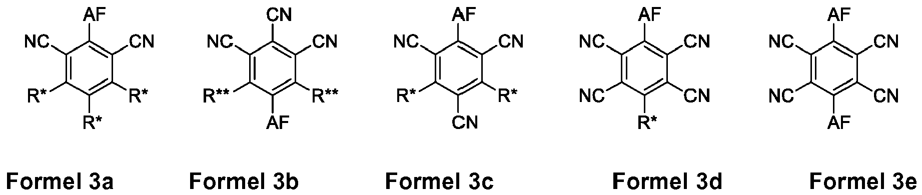 wobei die in den Ansprüchen 1 und 2 angegebenen Definitionen gelten und AF hier die Anknüpfungsposition an eine weitere chemische Einheit kennzeichnet.

**4.** Organisches Molekül nach einem oder mehreren der Ansprüche 1 bis 3, **dadurch gekennzeichnet dass** eine der beiden chemischen Einheiten AF1 oder AF2 eine Struktur gemäß einer der folgenden Formeln aufweist: 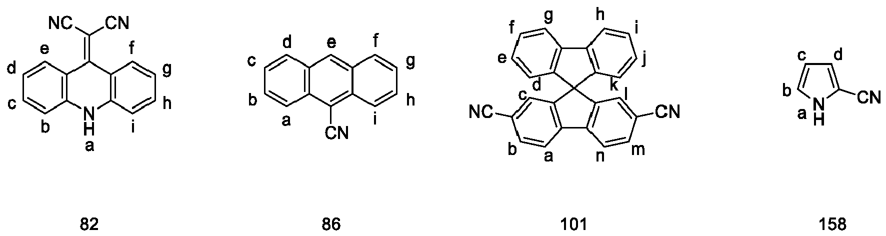 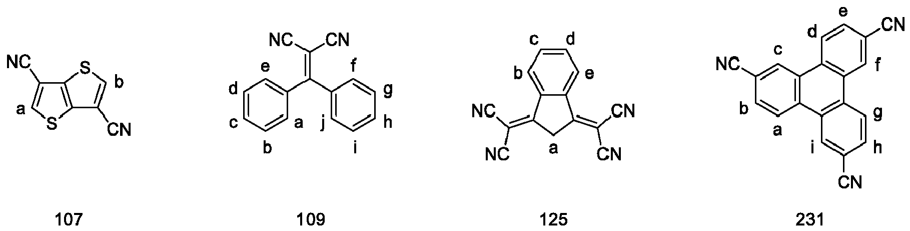 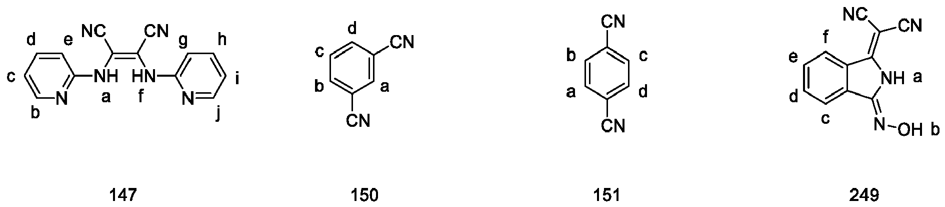 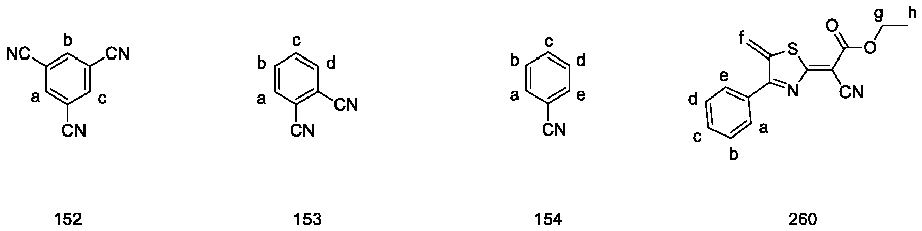 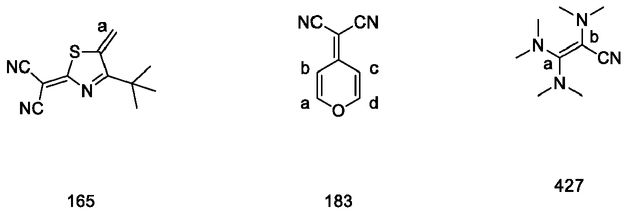 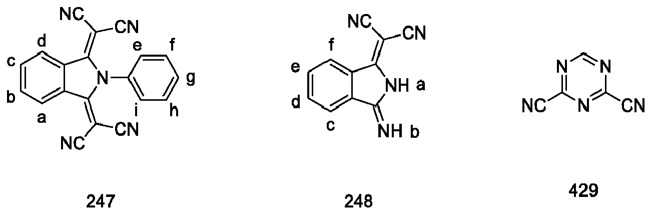 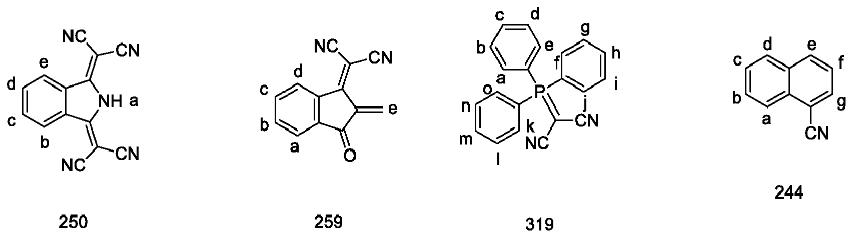 und wobei die Anknüpfungspositionen für eine weitere chemische Einheit AF mit Kleinbuchstaben gekennzeichnet sind;
und wobei die Reste AF1 und AF2 bevorzugt über Einfachbindungen direkt miteinander verbunden sind.

**5.** Organisches Molekül nach Anspruch 1 bis 4, wobei das organische Molekül an mindestens einer chemisch substituierbaren Position mindestens einen Rest R, insbesondere zur Steigerung der Löslichkeit und/oder der Polymerisierbarkeit, aufweist, wobei gilt:
R ist bei jedem Auftreten unabhängig voneinander ausgewählt aus der Gruppe bestehend aus H, Deuterium, Phenyl, Naphthyl, F, Cl, Br, I, N(R²)₂, -CN, -NC, -SCN, -CF₃, -NO₂, -OH, C(=O)OH, C(=O)OR³, C(=O)N(R³)₂, C(=O)SR³, C(=S)SR³, Si(R⁴)₃, B(OR⁵)₂, B(N(R⁶)₂)₂, C(=O)R³, P(=O)(R⁷)₂, As(=O)(R⁷)₂, P(=S)(R⁷)₂, As(=S)(R⁷)₂, S(=O)R³, S=NR³, S(=O)NR³, S(=O)₂NR³, S(=O)₂R³, O-S(=O)₂R³, SF₅, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R⁹ substituiert sein kann, wobei eine oder mehrere benachbarte CH₂-Gruppen durch -R⁹C=CR⁹-, -C=C-, bzw. eine benachbarte CH₂-Gruppe durch -Si(R⁴)₂-, -Ge(R⁴)₂-, -Sn(R⁴)₂, -C(=O)-, -C(=S)-, - C(=Se)-, -C=N-, -C(=O)O-, -C(=O)N(R³)-, -P(=O)(R⁷)-, -As(=O)(R⁷)-, -P(=S)(R⁷)-,-As(=S)(R⁷)-, -S(=O)-, -S(=O)₂-, -NR²-, -O-, oder -S- ersetzt sein können und wobei ein oder mehrere H-Atome durch Deuterium, F, Cl, Br, I, CN, CF₃ oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R⁹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R⁹ substituiert sein kann, oder eine Kombination dieser Systeme; dabei können zwei oder mehrere dieser Substituenten R auch miteinander ein monozyklisches aliphatisches Ringsystem mit insgesamt fünf oder sechs Ringgliedern bilden; wobei R² bis R⁹ definiert sind wie in Anspruch 2;
und wobei für polymerisierbare Reste gilt:
polymerisierbare Reste sind solche Reste, die polymerisierbare funktionelle Einheiten tragen, die mit sich selbst homopolymerisiert oder mit anderen Monomeren copolymiersiert werden können, wodurch das organische Molekül als Polymer mit Wiederholungseinheiten nach den Formeln 4 und/oder 5 erhalten werden 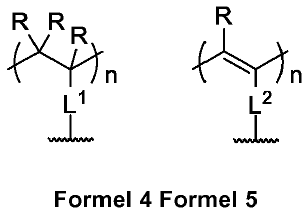 mit
gewellte Linie = Position, über die die Linkergruppe L¹ oder L² an das organische Molekül gebunden ist;
L¹ und L² = gleiche oder verschiedene Linkergruppen, aufweisend zwischen 0 bis 20, bevorzugt 1 bis 15, besonders bevorzugt 2 bis 10 Kohlenstoffatome; oder aufweisend eine Form -C(=O)O;
und wobei besonders bevorzugt
L¹ und L² = -X-L³ mit
X = O oder S;
L³ = eine weitere Linkergruppe ausgewählt aus der Gruppe aus einersubstituierten und unsubstituierten Alkylengruppe (linear, verzweigt oder cyclisch) und einer substituierten und unsubstituierten Arylengruppe, bevorzugt eine substituierte oder unsubstituierte Alkylengruppe mit 1 bis 10 Kohlenstoffatomen oder eine substituierte oder unsubstituierte Phenylengruppe, wobei auch Kombinationen möglich sind.

**6.** Verwendung eines organischen Moleküls nach Anspruch 1 bis 5 in einem optoelektronischen Bauelement.

**7.** Verfahren zur Herstellung eines optoelektronischen Bauelements, wobei ein organisches Molekül nach Anspruch 1 bis 7 verwendet wird, insbesondere mittels eines Vakuumverdampfungsverfahrens oder aus einer Lösung verarbeitet wird.

**8.** Optoelektronisches Bauelement, aufweisend ein organisches Molekül nach Anspruch 1 bis 5.

**9.** Optoelektronisches Bauelement nach Anspruch 8, aufweisend einer Anode und einer Kathode, die mindestens eine lichtemittierende Schicht umschließen, die das organische Molekül aufweist.

**10.** Optoelektronisches Bauelement nach Anspruch 8 oder 9, in dem die lichtemittierende Schicht mindestens ein Hostmaterial aufweist, wobei insbesondere der angeregte Singulettzustand (S1) und/oder der angeregte Triplettzustand (T1) des mindestens einen Hostmaterials höher ist als bei dem organischen Molekül.

**10.** Verwendung eines organischen Moleküls nach Anspruch 1 bis 9 als lumineszierender Emitter und/oder als Hostmaterial und/oder als Elektronentransportmaterial und/oder als Lochinjektionsmaterial und/oder als Lochblockiermaterial in einem optoelektronischen Bauelement.

**11.** Verwendung nach Anspruch 10, wobei das optoelektronische Bauelement ausgewählt ist aus der Gruppe bestehend aus:
• organischen lichtemittierenden Dioden (OLEDs),
• Licht-emittierenden elektrochemischen Zellen,
• OLED-Sensoren, insbesondere in nicht hermetisch nach außen abgeschirmten Gasund Dampf-Sensoren,
• organischen Dioden
• organischen Solarzellen,
• organischer Transistor
• organischen Feldeffekttransistoren,
• organischen Lasern und
• Down-Konversions-Elementen.

**12.** Verwendung nach Anspruch 10 oder 11, wobei der Anteil des organischen Moleküls an dem Emitter 1 % bis 99 % beträgt.

**13.** Verwendung nach Anspruch 10, 11 oder 12, wobei die Konzentration des organischen Moleküls als Emitter in optischen Licht emittierenden Bauelementen, insbesondere in OLEDs, zwischen 5 % und 80 % beträgt.

**14.** Optoelektronisches Bauelement, aufweisend ein organisches Molekül nach Anspruch 1 bis 9, insbesondere ausgeformt als ein Bauelement ausgewählt aus der Gruppe bestehend aus organischer lichtemittierender Diode (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**15.** Optoelektronisches Bauelement nach Anspruch 14, aufweisend
- ein Substrat,
- eine Anode und
- eine Kathode, wobei die Anode und die Kathode auf das Substrat aufgebracht sind, und
- mindestens eine lichtemittierende Schicht, die zwischen Anode und Kathode angeordnet ist und die ein organisches Molekül nach Anspruch 1 bis 6 enthält.

**16.** Optoelektronisches Bauelement nach Anspruch 15, in dem die lichtemittierende Schicht mindestens ein Hostmaterial aufweist, wobei der angeregte Singulettzustand (S₁) und/oder der angeregte Triplettzustand (T₁) des mindestens einen Hostmaterials höher ist als der angeregte Singulettzustand (S₁) und/oder der angeregte Triplettzustand (T₁) des organischen Moleküls nach Anspruch 1 bis 6.

**17.** Optoelektronisches Bauelement nach Anspruch 14 bis 16, aufweisend mindestens ein Hostmaterial bestehend aus einem Material gemäß Anspruch 1 bis 9.

**18.** Optoelektronisches Bauelement nach Anspruch 14 bis 17, wobei die lichtemittierende Schicht fluoreszente oder phosphoreszente Materialien beinhaltet und wobei es sich bei den Materialien der lichtemittierenden Schicht um organische Moleküle nach Anspruch 1 bis 9 handelt.

**19.** Optoelektronisches Bauelement nach Anspruch 18, in dem ein organisches Molekül gemäß Anspruch 1 bis 9 zusammen mit einem funktionellen Material einen Exciplex bilden.

**20.** Optoelektronisches Bauelement nach Anspruch 14 bis 19, wobei die Emission durch thermisch aktivierte verzögerte Fluoreszenz (TADF) charakterisiert ist.

**21.** Optoelektronisches Bauelement nach Anspruch 14 bis 19, in dem ein organisches Molekül nach Anspruch 1 bis 9 als Ladungstransportschicht verwendet wird.

**22.** Verfahren zur Herstellung eines optoelektronischen Bauelements, wobei ein organisches Molekül nach Anspruch 1 bis 9 verwendet wird.

**23.** Verfahren nach Anspruch 22, wobei die Verarbeitung des organischen Moleküls mittels eines Vakuumverdampfungsverfahrens oder aus einer Lösung erfolgt.
